# EUROPÄISCHE PATENTSCHRIFT

(11) **EP 1 012 256 B1**
(45) Veröffentlichungstag und Bekanntmachung des Hinweises auf die Patenterteilung: **31.03.2004**
(21) Anmeldenummer: 98905267.5
(22) Anmeldetag: 02.01.1998
(51) Int. Cl.: C12N 15/62, C12N 15/29, C07K 19/00, C12P 21/02, A61K 35/78, A61K 38/16, A61K 47/48, C12Q 1/68

(54) **REKOMBINANTE FUSIONSPROTEINE AUF DER BASIS RIBOSOMEN-INAKTIVIERENDER PROTEINE DER MISTEL VISCUM ALBUM**
RECOMBINANT FUSION PROTEINS BASED ON RIBOSOME-INACTIVATING PROTEINS OF EUROPEAN MISTLETOE VISCUM ALBUM
PROTEINES RECOMBINANTES DE FUSION A BASE DE PROTEINES DU GUI VISCUM ALBUM QUI DESACTIVENT LES RIBOSOMES

(30) Priorität: 02.01.1997 EP 97100012
(43) Veröffentlichungstag der Anmeldung: 28.06.2000
(73) Patentinhaber: VISCUM Aktiengesellschaft, 51429 Bergisch Gladbach (DE)
(72) Erfinder: ECK, Jürgen, D-64646 Heppenheim (DE); SCHMIDT, Arno, D-64572 Büttelborn 3 (DE); ZINKE, Holger, D-64404 Bickenbach (DE)
(74) Vertreter: VOSSIUS & PARTNER
(86) Internationale Anmeldenummer: PCT/EP1998/000009
(87) Internationale Veröffentlichungsnummer: WO 1998/029540

(56) Entgegenhaltungen:
- EP-A- 0 751 221
- WO-A-98/18820
- DE-A- 4 221 836
- TONEVITSKY A G ET AL: "IMMUNOTOXIN WITH MISTLETOE LECTIN I A-CHAIN AND RICIN A-CHAIN DIRECTED AGAINST CD5 ANTIGEN OF HUMAN T-LYMPHOCYTES;COMPARISON OF EFFICIENCY AND SPECIFICITY" INTERNATIONAL JOURNAL OF IMMUNOPHARMACOLOGY, Bd. 13, Nr. 7, 1991, Seiten 1037-1041, XP002019007
- PAPROCKA M ET AL: "THE ACTIVITY OF TWO IMMUNOTOXINS COMPOSED OF MONOCLONAL ANTIBODY MOAB-16 AND A-CHAIN OF RICIN (MOAB-16-RTA) OR A-CHAIN OF MISTLETOE LECTINI (MOAB-16-MLIA)" ARCHIVUM IMMUNOLOGIAE ET THERAPIAE EXPERIMENTALIS, Bd. 40, Nr. 3/04, 1992, Seiten 223-227, XP002019006
- GABIUS, H.-J. ET AL.: "The immunomodulatory beta-galactosidase-specific lectin from mistletoe: partial sequence analysis, cell and tissue binding, and impact on intracellular biosignalling of monocytic leukemia cells" ANTICANCER RESEARCH, Bd. 12, 1992, Seiten 669-676, XP002074009
- DIETRICH J B ET AL: "IDENTITY OF THE N-TERMINAL SEQUENCES OF THE THREE A CHAINS OF MISTLETOE (VISCUM ALBUM L.) LECTINS: HOMOLOGY WITH RICIN-LIKE PLANT TOXINS AND SINGLE-CHAIN RIBOSOME-INHIBITING PROTEINS" ANTI-CANCER DRUGS, Bd. 3, Nr. 5, 1992, Seiten 507-511, XP002019008

## Beschreibung

Die Erfindung betrifft Nukleinsäuremoleküle, die Fusionsproteine codieren, die als Komponenten mindestens ein Effektormodul, ein Prozessierungsmodul und ein Targetingmodul enthalten. Vorzugsweise codieren die erfindungsgemäßen Nukleinsäuremoleküle ferner ein Modulatormodul und/oder ein Affinitätsmodul. Die Erfindung betrifft ferner Vektoren, die diese Nukleinsäuremoleküle enthalten, Wirte, die mit den erfindungsgemäßen Vektoren transformiert sind, Fusionsproteine, die von den erfindungsgemäßen Nukleinsäuren codiert oder von den erfindungsgemäßen Wirten produziert werden sowie Arzneimittel, die die erfindungsgemäßen Polypeptide oder Vektoren enthalten. Diese Arzneimittel haben besondere Bedeutung bei solchen Erkrankungen, die auf der pathologischen Vermehrung und/oder der erhöhten Aktivität von Zellpopulationen beruhen. Eine vorübergehende, schubartige starke Proliferation, Infiltration und Immunaktivität von Zellen des Immunsystems findet man bei Autoimmunerkrankungen und Allergien, wobei die Spezifität dieser Immunzellen auf der Reaktion auf jeweils bestimmte Antigene bzw. Allergene beruht. Ferner können solche Arzneimittel auch vorteilhaft zur Tumortherapie eingesetzt werden. Die in dieser Erfindung beschriebenen Polypeptide und Vektoren können zur Entwicklung von Arzneimitteln und zur Testung von Toxinaktivität modulierenden Faktoren eingesetzt werden. Somit betrifft die Erfindung ferner entsprechende Verfahren, Verwendungen und Kits. Vorzugsweise werden die Module mit Ausnahme des Affinitäts- und des Targetingmoduls von Nukleinsäuren codiert, die aus der das Mistellektin-Proprotein codierenden Sequenz stammen oder davon abgeleitet sind.

Die medizinische Forschung hat in den letzten Jahren ein breites Spektrum von Erkrankungen aufgedeckt, die durch Veränderung oder Entartung von körpereigenen Zellen hervorgerufen werden, was sich z. B. in einer zellspezifischen oder veränderten Rezeptorausstattung widerspiegelt. Eine verbreitete Strategie zur Entwicklung von Therapieansätzen gründet sich auf das Prinzip, eine zellabtötende Substanz, die von sich aus nicht in der Lage ist, in das Zellinnere einzudringen, mit einer zweiten nicht-toxischen Substanz - die dafür in der Lage ist, über die Bindung an ein Oberflächenprotein ins Zellinnere einzudringen - zu koppeln. Je zelltypspezifischer das Targetingmolekül ist, desto selektiver lassen sich pathogene Zellen abtöten, ohne gesunde Zellen zu schädigen. Solche zelltypspezifischen toxischen Fusionsproteine werden in Form sogenannter Immuntoxine und Mitotoxine (Vitetta et al., 1987; Lambert et al., 1988; Lappi et al., 1990; Pastan et al., 1991; Ramakrishnan et. al., 1992; Pastan et al., 1992; Brinkman, 1996) zur selektiven Abtötung von Tumorzellen eingesetzt.

Bekannte Beispiele zellabtötender Komponenten sind die bakteriellen Toxine Diphterietoxin (Collier, 1988), Pseudomonas Exotoxin (Pastan et al., 1989) und Tetanus Toxin (Brinkmann, 1996), sowie pflanzliche ribosomeninaktivierende Proteine (RIP) (Barbieri et al., 1993). Bei den pflanzlichen Toxinen unterscheidet man Typ I RIPs wie z. B. Gelonin oder Saporin, die aus einer einzigen toxischen Domäne bestehen, und Typ II-RIPs (zu denen auch das Mistellektin zählt), die eine zweite Domäne mit zuckerbindenden Eigenschaften besitzen (Stirpe et al., 1992; Barbieri et al., 1993). Bekanntester Vertreter der letzteren Gruppe ist das Ricin. Die Entfaltung der toxischen Wirkung setzt einen komplexen Aufnahme- und Prozessierungsweg voraus: nach rezeptorvermittelter Aufnahme, Transport über "Clathrin-coated" Vesikel in Endosomen (Nicolson, 1974) erfolgt die Prozessierung/Freisetzung der Toxin-Komponente aus dem Fusionsprotein als Voraussetzung für die Translokation in das Cytoplasma. Dort entfaltet das Toxin seine toxische Wirkung und tötet die Zelle ab, Mistellektin ist als potenter Apoptoseinduktor beschrieben (Janssen et al, 1996). Diese Eigenschaft ist wiederum vom Zusammenwirken von A-und B-Kette abhängig, wobei die RIP-Aktivität unverzichtbar ist. Die Zytotoxizität von Mistellektin ist konzentrations- und zeitabhängig apoptotischer oder nekrotischer Natur. Werden hohe Konzentrationen bzw. Dosierung angewendet, ist ein nekrotischer Zelltod zu beobachten. Das gleiche trifft für moderat-toxische Konzentrationen zu, die jedoch über einen Zeitraum größer 24 h appliziert werden. Im Zeitraum weniger Stunden oder bei niedrigen Konzentrationen ist die Art des ML-induzierten Zelltodes die Apoptose; dies wurde bei verschiedenen Zelltypen festgestellt (MOLT-4, THP-1, PBMC) (Möckel et al, 1997).

Zu den anfänglichen Versuchen, ein Toxin mit einem Targetingmolekül zu verbinden, zählte die chemische Kopplung über Thioether (Masuho et al., 1982). Teilweise kommt es durch die irreversible Kopplung jedoch zur inaktivierung des Toxins (Vitetta et al., 1993). Daher verwendet man zumeist Kopplungsagentien, die zur Kopplung Ober eine Disulfidbrücke führen, wie z. B. N-Succinimidyl-3-(2-pyridyldithio)propionat (SPDP) (Carlsson *et al*., 1978; Jansen et al., 1982). Dabei muß man jedoch den Nachteil in Kauf nehmen, daß disulfidverbrückte Komponenten *in vivo* nur eine relativ geringe Stabilität besitzen. Zudem wurde mit dieser proteinchemischen Modifikation oftmals ein starker Aktivitätsverlust beobachtet (Thorpe et al., 1981; Battelli et al., 1990; Bolognesi et al., 1992).

Weiterhin wurden auch Mistellektine chemisch, unter Verwendung von SPDP, mit Antikörpern gekoppelt. So beschreibt Tonevitsky et al. (Intern. J. of Immunopharmac. (1991), 1037-1041) die chemische Kopplung von Mistellektin I A-Kette mit einem monoklonalen anti-CD5 Antikörper. Paprocka et al. (Arch. Immun. Ther. Exp. (1992), 223-227) koppelten chemisch die A-Kette des Mistellektins I mit dem monoklonalen anti-LI210V Antikörper. Jedoch, wie auch in Paprocka (1992) beschrieben, kann eine hohe unspezifische Cytotoxizität der Immuntoxine auf einer durch die Bindung des Antikörpers verursachten Veränderung der Konformation der Mistellektin A-Kette beruhen.

Ein weiterer gravierender Nachteil der chemischen Kopplung ist die Erzeugung eines inhomogenen Substanzgemisches, das die Anwendung aufwendiger Methoden zur Anreicherung des gewünschten Produktes nach sich zieht (Pastan, 1992).

Um das Problem der chemischen Kopplung zu umgehen, hat man mit der Entwicklung von bifunktionellen Antikörpern begonnen, die mit der einen Bindesteite ein Toxin und mit der anderen eine Zielzelle binden können (Milstein et al., 1983; Webb et al., 1985; Glennie et al., 1988). Während hierdurch eine einfache Freisetzung des Toxins im Zuge der Intemalisierung ermöglicht wurde, zeigte sich bereits während der Zirkulation im Blut eine partielle Dissoziation der Komplexe und damit eine partielle unspezifische Toxizität durch die Toxine. Darüber hinaus ist die Darstellung der bispezifischen Antikörper ein sehr aufwendiges Verfahren. Durch das hohe Molekulargewicht dieser Konstrukte wird zudem das immunogene Potential erhöht, sowie die Tumorpenetration verschlechtert (Brinkmann, 1996). Des weiteren ist die Darstellung der bispezifischen Antikörper ein sehr aufwendiges Verfahren.

Durch moderne molekularbiologische Methoden ist es in letzter Zeit gelungen, toxische Proteine wie Diphterietoxin, Pseudomonas Exotoxin, Ricin oder Saporin zu klonieren (Greenfield et al., 1983; Gary et al., 1984; Lamb et al., 1985; Benatti et al., 1989) und somit genetischen Fusionen mit Zieldomänen zugänglich zu machen. Mit der Verwendung von rekombinanten bakteriellen Toxinen konnten zwar betreffend ihrer Wirksamkeit medizinische Erfolge erzielt werden, die Verwendung dieser Toxine ist Jedoch problematisch, da große Teile der Bevölkerung durch Schutzimpfungen immunisiert worden sind und so neutralisierende Antikörper gegen die Toxin-Komponente besitzen (Brinkmann, 1996). Vorteilhaft ist daher die Anwendung pflanzlicher Toxine, wie z.B. des Mistellektins oder des Ricins. Zur Entfaltung der toxischen Wirkung (von Typ II RIPs bzw. rekombinanten Fusionsproteinen) ist jedoch die intrazelluläre Freisetzung des Toxins/der Toxin-komponente entscheidend (Barbieri et al., 1993). Beispielsweise wurde die A-Kette des Ricins (Ricin A) zur rekombinanten Konstruktion von Mitotoxinen eingesetzt, wobei zwei rekombinante IL2-Ricin A Fusionsproteine konstruiert wurden, die sich in der Wahl der Linkersequenz unterschieden haben. Das Konstrukt mit dem intrazellulär proteasesensitiven Diphterietoxinloop ist cytotoxisch gegenüber CTLL-2-Zellen, während die zweite Variante mit einer nicht intrazellulär prozessierbaren Linkersequenz nicht cytotoxisch ist (Cook et al., 1993). Die Autoren nutzen hierbei die natürlicherweise in bakteriellen Toxinen vorkommenden proteasesensitiven Sequenzen. Eine Übertragung der Befunde auf andere Toxine als Effektormodul ist nicht oder nur eingeschränkt möglich. So müssen für andere als die von Cook beschriebenen Toxine neue Aktivierungs/Prozessierungsmöglichkeiten geschaffen werden, Natürlicherweise werden Typ II-RIPs in der Pflanze in Form RIP-inaktiver Prä-Pro-Proteine synthetisiert, und dann in speziellen Zellkompartimenten zu reifen Toxinen prozessiert (Lord, 1985). Gezeigt werden konnte die Translation von ProRicin-mRNA in *Xenopus* Oocyten (Westby et al., 1992). Dabei haben sich jedoch keine Anhaltspunkte für eine *in vivo* Aktivierung des Pro-Proteins ergeben, was den Einsatz eines rekombinanten Pro-Ricins als Toxin ausschließt (Richardson et al., 1989). Auf der Basis dieser und anderer Ergebnisse ist man daher davon ausgegangen, daß die Prozessierung der Pro-Sequenzen der Typ II-RIPs durch spezielle pflanzliche Proteasen erfolgt, und hat dieses Prinzip auch für das Mistellektin angenommen (Hara-Nishimura et al., 1991).

Bei der Suche nach einem geeigneten Toxin als Wirkstoff in Immuntoxinen wurde vor allem das Ricin untersucht. Auf Basis der A-Domäne des Typ II-RIPs Ricin (Ricin A) wurden eine ganze Reihe von Immuntoxinen hergestellt und zur Krebstherapie erprobt (Spitler et al., 1987; Shen et al., 1988; Byers et al., 1989; Vitetta et al., 1991). Eine nachteilige Eigenschaft von Ricin A ist jedoch, daß es auch unspezifisch in Zellen eindringen kann, so daß es bei den meisten Patienten zu starken Nebenwirkungen, wie z.B. dem "vascular leak syndrome" kommt (Gould et al., 1989; Soer-Rodriguez et al., 1993). Darüber hinaus sind in einer weiteren Studie Arbeiten zum Einsatz von Saporin als Bestandteil von Immuntoxinen beschrieben worden. Diese Studie befaßt sich mit dem Vergleich von biochemischer und rekombinanter Darstellungsmethode von Immun- bzw. Mitotoxinen, wobei das Typ I-RIP Saporin sowohl chemisch, als auch durch Genfusion mit dem Mitogen "bFGF" gekoppelt wurde (Lappi et al., 1994). Die auf unterschiedlichen Wegen hergestellten Substanzen zeigen beide die gleiche anti-Tumor-Wirksamkeit in *in vitro-* und in *in vivo*-Studien, wobei die Darstellung der rekombinanten Substanz wesentlich problemloser möglich ist. Allerdings wurde die intrazelluläre Freisetzung des Toxins erst durch den nicht verallgemeinerbaren Umstand ermöglicht, daß sich eine proteasesensitive Spaltstelle im verwendeten Targetingmolekül bFGF befindet. Eine breite Anwendbarkeit der von den Autoren erbrachten Daten auf eine breite Palette von interessierenden Zielzellen erscheint daher nicht möglich.
Sun et al. (1997) beschreiben ein chemisch-kovalentes Konjugat bestehend aus der Cholera Toxin B-Untereinheit (CTB) und dem Myelin Basic Protein (MBP), mit dem bei oraler Applikation von 50 µg Protein die EAE, das Tiermodell der MS, effektiv unterdrückt werden kann. Das Konjugat mit dem Toxin ist 50 bis 100-fach toxischer als das Antigen MBP alleine. Die beiden Komponenten MBP und CTB wurden jeweils aus der natürlichen Quelle isoliert. Dieser Ansatz zeigt, daß prinzipiell ein Toxin über die Antigenerkennung an den Wirkort also zu den Targetzellen gebracht werden kann. Jedoch beinhaltet die Herstellungsweise der Konjugate die beschriebenen Schwierigkeiten der chemischen Kopplung und der Limitierung der Verfügbarkeit und gleichbleibenden Reinheit der Komponenten.
Fusionsproteine wurden zur Anwendung als Vakzine beschrieben (Price, 1996). Dazu wurden im Hefe-Expressionssystem Antigene an GM-CSF gekoppelt, um die Immunantwort zu stimulieren, wobei das jeweilige Antigen immer an den C-Terminus des GM-CSF, wahlweise unter Einschub eines Linkers, gekoppelt wird. Die beschriebenen Fusionsproteine sind in ihrer Anwendnung limitiert auf die Stimulation von Antigen-präsentierenden Zellen durch den Wachstumsfaktor GM-CSF und in ihrer Herstellung auf die Expression in Hefe.
Better et al. (1995) beschreiben Fusionsproteine aus humanisierten Antikörpern und dem RIP Gelonin. Damit konnten die Autoren CD5 positive T- und B-Zellen targetieren. Die Toxizitäten waren dabei sehr stark unterschiedlich je nach Orientierung und Art der Komponenten. PBMC aus 2 verschiedenen Spendern waren unempfindlich gegen Antikörper-Ricin A-Kette Fusionsproteine, jedoch sensitiv gegen solche Fusionsproteine mit Gelonin als Toxin. Dies zeigt, daß die Auswahl eines geeigneten Toxins entscheidend für die Wirksamkeit eines Immunfusionsproteins sein kann. Der Ansatz von Better et al. setzt jedoch die Verfügbarkeit von Antikörpergenen kodierend für solche Antikörper, die eine spezifische Determinante von Targetzellen erkennen. Diese Voraussetzungen sind jedoch gerade im Falle von autoreaktiven T-Zellen nicht unbedingt gegeben, da diese sich vielmehr über ihre Antigen-Erkennung definieren.
Ein anderer Ansatz, um autoreaktive T-Zellen durch Präsentation ihres spezifischen Antigens unschädlich zu machen beruht auf einer Beladung von aus Milzzell-Membranen isolierten MHC-Molekülen mit Antigenfragmenten, z.B. MBP-, HSP- und Acetylcholinrezeptorpeptiden (Spack et al., 1995). Durch die Präsentation des jeweilgen Antigens ohne kostimulatorische Signale werden die T-Zellen anergisch, d.h. die Bindung des Antigens löst keine Proliferation aus, sondern die Zellen verbleiben im Ruhezustand. Im Tiermodell der Autoimmunkrankheit Myastenia Gravis konnte mit einem solchen Protein-Komplex die Krankheitsprogression verhindert werden. Der Nachteil des Konzeptes der Anergie-Induktion besteht in der nicht lang anhaltenden Wirkung, da das per se nicht toxische Antigen in geringen Mengen die Zelle nicht abtötet, sondern nur vorübergehend inhibiert.
Allgemein fehlt im derzeitigen Stand der Technik ein modulares System geeigneter Effektor-, Prozessierungs-, Modulator-, Targeting- und Affinitätsmodule, das eine universelle Anwendbarkeit in unterschiedlichen medizinischen Indikationen ermöglicht. Mit der Kenntnis von krankheitsrelevanten Zellpopulationen, insbesondere im Bereich der immunologisch kompetenten Zellen, wäre es wünschenswert, diese gezielt beeinflussen bzw. ausschalten zu können.
Aufgabe der vorliegenden Erfindung war somit, die aus dem Stand der Technik bekannten Nachteile in der Konstruktion von Immuntoxinen zu beseitigen und gleichzeitig sicherzustellen, daß die Immuntoxine ihre toxische Wirkung in einer breiten Palette von Zielzellen erst intrazellular entfalten.

Die Lösung dieser Aufgabe wird durch die in den Ansprüchen gekennzeichneten Ausführungsformen erzielt.

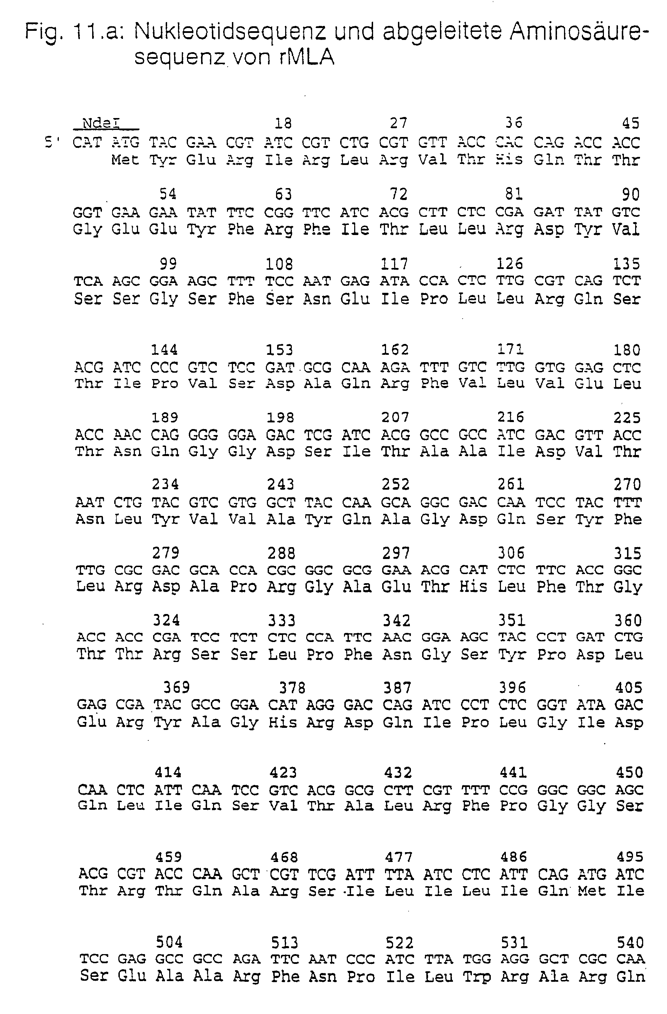

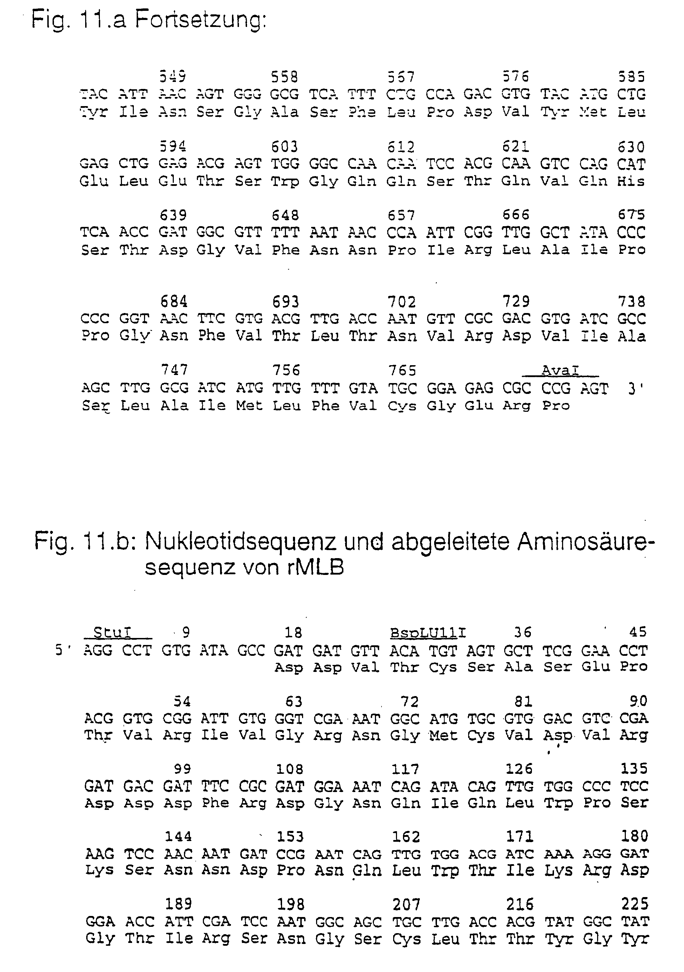

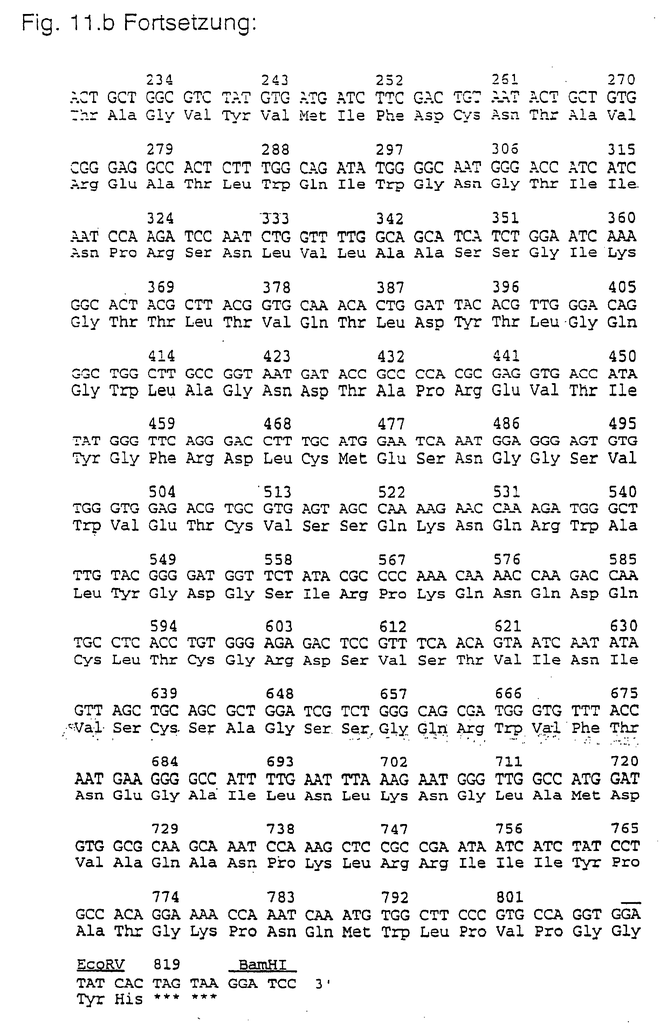

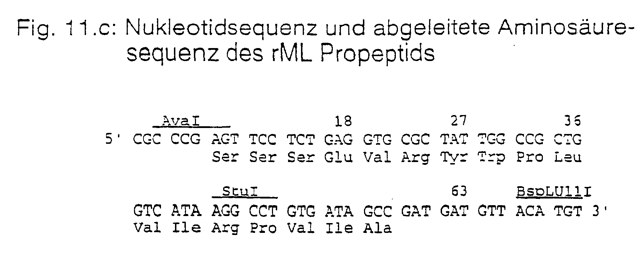

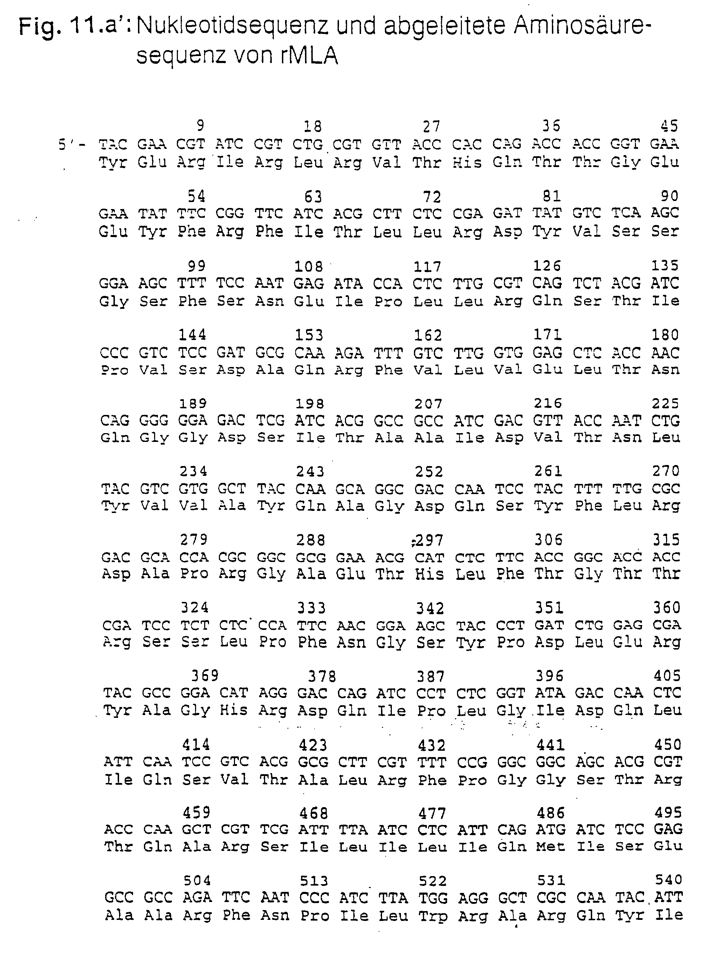

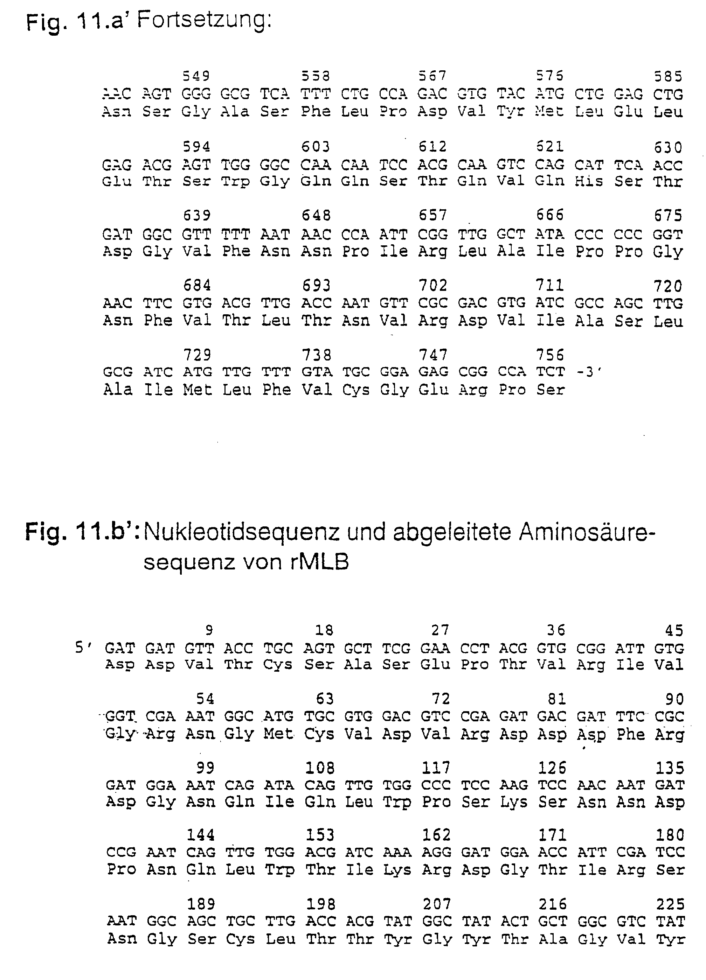

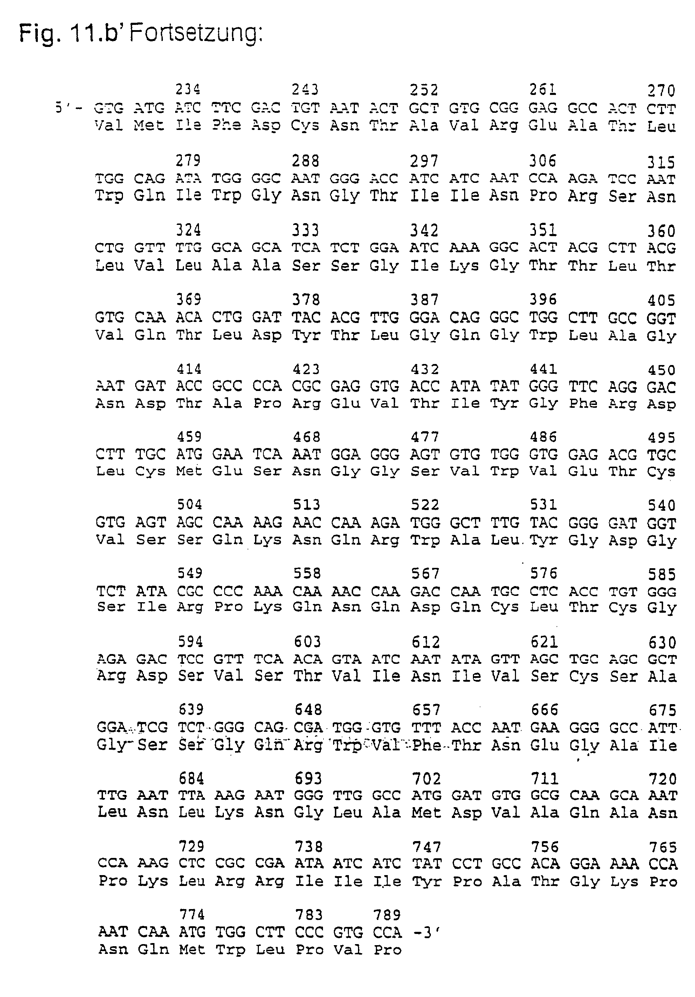

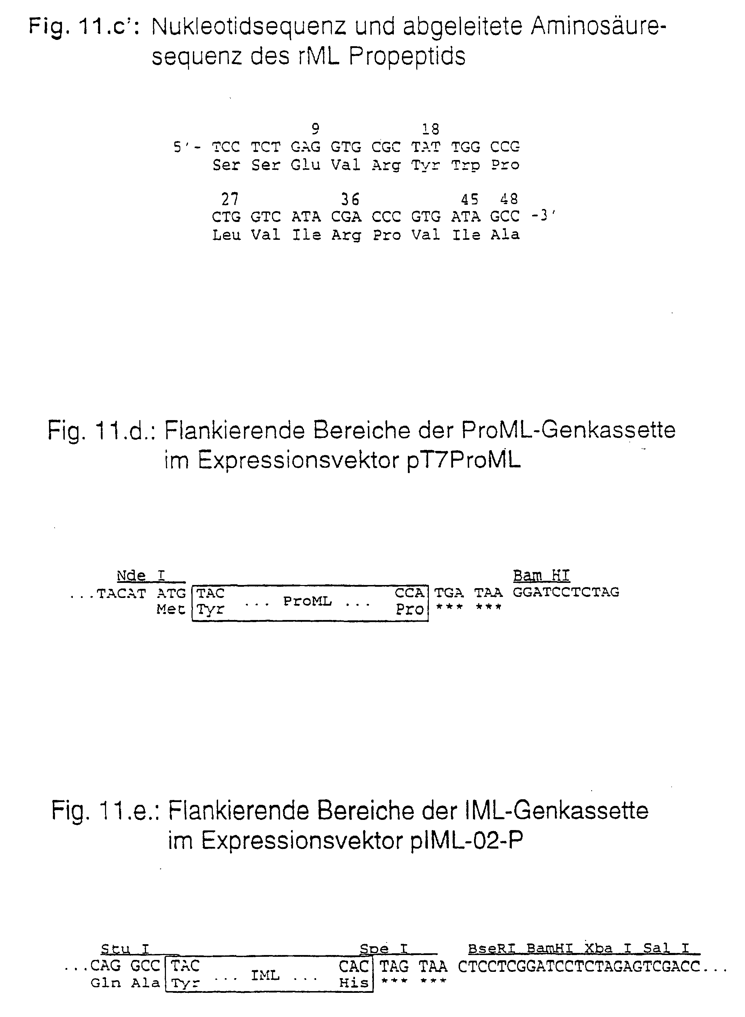

Die Erfindung betrifft demgemäß ein Nukleinsäuremolekül, das ein Fusionsprotein codiert, das die folgenden Komponenten aufweist:
(a) ein Effektormodul, das intrazellulär cytotoxisch wirkt;
(b) ein Prozessierungsmodul, das kovalent mit dem Effektormodul verbunden ist, und das eine Erkennungssequenz für eine Protease aufweist, wobei das Prozessierungsmodul das Misteflektin-Propeptid umfaßt oder ein Fragment oder Derivat davon, das eine Erkennungssequenz für eine Protease aufweist und die intrazelluläre Freisetzung des Effektormoduls in der Zielzelle durch zelleigene Proteasen im Zuge der rezeptorvermittelten Endocytose in den Endosomen und Prälysosomen stattfinden lässt, umfaßt und wobei das Mistellektin-Propeptid von einem Nukleinsäuremolekül codiert wird, ausgewählt aus der Gruppe bestehend aus:
   (i) Nukleinsäüremolekülen, die eine Nukleotidsequenz umfassen, die die in Fig. 11.c angegebene Aminosäuresequenz oder ein Fragment davon codiert;
   (ii) Nukleinsäuremolekülen, die die in Fig. 11.c angegebene Nukleotidsequenz oder ein Fragment davon umfassen;
   (iii) Nukleinsäuremolekülen, die mit einem Nukleinsäuremolekül aus (i) oder (ii) unter stringenten Bedingungen hybridisieren; und
   (iv) Nukleinsäuremolekülen, die zu den in (iii) genannten Nukleinsäuremolekülen entsprechend dem genetischen Code degeneriert sind; und
(c) ein Targetingmodul, das kovalent mit dem Prozessierungsmodul verbunden ist, und das spezifisch an die Oberfläche einer Zelle bindet, wodurch die Intemalisierung des Fusionsproteins in die Zelle vermittelt wird.

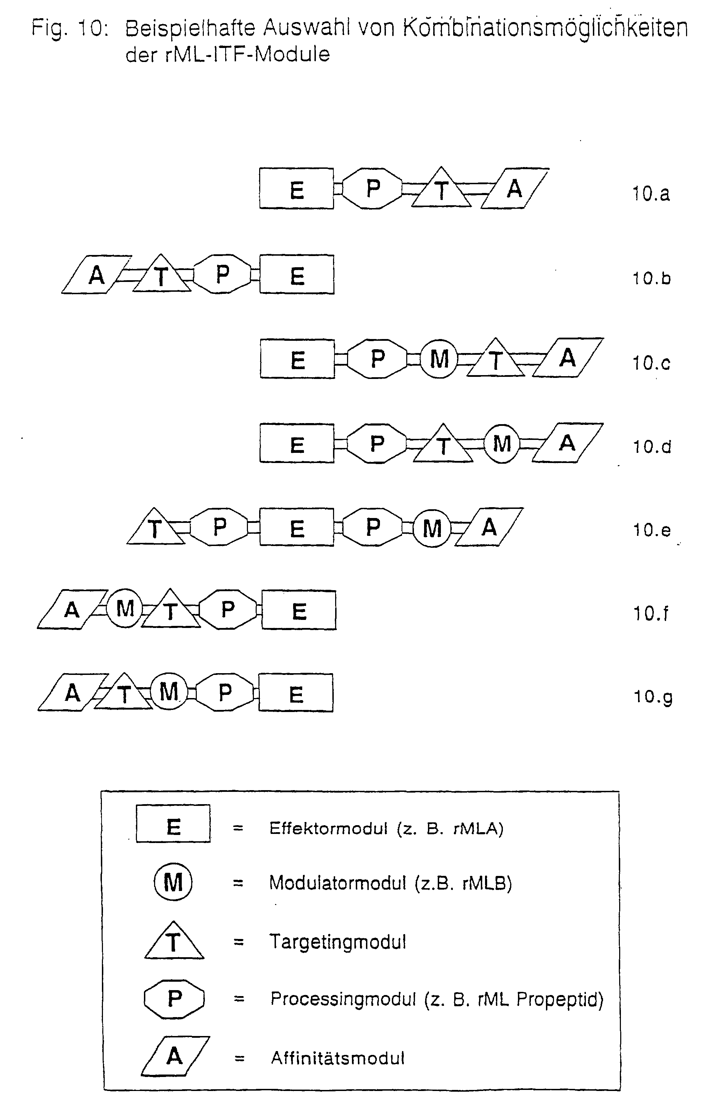

Unter dem Begriff "Modul" wird erfindungsgemäß ein Peptid verstanden, das von einer DNA-Sequenz codiert wird und bestimmte funktionelle Eigenschaften aufweist. Diese funktionellen Eigenschaften begründen sich aus der Primär-, Sekundärund/oder Tertiärstruktur solcher Peptide und betreffen biochemische, molekulare, enzymatische, zelluläre und/oder physiologische Funktionen. Ein erfindungsgemäßes Modul zeichnet sich weiter dadurch aus, daß es auf DNA-Ebene günstige Adaptoren aufweist, die eine Fusion an andere Module leicht erlauben und diese Adaptorsequenzen auf Peptidebene mit den Funktionen der Module nicht nachteilig interferieren.
Durch den Begriff "Fusionsprotein" ist erfindungsgemäß festgelegt, daß es sich bei den erfindungsgemäßen Nukleinsäuren und den dadurch codierten Fusionsproteinen um rekombinant hergestellte Moleküle handelt.
Unter dem Begriff "Targetingmodul, das kovalent mit dem Processierungsmodul verbunden ist" werden erfindungsgemäss auch solche Ausführungsformen verstanden, in denen zwischen diesen beiden Modulen weitere Module oder Sequenzen kovalent eingelagert sind. In diesem Zusammenhang wird auf Fig. 10.c verwiesen, das eine derartige erfindungsgemässe Ausführungsform darstellt: Dort ist das Targetingmodul mit dem Processierungsmodul über ein Modulatormodul kovalent verbunden. Wichtig ist im Sinne dieser Erfindung, dass die Verknüpfung von Processierungs- und Targetingmodul, mit oder ohne intermediäre Sequenz, kovalenten Charakter hat.

Erfindungsgemäß besteht die Funktion des Effektormoduls in der Abtötung oder nachhaltigen Veränderung der vitalen Prozesse von Zielzellen. Dies kann durch enzymatische Aktivitäten des Effektormoduls ausgelöst werden, indem physiologische intrazelluläre Prozesse beeinträchtigt werden (z. B. Stoffwechsel-, insbesondere Energiestoffwechselprozesse, molekulargenetische Prozesse, insbesondere Translation, Transkription und Replikation und spezifische zelluläre Kaskaden wie z. B. die Induktion apoptotischer Prozesse). In jedem Fall wird über die intrazelluläre Aktivität des Effektormoduls eine Zielzelle in ihrem physiologischen Status, z. B. im Wachstumsverhalten, verändert, z. B. retardiert oder aber vollständig abgetötet und vernichtet. Als ein bevorzugtes Beispiel für ein geeignetes Effektormodul dient die rekombinante A-Domäne des Mistellektins (rMLA) oder ein intrazellulär toxisches Fragment oder Derivat davon. Unter dem Begriff "Fragment" einer Mistellektin A-Kette wird erfindungsgemäß ein Peptid verstanden, das einen Teil der Aminosäuresequenz dieser Kette aufweist und intrazellulär toxische Aktivität aufweist. Die Toxizität muß nicht das gleiche Ausmaß aufweisen wie bei der vollständigen A-Kette. Ein Fragment kann beispielsweise durch proteolytische Spaltung der rekombinant hergestellten A-Kette oder durch rekombinante Manipulation der die A-Kette codierenden Nukleinsäure und nachfolgende Expression erzeugt werden. Der Fachmannn weiss auf der Basis seiner allgemeinen Fachkenntnis und der Lehre dieser Erfindung, wie er in dieser Anmeldung hier und später genannten Fragmente rekombinant herstellen und auf Aktivität testen kann. Die katalytische Aktivität von rMLA besteht in der Depurinierung der 28S rRNA eukaryontischer Zellen. Der Einsatz von rMLA als Effektormodul ist von besonderem Interesse, da es in therapeutischen Dosen den Zelltod vorwiegend durch Induktion von Apoptose herbeiführt, so daß im Gegensatz zur Nekrose die Entstehung von gewebsschädigenden Entzündungsreaktionen durch Freisetzung von Zelltrümmern und intrazellulären Zellbestandteilen weitgehend unterbleibt. Der programmierte Zelltod (Apoptose) spielt u.a. eine Rolle bei der Regulation von Zellpopulationen des Immunsystems z.B. auch bei der Eliminierung von T-Zellen, die durch ihr spezifisches Antigen je nach Konzentration stimuliert oder "überreizt" werden können. Dieses Phänomen stellt bei im Fall einer Autoimmunerkrankungen den natürlichen Mechanismus zur Eindämmung der Autoimmunreaktion (Beendigung eines Schubes) dar (Schmied et al., 1993) und kann daher therapeutisch genutzt werden, um autoreaktive T-Zellen durch Gabe bestimmter Mengen des Autoantigens in die Apoptose zu treiben (Gold et al., 1997).

Die Funktion der Prozessierungsmodule besteht erfindungsgemäß zum einen in der kovalenten Verknüpfung des Effektormoduls mit Modulator-, Targeting- oder Affinitätsmodulen zu einer Polypeptidkette, was eine rekombinante Darstellung der Fusionsproteine erlaubt. Zum anderen zeichnen sie sich durch den Gehalt geeigneter Erkennungssequenzen für Proteasen aus, was die intrazelluläre Freisetzung des Effektormoduls in der Zielzelle durch zelleigene Proteasen im Zuge der rezeptorvermittelten Endocytose in den Endosomen und Prälysosomen stattfinden läßt. Das Prozessierungsmodul des Mistellektins erfüllt, beispielsweise bei C-terminaler Fusion an die rMLA, im Gegensatz zu entsprechenden Sequenzen in Propeptiden anderer pflanzlicher Typ II-RIPs wie z. B. des Ricins, überraschenderweise sowohl die Voraussetzungen zur intrazellulären Prozessierung durch endosomale Proteasen von Säugerzellen bzw. humanen Zellen, sowie rMLA-inaktivierende Eigenschaften im nicht-prozessierten Zustand. Vorzugsweise sind die das Prozessierungsmodul spaltenden Proteasen Säugerproteasen. Besonders bevorzugt sind Proteasen menschlichen Ursprungs. Weiterhin ist bevorzugt, dass diese Proteasen intrazellulär vorkommen.

Als Targetingmodule werden im Sinne der Erfindung alle Moleküle auf Polypeptidbasis verstanden, die in der Lage sind, dem erfindungsgemäßen Fusionsprotein über eine spezifische Affinität zu einem Zelloberflächenprotein Zugang in das Zellinnere zu verschaffen. Als Zielzellen eignen sich insbesondere Immunzellen des Blutes, wie z. B. T-Lymphocyten, die über ihre individuelle Rezeptorausstattung durch den Einsatz geeigneter Targetingmodule diskriminiert werden können. Als Targetingmodule können Proteine, Proteinfragmente oder Peptide dienen. Beispielsweise könnte es sich hierbei um MHC-bindende Peptide handeln, was sich zur selektiven Inaktivierung klonaler T-Zellinien, beispielsweise allergener T_{H}2-Zellinien, ausnutzen ließe.

Mit der in der anhängigen europäischen Patentanmeldung mit der Anmeldenummer EP 95109949.8 beschriebenen Aufklärung der Nukleotidsequenz des Mistellektin-Gens ist die Basis für die vorliegende Erfindung geschaffen worden.

Durch die rekombinante Verfügbarkeit des ProML-Gens wurde es möglich, mit einem flexiblen modularen Konzept (exemplarisch dargestellt in *Fig*. *10.a-10.g*) mit überraschend geringem Aufwand neue Immuntoxin-Wirkstoffe mit einem breiten Spektrum an Zielzellspezifitäten zu erzeugen. Die Verwendung kurzer Peptide als Targetingmodule, die insbesondere zur gezielten Bindung an T-Zell-Rezeptoren eingesetzt werden können, ermöglicht eine direkte chemische Synthese der dazu jeweils benötigten DNA-Sequenz (die Bestandteil der erfindungsgemäßen Nukleinsäure wird), was wesentlich zeitsparender als z. B. die Konstruktion geeigneter Antikörper ist. Ein weiterer Vorteil des erfindungsgemäßen Konzepts zur Herstellung von neuen hochspezifischen Toxinen gegenüber der Konstruktion von Immuntoxinen mittels bispezifischer Antikörper besteht in der kovalenten Verbindung der Module mittels Prozessierungsmodulen, die eine extrazelluläre Dissoziation der Module verhindern sowie die intrazelluläre Freisetzung des Toxins ermöglichen. Des weiteren wurde erfindungsgemäß gefunden, daß sich das natürliche Propeptid des Mistellektins auf Grund seiner proteasesensitiven Eigenschaften, welche für die Propeptide anderer Typ II-RIPs bisher nicht beschrieben worden sind, hervorragend als Quelle für geeignete Prozessierungsmodule für die Konstruktion der erfindungsgemäßen Fusionsproteine eignet. Überraschend ist an diesem Befund insbesondere, daß Prozessierungsmodule pflanzlichen Ursprungs auch von nicht-pflanzlichen Proteasen erkannt werden, was ihren universellen Einsatz ermöglicht.

Der Begriff "pflanzlichen Ursprungs" bedeutet im Sinne dieser Erfindung eine Peptidsequenz, die durch ein Nukleinsäuremolekül codiert wird, das zu Bereichen des pflanzlichen Genoms homolog ist oder ein Bestandteil davon ist, wobei eine Homologie der Nukleinsäuremoleküle durch Hybridisierung unter stringenten Bedingungen gegeben ist.

Ein weiterer Vorteil der Erfindung ist, daß bei der Anwendung der erfindungsgemäßen Fusionsproteine keine Probleme durch diverse Schutzimpfungen auftreten, wie es bei Immun- und Mitotoxinen auf Basis bakterieller Toxine der Fall ist. Verbesserte Eigenschaften zeigt rMLA als Effektormodul der erfindungsgemäßen Fusionsproteine gegenüber dem bisher zur Konstruktion von Immuntoxinen am häufigsten verwendeten Ricin A. Beim direkten Vergleich sind chemisch gekoppelte Immuntoxine auf MLA-Basis um ein vielfaches wirksamer als diejenigen auf Ricin A-Basis. Dazu kommt, daß Ricin A sowie Immuntoxine auf Ricin A-Basis starke Nebenwirkungen durch ihre unspezifische Toxizität zeigen, was für MLA bisher nicht beschrieben worden ist.

Ein anderer Vorzug der erfindungsgemäßen Fusionsproteine ist die Möglichkeit deren rekombinanter Darstellung, die vorzugsweise in *E. coli* erfolgt. Diese bevorzugte Ausführungsform der erfindungsgemäßen Fusionsproteine ist somit frei von Glycosylierungen und wird daher nicht, wie die aus der Pflanze gewonnenen Toxine, von den Glycosidrezeptoren der Leberzellen gebunden. Dies führt zu weniger Leberschäden bei gleichzeitig verlängerter Halbwertszeit im Blut und stellt daher eine starke Verbesserung der therapeutischen Möglichkeiten dar. Pflanzliche Toxine sind nämlich vorwiegend mit endständigen Mannose-Resten glykosyliert, was zu einem raschen Abbau in der Leber führt. Ein großer Vorteil der rekombinanten Darstellung von Fusionsproteinen, z. B. in *E. coli* ist, daß diese Proteine keine Glykosylierung aufweisen, wodurch sich die unspezifische Toxizität pflanzlicher Toxine auf nicht-parenchymale Hepatozyten (Skilleter et al., 1985; Magnusson et al., 1993) verringert und gleichzeitig die therapeutische Halbwertszeit verlängert wird (Vitetta et al., 1993). Somit ergeben sich für die Verwendung des erfindungsgemäßen Fusionsproteins beispielsweise zur zielgerichteten Inaktivierung von pathologischen Immunzellen des Blutes eine breite Palette von Vorteilen gegenüber den bisher bekannten Toxinen. Die enormen Vorzüge dieser Eigenschaften der erfindungsgemäßen Fusionsproteine vor allem im medizinischen Bereich liegen für den Fachmann auf der Hand.

Ein weiterer gewichtiger Vorteil der erfindungsgemäßen Fusionsproteine ist, daß sie verglichen mit herkömmlichen Immuntoxinen ein wesentlich geringeres Molekulargewicht aufweisen können, was zum einen die Gefahr von Immunreaktionen absenkt und zum anderen die Verteilung des Wirkstoffes in dichtem Zellgewebe verbessert.

In einer bevorzugten Ausführungsform betrifft die Erfindung ein Nukleinsäuremolekül, wobei
(a) die Mistellektin A-Kette oder ein intrazellulär cytotoxisch wirkendes Fragment oder Derivat hiervon von einem Nukleinsäuremolekül codiert wird, ausgewählt aus der Gruppe bestehend aus:
   (i) Nukleinsäuremolekülen, die eine Nukleotidsequenz umfassen, die die in Fig. 11.a angegebene Aminosäuresequenz oder ein Fragment davon codieren;
   (ii) Nukleinsäuremolekülen, die die in Fig. 11.a angegebene NuMeotidsequenz oder ein Fragment davon umfassen; und
   (iii) Nukleinsäuremolekülen, die mit einem Nukleinsäuremolekül aus (i) oder (ii) unter stringenten Bedingungen hybridisieren; und
   (iv) Nukleinsäuremolekülen, die zu den in (iii) genannten Nukleinsäuremolekülen entsprechend dem genetischen Code degeneriert sind; und/oder
(b) das Mistellektin-Propeptid oder ein Fragment oder Derivat davon, das eine Erkennungssequenz für eine Protease aufweist, von einem Nukleinsäuremolekül codiert wird, ausgewählt aus der Gruppe bestehend aus:
   (i) Nukleinsäuremolekülen, die eine Nukleotidsequenz umfassen, die die in Fig. 11.c angegebene Aminosäuresequenz oder ein Fragment davon codieren;
   (ii) Nukleinsäuremolekülen, die die in Fig. 11.c angegebene Nukleotidsequenz oder ein Fragment davon umfassen;
   (iii) Nukleinsäuremolekülen, die mit einem Nukleinsäuremolekül aus (i) oder (ii) unter stringenten Bedingungen hybridisieren; und
   (iv) Nukleinsäuremolekülen, die zu den in (iii) genannten Nukleinsäuremolekülen entsprechend dem genetischen Code degeneriert sind.

Hybridisierung im Sinne der Erfindung bedeutet Hybridisierung unter konventionellen Hybridisierungsbedingungen. Vorzugsweise findet die Hybridisierung unter stringenten Bedingungen statt. Derartige Bedingungen sind z.B. in Sambrook et al., "Molecular Cloning, A Laboratory Handbook", CSH Press, Cold Spring Harbor; 1989 oder in Hames und Higgins "Nucleic acid hybridisation", IRL Press, Oxford, 1985 beschrieben. Solche Bedingungen werden beispielsweise in einen Hybridisierungspuffer erreicht, der 0,1 x SSC und 0,1% SDS aufweist, wobei die Hybridisierung und ggf. nachgeordnete Waschvorgänge (Waschpuffer enthält ggf. ebenfalls 0,1 x SSC und 0,1% SDS) vorzugsweise bei etwa 65°C stattfinden.

In einer weiteren Ausführungsform betrifft die Erfindung ein Nukleinsäuremolekül, wobei
das Effektormodul die Mistellektin A-Kette oder ein intrazellulär wirkendes Fragment oder Derivat davon umfasst.

Die vorgenannten Devivate können natürlich auftretende oder künstlich, z.B durch rekombinante DNA-Techniken erzeugte Derivate sein. Dazu gehören auch Moleküle, die sich von den vorgenannten Nukleinsäuremolekülen durch Degeneration des genetischen Codes unterscheiden. Selbstverständlich fallen posttranslationale oder erst später nach der Herstellung des Fusionsproteins vorgenommene Veränderungen der vorgenannten Effektormodute und/oder Prozessierungsmodule unter den Begriff Derivate, solange diese Derivate eine gleiche oder ähnliche Aktivität und/oder Funktion wie die vorgenannten Effektormodule und/oder Prozessierungsmodule aufweisen.

In einer weiteren bevorzugten Ausführungsform betrifft die Erfindung ein Nukleinsäuremolekül, wobei das Fusionsprotein ferner die folgende Komponente aufweist:
ein Modulatormodul, das kovalent mit dem Prozessierungsmodul, dem Effektormodul und/oder mit dem Targetingmodul verbunden ist, und das die intrazelluläre toxische Wirkung des Effektormoduls moduliert.

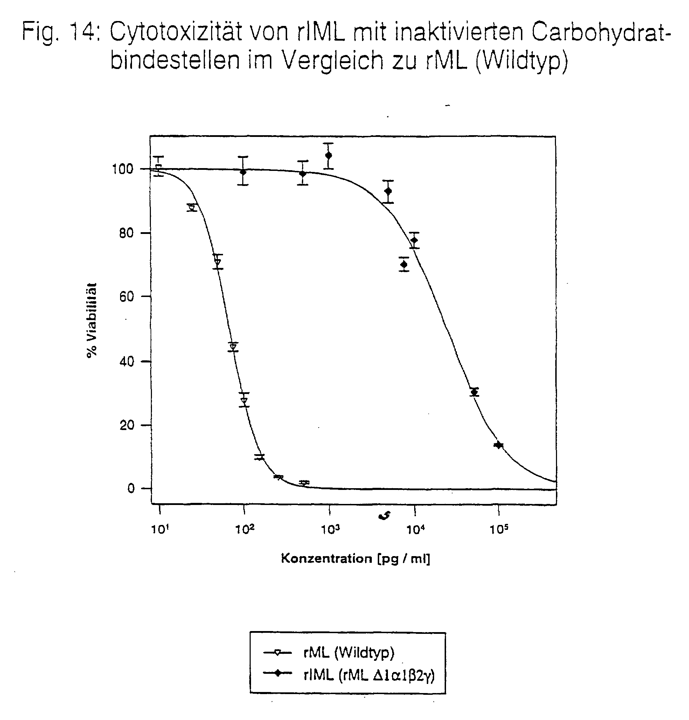

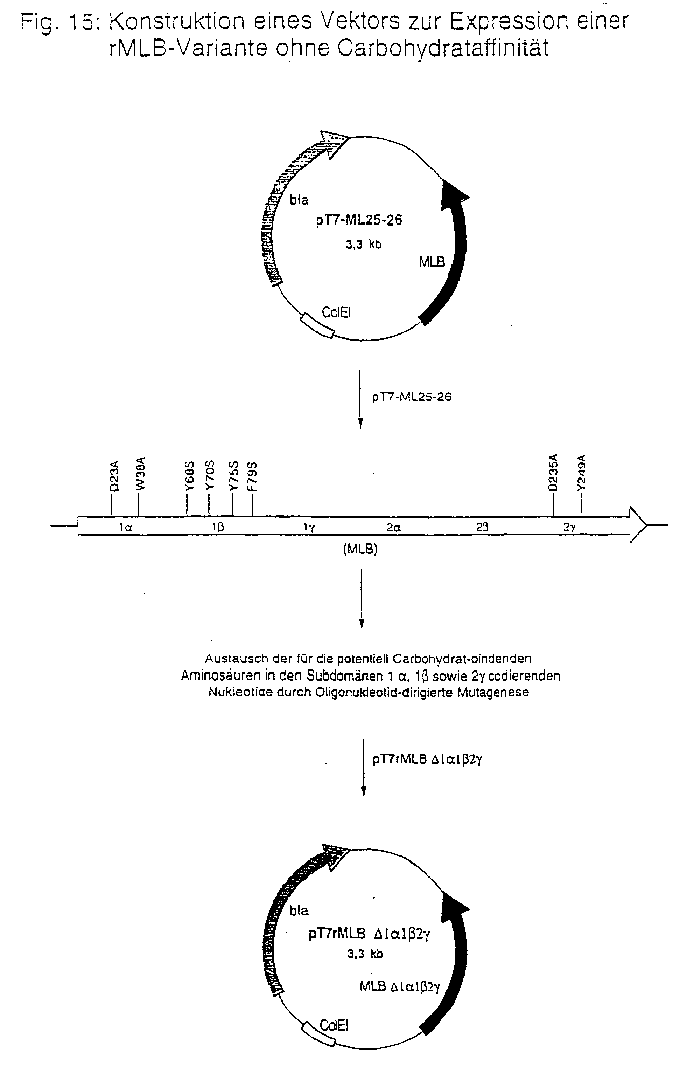

Als "Modulatormodul" werden erfindungsgemäß alle Polypeptidsequenzen verstanden, die in der Lage sind, die Entfaltung der cytotoxischen Wirkung eines Effektormoduls intrazellulär zu modulieren und die mit mindestens einem weiteren Modul des erfindungsgemäßen Fusionsproteins vorzugsweise durch ein die beiden Module verbindendes Prozessierungsmodul auf genetischer Ebene verbunden sind. Geeignete Modulatormodule können beispielsweise bei der Membrantranslakation assistierende Komponenten sein oder solche, die an intrazellulären Transportmechanismen partizipieren. Dabei liegt die gewünschte Modulation vorzugsweise in einer Verstärkung der zelltypspezifischen Wirksamkeit oder einer Vermeidung von unspezifischer Toxizität. Im Falle von rMLA wurde gefunden, daß diese Anforderungen von der rekombinanten B-Domäne des Mistellektins (rMLB) erfüllt werden, die eine Erhöhung der Toxizität des Effektormoduls durch aktive Unterstützung seiner Translokation vom endoplasmatischen Retikulum ins Cytoplasma der Zelle bewirkt. In der Vergangenheit konnte bereits gezeigt werden, daß durch Verwendung von Typ II RIPs an Stelle von Typ I RIPs zur Herstellung von z. B. antitumoralen Agenzien der cytotoxische Effekt dieser Substanzklasse um Größenordnungen gesteigert werden konnte, daß jedoch der daraus erhoffte therapeutische Nutzen solcher Präparate durch die auftretenden schwersten Nebenwirkungen letztlich nicht erzielt werden konnte. Einen möglichen Ausweg aus diesem Dilemma zeigen Bestrebungen, die Zuckerbindestellen der Ricin B-Kette nach der Kopplung an Antikörper durch chemische Derivatisierung zu inaktivieren - sogenanntes "blocked Ricin" (Shah et al., 1993) - was das Problem aber aufgrund der nach wie vor auftretenden starken Nebenwirkungen keineswegs gelöst hat. In einer erfinderungsgemäßen besonders bevorzugten Ausführungsform wird erstmals der Weg gegangen, die für die Zuckerbindung verantwortlichen Aminosäuren, unter Anwendung molekularbiologischer Verfahren, gegen diesbezüglich biologisch nicht funktionelle (funktionell inerte) Aminosäuren auszutauschen. Für das dem Mistellektin ähnliche Ricin sind seit längerem zwei Zuckerbindestellen in der 1α und 2γ Subdomäne der B-Kette aus der Literatur bekannt (Rutenber et al., 1987; Vitetta et al., 1990; Swimmer et al., 1992; Lehar et al., 1994). Die aufgrund dieser Anhaltspunkte erfindungsgemäß durchgeführten Versuche, die Carbohydrataffinität des rekombinanten Mistellektins zu inaktivieren haben ergeben, daß die für Ricin beschriebenen Zuckerbindestellen auch im Mistellektin zu finden sind. Überraschenderweise hat sich jedoch gezeigt, daß die Austausche der analogen, für Ricin beschriebenen Aminosäuren, im Falle des Mistellektins die Zuckerbindung nicht ausschalten, sondern lediglich um den Faktor 5 abschwächen konnten. Eine darauffolgende eingehendere Analyse der Kristallstruktur von Ricin B auf die Anwesenheit weiterer kryptischer Zuckerbindestellen durch computergestützte Kraftfeldberechnungen hat Hinweise auf die Anwesenheit einer potentiellen dritten Zuckerbindestelle - sowie für Laktose als auch für N-Acetyl-Neuraminsäure - in der 1β-Subdomäne geliefert. In der Literatur wurde für Ricin B eine dritte Zuckerbindestelle - dort ebenfalls In der 1β-Domäne - unter Beteiligung einer einzigen Aminosäure berichtet (Frankel et al., 1996), was diese Annahmen zusätzlich bestätigt. Nach Substitution der nach durchgeführten Berechnungen vier vermuttich an der Carbohydratbindung beteiligten Aminosäuren der 1β-Domäne des rekombinanten Mistellektins, zusätzlich zu den Austauschen in der 1α und 2γ Domäne (*Bsp.* 7, *Fig. 15*), konnte überraschenderweise tatsächlich ein nahezu vollständiger Verlust der Fähigkeit der B-Ketten-Variante "rMLB Δ1α1β2γ" zur Bindung an eine Laktosyl-Agarose Affinitätsmatrix beobachtet werden. Des weiteren zeigt rMLB Δ1α1β2γ (rIMLB) nicht nur die gleiche Faltungskompetenz wie die Wildtypsequenz, sondern auch nach wie vor die Fähigkeit zur kovalenten Assoziation mit der rekombinanten Mistellektin A-Kette (*Bsp. 8.c*). *Fig*. *13* zeigt eine Western-Blot Analyse der *in vitro* Assoziation von rMLB Δ1α1β2γ-mit rMLA unter immunochemischer Detektion mit monoklonalen Antikörpern gegen beide Einzelketten auf Höhe des erwarteten Molekulargewichts des Holo-Toxins von ca. 60 kDa. Die Cytotoxizität des so erhaltenen, nicht-carbohydratbindenden Holo-Toxins (rIML) gegenüber der humanen lymphatischen Zellinie MOLT-4 ergibt 50 % Viabilität bei einer rIML-Konzentration von 25 ng/ml. Dies entspricht im Vergleich zu 70 pg/ml im Falle der Verwendung von rML einer Abschwächung der unspezifischen *in vitro* - Toxizität um den Faktor 350 (*Bsp. 9. Fig. 14*).
Die Verfügbarkeit eines derart modifizierten Modulatormoduls (rIMLB), eröffnet nun erstmals die Möglichkeit zur rekombinanten Darstellung von Anti-Immunzell-Toxinen, bei denen die Aussicht besteht, daß die fatalen Nebenwirkungen der bislang verfügbaren Substanzen auf der Basis der natürlichen Typ II RIPs durch Verwendung von rIMLB auf ein vertretbares Maß gesenkt werden können. Zur Gewährleistung einer Targetingmodut-vermittelten Spezifität kann im Falle-von rMLB die Carbohydratbindung durch gerichteten Aminosäureaustausch, beispielsweise durch die Austausche D23 zu A, W38 zu A, D235 zu A, Y249 zu A, Y68 zu S, Y70 zu S, Y75 zu S, F79 zu S (die Nomenklatur bezieht sich auf die Aminosäuresequenz der rMLB gemäß *Fig. 11b* mit D1 als N-terminaler Aminosäure) minimiert werden.

In einer weiteren bevorzugten Ausführungsform betrifft die Erfindung ein Nukleinsäuremolekül, wobei das Modulatormodul oder ein Fragment hiervon, das die biologische Aktivität des Mistellektin B-Kette besitzt von einem Nukleinsäuremolekül codiert wird, ausgewählt aus der Gruppe bestehend aus:
(i) Nukleinsäuremolekülen, die eine Nukleotidsequenz umfassen, die die in Fig. 11.b angegebene Aminosäuresequenz oder ein Fragment davon codiert, wobei das Fragment die biologische Aktivität der Mistellektin B-Kette besitzt;
(ii) Nukleinsäuremolekülen, die die in Fig. 11.b angegebene Nukleotidsequenz oder ein Fragment davon umfassen, wobei das Fragment eine Aminosäuresequenz codiert, die die biologische Aktivität der Mistellektin B-Kette besitzt;
(iii) Nukleinsäuremolekülen, die mit einem Nukleinsäuremolekül aus (i) oder (ii) unter stringenten Bedingungen hybridisieren; und
(iv) Nukleinsäuremolekülen, die zu den in (iii) genannten Nukleinsäuremolekülen entsprechend dem genetische Code degeneriert sind.

In einer weiteren bevorzugten Ausführungsform betrifft die Erfindung ein Nukleinsäuremolekül, wobei das Modulatormodul die vorgenannte modulierende Aktivität besitzt und ein Allel oder Derivat der vorgenannten Mistellektin B-Kette durch Aminosäure-Deletion, Substitution, Insertion, Addition und/oder Austausche umfaßt.

Als weitere Modulatomzodule im Sinne dieser Erfindung dienen kurze Peptidfragmente wie z. B. die Peptide "KDEL" oder "HDEL". Dabei handelt es sich um Signalpeptide, die den aktiven retrograden Transport von Proteinen in Richtung endoplasmatisches Reticulum vermitteln, was sich zu einer Erhöhung der Toxizität aufgenommener Effektormodule ausnutzen läßt (Wales et al., 1993). Ebenfalls als Modulatormodule zu klassifizieren sind erfindungsgemäß Polypeptidsequenzen, die die katalytische Aktivität eines Effektormoduls außerhalb einer Zelle neutralisiert halten. Als Beispiel zu nennen ist das Propeptid des Mistellektins, welches die katalytische Aktivität von rMLA inaktiviert und erst im Zuge einer intrazellulären Prozessierung in prälysosomalen Zellkompartimenten die katalytische Aktivität von rMLA freigibt. Dies bringt den Vorteil einer drastisch verringerten unspezifischen Toxizität von im Blut zirkulierenden Fusionsproteinen mit sich.

Der Modulation der Toxizität durch ein Modulatormodul kommt hohe Bedeutung zu. So kann es wünschenswert sein, in Zielzellen die Toxizität eines Effektormoduls zu reduzieren, um vorteilhaftere Inierferenzen mit der Zielzelle zu erreichen. Beispielsweise kann ein langsames Abtöten von Zielzellen erwünscht sein, um Ausschüttungen potentiell schädlicher zellulärer Komponenten in den Organismus zu verhindern. So können nachteilige Reaktionen wie Sofort-Typ-Überreaktionen oder anaphytaktische Schocks verhindert werden. Auch ist durch Modulation der toxischen Effekte eine Induktion von zellulären Programmabläufen wie der Apoptose möglich. Die Apoptose ist ein natürlicher Mechanismus der Monaten Selektion und damit für das umliegende Gewebe und den Gesamtorganismus eine vergleichsweise sanfte Methode der spezifischen Eliminierung von pathologischen Zellen.

Im Zusammenhang mit dieser Ausführungsform wurde erfindungsgemäß gefunden, daß rMLB die Toxizität von rMLA modulieren kann, wodurch die Möglichkeit besteht, die Toxizität der erfindungsgemäßen Fusionsproteine gezielt zu beeinflussen. Dieser Befund ist medizinisch von allergrößter Bedeutung. Erstmals ist es nämlich möglich geworden, die Wirkung ein und desselben Immuntoxins in ein und derselben Zelle durch die Wahl eines geeigneten Modulators zu variieren. Der Fachmann geht selbstverständlich davon aus, daß die modulierende Wirkung der rMLB-Kette auch auf andere Toxine, beispielsweise auf solche vom RIP I- oder RIP II-Typ wirkt. Auf der Basis der Kenntnis der modulierenden Wirkung der rMLB-Kette ist der Fachmann ohne weiteres in der Lage, die modulierende Wirkung anderer zuckerbindender Moleküle, beispielsweise solcher, die natürlicherweise in Typ II-RIPs vorkommen, zu testen. Die Eigenschaft der Mistellektin B-Kette über die Zuckerbindung hinaus modulierend bei Aufnahme und Aktivierung von Effektormolekülen zu wirken, läßt erwarten, daß zumindest auch andere Typ II RIP B-Ketten pflanzlichen Ursprungs ein ähnliches Eigenschaftenprofil haben. Derartige Modulatoren sind im Sinne der Erfindung ebenfalls vorteilhaft zu verwenden. Derartige Modulatoren sind ebenfalls von der vorliegenden Erfindung umfaßt.

In einer weiteren bevorzugten Ausführungsform der Erfindung weist das Nukleinsäuremolekül für das Fusionsprotein ferner die folgende Komponente auf:
(e) ein Affinitätsmodul zur Aufreinigung, das kovalent mit dem Effektormodul, dem Prozessierungsmodul, den Targetingmodul und/oder den Modulatormodul verbunden ist.

Als Affinitätsmodule werden Komponenten der erfindungsgemäßen Fusionsproteine bezeichnet, die nicht auf einen therapeutischen Effekt abzielen, sondern auf die Möglichkeit, die erfindungsgemäßen Fusionsproteine z.B. über affinitätschromatographische Verfahren aufzureinigen. Dem Fachmann sind selbstverständlich auch andere Verfahren wie lonenaustausch-, Geipermeations- oder Hydrophobe-Interaktions-Chromatographie geläufig, mit denen er die Fusionsproteine aufreinigen kann. Dagegen ist es unter Verwendung der Affinitätsmodule möglich, mit Hilfe affinität schromatographischer Verfahren vorzugsweise homogene oder im wesentlichen homogene Wirkstoffe zu erhalten. Idealerweise handelt es sich bei den Affinitätsmodulen um kurze Peptidfragmente, wie z. B. eine Hexahistidinsequenz mit Affinität zu Sepharose-Chelatkomplexen, die vorzugsweise an der Sequenzperipherie fusioniert werden (*Fig. 10.a* - *10.g*). Diese Ausführungsform der Erfindung ermöglicht vor allem eine schnelle und unproblematische Aufreinigung des erfindungsgemäßen Fusionsproteins.

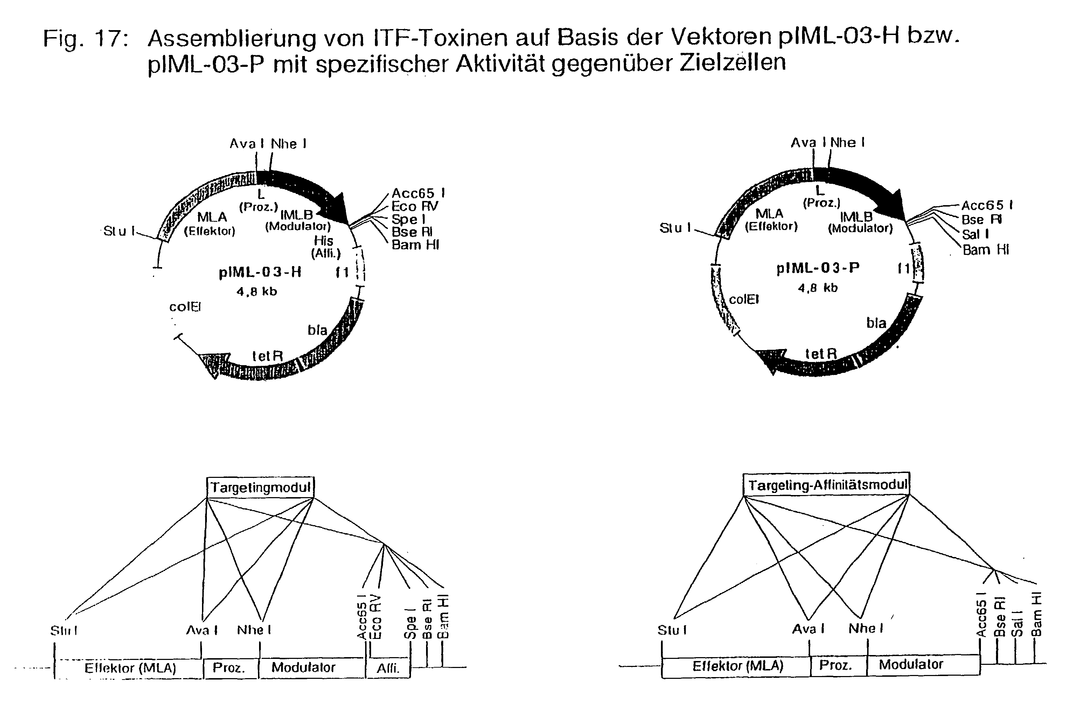

Bedingt durch die rekombinante Herstellung des Fusionsproteins können die in den vorstehend genannten Ausführungsformen genannten Module durch beliebige Kombination der entsprechenden Nukleinsäuresequenzen in der jeweils gewünschten Reihenfolge angeordnet werden. Der Fachmann ist aufgrund seines Fachwissens in der Lage, entsprechende rekombinante Nukleinsäuremoleküle, beispielsweise durch Einbau geeigneter Restriktionsspaltstellen, herzustellen. Eine Auswahl von Kombinationsmöglichkeiten bzw. Anordnungen ist in Fig. *10.a. - 10.g* gezeigt. Das periplasmatische Zellkompartiment von *E. coli* kommt den Anforderungen eines disulfidbrükken-haltigen Proteins an die zur Ausbildung einer funktionellen Tertiärstruktur erforderlich Mikroumgebung am nächsten. Von daher wurde, wie im *Beispiel 10* ausführlich beschrieben, ein periplasmatisches modulares Expressionssystem konstruiert, daß die einfache Realisierung aller erforderlichen Anordnungen der einzelnen Module in den ITF-Expressionsvektoren erlaubt (*Fig. 17*).

In einer weiteren bevorzugten Ausführungsform des erfindungsgemäßen Nukleinsäuremoleküls erkennt das Targetingmodul spezifisch eine Zelle des Immunsystems, eine Tumorzelle oder eine Zelle des Nervensystems.

Ein Schwerpunkt derzeitiger Forschungen liegt im Bereich der Rezeptorausstattung von Immunzellen, was zu einer schnell wachsenden Zahl von bekannten Rezeptoren sowie deren Liganden führt. Aufgrund des modularen Aufbaus der erfindungsgemäßen Fusionsproteine lassen sich neue Erkenntnisse auf diesem Gebiet schneller als bisher zur Erzeugung therapeutisch nutzbarer Wirkstoffe umsetzen. Dieser Aspekt gewinnt besondere Bedeutung in der Entwicklung von individuell auf Patienten abgestimmten Präparaten. Aussichtsreiche Anwendungsmöglichkeiten solcher modular aufgebauten Fusionsproteine bestehen in der Behandlung von Funktionsstörungen des Nerven- und des Immunsystems. Bei diesen Zellen handelt es um vorwiegend im Blut- oder im Lymphsystem zirkulierende Zellen, die eine gute physikalische Zugänglichkeit für die erfindungsgemäßen Fusionsproteine bieten. Die Probleme einer schlechten Tumorpenetration durch Immuntoxine sind somit nicht gegeben. Außerdem ist gerade für Zellen des Immunsystems die Apoptose ein natürlicher Mechanismus der klonalen Expansionskontrolle, so daß die Verwendung beispielsweise von rMLA als Effektormodul die natürliche Suszeptibilität der Immunzellen für Apoptose vorteilhaft nutzt (sh. auch Bussing et al., 1996). Weiterhin eignen sich die Vorteile des modularen Systems besonders zur Behandlung von Allergien, da hierfür ein großes Repertoire an verschiedenen patientenspezifischen Targetingmodulen benötigt wird. Beispielsweise kommt es bei Allergien vom Soforttyp zu einem von T_{H}2-Zellen induziertem B-Zell-Klassensprung auf die allergene IgE-Produktion, im Gegensatz zur T_{H}1-Zellen vermittelten IgG-Antwort. Ein Therapieansatz unter Verwendung der erfindungsgemäßen Fusionsproteine besteht darin, allergene, normalerweise MHCII-präsentierte Peptide als Targetingmodule einzusetzen und so selektiv die responsiven T_{H}2-Zellen aus dem Körper eines Patienten zu eliminieren. Nach dem gleichen Prinzip ist eine Therapie von Autoimmunerkrankungen möglich. Die derzeitig angewendeten Therapien der MS als Beispiel von Autoimmunerkrankungen umfassen vielfältige Eingriffe in die Regulation des Immunsystems (Hohlfeld, 1997). Bei der ursächlichen Behandlung von Autoimmunerkrankungen steht die Depletion der jeweiligen Autoantigen-spezifischen T-Zellen im Vordergrund. Ein derzeit favorisierter Ansatz beruht auf der Expression eines bestimmten TCR-Subtyps, z.B. konnte bei der MS durch eine Vakzinierung mit dem Vβ5.2 Peptid die Aktivität der MBP-reaktiven T-Zellen moduliert werden (Vandenbark et al., 1996). Das Prinzip dieser Methode beruht vor allem auf einer Verschiebung der Cytokin-Antwort von Th1 nach Th2, also von proinflammatorischen zu inhibitorischen Cytokinen. Es wird also letzlich eine systemische Wirkung hervorgerufen.
Im Falle der demyelinisierenden Neuropathie (Guillain Barre Syndrom, Neuritis) ist das Autoantigen das Myelin des peripheren Nervensystems (P2). Im Tiermodell der Neuritis EAN konnte der AS-Bereich 53-78 als neuritogenes Peptid identifiziert werden. Die EAN kann entweder aktiv durch das Neuritogen P2 direkt oder durch den adoptiven Transfer von neuritogenen T-Zellen, die aus erkrankten Ratten isoliert wurden, induziert werden.
Das rekombinante P2-Peptid wurde bereits erfolgreich zur Linderung der EAN in der Ratte eingesetzt, wobei die Apoptose-induzierende Wirkung von P2 (Dosis 100 µg täglich i.v.) ausgenutzt wurde (Weishaupt et al., 1997).
Vorraussetzung zur Linderung eines Krankheitsschubes im Patienten ist jedoch, daß eine entsprechend hohe, Apoptose-induzierende Konzentration des Antigens zu den autoreaktiven T-Zellen in der Peripherie oder zum Ort der Autoimmunreaktion gelangt. Bei Bindung geringer Mengen Antigen an T-Zellen findet natürlicherweise eine Proliferation statt. Durch die Kopplung eines Toxins an die spezifische Erkennungssequenz der neuritogenen T-Zellen kann somit eine sichere T-Zell Eliminierung vermittelt werden, ohne die Gefahr eines gegenteiligen stimulatorischen Effektes zu bergen. Auslöser beispielsweise der Multiplen Sklerose ist die Entstehung und Vermehrung von autoreaktiven T-Lymphozyten (Olive, 1995), die ein Abbauprodukt des "myelin-basic-proteins" - in der Mehrzahl der Fälle die Sequenz "VHFFKNIVTPRTP" - erkennen. Dies führt dazu, daß die Nervenzellen des Patienten vom körpereigenen Immunsystem angegriffen werden. Auch hier ist die Verwendung von krankheitsauslösenden Peptiden als Targetingmodul der Schlüssel zur Anwendung einer auf der Erfindung basierenden Therapieform. Eine weitere derartige Erkrankung ist Myasthenia Gravis, bei der es zu einer Autoimmunreaktion gegen Acetylcholinrezeptoren kommt. Ferner kommt eine Behandlung diverser Leukämien oder Neoplasien in Frage.

Somit ist in einer besonders bevorzugten Ausführungsform der Erfindung die Zielzelle eine Zelle des Immunsystems. Dies kann zum einen eine Zelle des unspezifischen Immunsystems wie zum anderen eine Zelle des spezifischen Immunsystems sein. Im letzteren Falle. kann es sich um B-Zellen oder T-Zellen, insbesondere T_{H}2-Zellen handeln. Ferner können auch entartete Zellen des Immunsystems Zielzellen sein. Auch Zellen, insbesondere entartete Zellen des Nervensystems, beispielsweise Nervenzellen, können bei Wahl geeigneter Targeting-Module Zielzellen sein.

In einer weiteren bevorzugten Ausführungsform des erfindungsgemäßen Nukleinsäuremoleküls ist das Affinitätsmodul eine Histidinsequenz, Thioredoxin, Strep-Tag, T7-Tag, FLAG-Tag, Maltose-Bindungs-Protein oder GFP (Green Fluorescent Protein). Das Affinitätsmodul ist hierbei eine Peptidsequenz, die sich durch eine Liganden-Bindungs-Spezifität oder durch das Vorhandensein geeigneter Epitope auszeichnet, die eine selektive Aufreinigung vorzugsweise durch affinitätschromatographische Verfahren, z. B. mittels immobilisierter Liganden oder immobilisierter Antikörper ermöglicht. Derartige Affinitätsmodule haben stets die Eigenschaft, hochspezifisch und mit hohen Bindungskonstanten Liganden zu binden, die ihrerseits als Liganden an vorzugsweise chromatographische Matrices gekoppelt sind. So können in Prozessen mit lediglich wenigen Stufen hochgradig gereinigte Fusionsproteine aus Lysaten oder Zellüberständen dargestellt werden.

Eine andere bevorzugte Ausführungsform der Erfindung betrifft ein Nukleinsäuremolekül, wobei das Modulatormodul die Mistellektin B-Kette oder ein Fragment oder Derivat davon oder die Peptide KDEL oder HDEL umfaßt.
In dieser Ausführungsform werden beispielsweise die rMLB-Sequenzen durch Fragmente oder Derivate von rMLB ersetzt. Wie bereits im Zusammenhang mit der Verwendung der rMLA-Kette vorstehend diskutiert wurde, ist der Fachmann auf der Basis seines Fachwissens in der Lage, rekombinante Nukleinsäuren bereitzustellen, die derartige Fusionsproteine codieren. Hinsichtlich eines Tests, mit dem die Modulatorfunktion der Fragmente oder Derivate nachgewiesen werden kann, sei auf die nachfolgenden Beispiele verwiesen.

In einer besonders bevorzugten Ausführungsform des erfindungsgemäßen Nukieinsäuremoleküls weist die Mistellektin B-Kette einen Austausch in Aminosäureposition 23, 38, 68, 70, 75, 79, 235 oder 249 oder eine Kombination derartiger Austausche auf. Besonders bevorzugt ist erfindungsgemäß ferner die Ausführungsform, wobei der Austausch in Position D23 zu A, W38 zu A, D235 zu A, Y249 zu A, Y68 zu S, Y70 zu S, Y75 zu S, F79 zu S vorliegt (die Nomenklatur bezieht sich auf die Aminosäuresequenz der rMLB gemäß Fig. 11b mit D1 als N-terminaler Aminosäure).
Diese Ausführungsform ist vor allem deswegen bevorzugt, da die Aminosäurereste in den genannten Positionen an der Ausbildung von Zuckerbindungsstellen teilhaben, die Zucker oder Glycoproteine oder Glycolipide auf Zelloberflächen binden können.
Die Eliminierung der Zuckerbindungsstellen bewirkt, daß ein unspezifisches, zuckervermitteltes Andocken an nicht gewünschte Zellen unterbleibt. Die Häufigkeit, mit der das erfindungsgemäße Fusionsprotein tatsächlich den Ort der intendierten Wirkung erreicht, wird somit nochmals signifikant erhöht.

In einer weiteren bevorzugten Ausführungsform der Erfindung ist das Nukleinsäuremolekül DNA.

In einer anderen bevorzugten Ausführungsform des erfindungsgemäßen Nukleinsäuremoleküls ist dieses RNA.

Die Erfindung betrifft ferner einen Vektor, der ein erfindungsgemäßes Nukleinsäuremolekül enthält.
Die Konstruktion von geeigneten Vektoren für die Propagation und vorzugsweise die Expression der erfindungsgemäßen Nukleinsäure ist dem Fachmann geläufig. Sofern der Vektor für die Herstellung des Fusionsproteins eingesetzt wird, wird der Fachmann Wert darauf legen, daß er eine möglichst hohe Ausbeute an Fusionsprotein erzielt und dementsprechend beispielsweise einen starken Promoter in den Vektor einfügen. Andererseits und beispielsweise, wenn der Vektor Bestandteil eines Arzneimittels ist, kann es von Vorteil sein, daß die Expression der Nukleinsäuren erst in der Zielzelle eingeschaltet wird. In diesem Falle wird der Fachmann ein induzierbares Expressionssystem wählen. Der Vektor kann im Sinne dieser Erfindung auch mehr als eine erfindungsgemäße Nukleinsäure enthalten.

Für die Expression bzw. Propagierung des Vektors ist ferner ein geeigneter Wirt notwendig. Somit betrifft die Erfindung ferner einen Wirt, der mit dem erfindungsgemäßen Vektor transformiert ist oder ein erfindungsgemäßes Nukleinsäuremolekül enthält. Von der Erfindung sind auch solche Wirte umfaßt, die mehrere erfindungsgemäße Vektoren und/oder Nukleinsäuremoleküle enthalten.
Transformationsverfahren sind im Stand der Technik für die verschiedensten Zelltypen und Wirtsorganismen beschrieben und können vom Fachmann nach geeigneten Gesichtspunkten ausgewählt werden.

Besonders bevorzugt sind erfindungsgemäß die folgenden prokaryontische Wirte: E. coli, Bacillus subtilis oder Streptomyces coelicolor und die folgenden eukaryontischer Wirte: Saccharomyces sp., Aspergillus sp., Spodoptera sp. oder Pichia pastoris. Im Falle von eukaryontischen Expressionssystemen ist die Verwendung von Modulatormodulen besonders vorteilhaft, da eine Schädigung des Wirtes durch das Expressionsprodukt mit Hilfe eines Modulatormoduls verhindert werden kann.

Die Erfindung betrifft ferner ein Fusionsprotein, das von einem erfindungsgemäßen Nukleinsäuremolekül codiert oder von einem erfindungsgemäßen Wirt produziert wird.
Die Vorzüge und Anwendungsmöglichkeiten des erfindungsgemäßen Fusionsproteins sind bereits im Zusammenhang mit des verschiedenen Ausführugsformen des erfindungsgemäßen Nukleinsäuremoleküls diskutiert worden, auf die hiermit Bezug genommen wird.

Darüber hinaus betrifft die Erfindung Verfahren zur Herstellung des erfindungsgemäßen Fusionsproteins, wobei man einen erfindungsgemäßen Wirt unter geeigneten Bedingungen züchtet und das Fusionsprotein isoliert.
Das erfindungsgemäße Verfahren ist vorzugsweise ein mikrobiologisches, fermentatives Verfahren, das unter üblichen Bedingungen durchgeführt wird. Dabei kann das erzeugte Fusionsprotein aus dem Überstand oder aus dem Wirt nach dessen Aufschluß isoliert werden. Letztere Ausführungsform schließt auch das Denaturieren und Renaturieren des Fusionsproteins ein, sofern dieses beispielsweise in Bakterien in Form von Einschlußkörpern produziert wird.

Die pharmazeutischen Implikationen und die grundlegende Bedeutung der Erfindung für die Medizin ist bereits vorstehend erörtert worden. Demgemäß betrifft die Erfindung auch ein Arzneimittel, das ein erfindungsgemäßes Fusionsprotein und einen pharmazeutisch verträglichen Träger enthält.

Bisher beschriebene Versuche der Herstellung von Immuntoxinen unter Verwendung der A-Domäne des Mistellektins mußten bisher auf dem Wege der biochemischem Kopplung, z. B. mit SPDP, durchgeführt werden (Paprocka et al., 1992). In zwei derartigen Studien (Tonevitsky et al., 1991, 1996) wurde die Wirksamkeit der erhaltenen nMLA-Immuntoxine mit den entsprechenden Ricin A-Immuntoxinen verglichen, wobei die nMLA-Immuntoxine eine 15 - 80 mal höhere Wirksamkeit als die auf Ricin A-basierenden Immuntoxine gezeigt haben. Durch die bisher nicht zum Stand der Technik gehörende Möglichkeit, auf rekombinant herstellbare Mistellektinkomponenten zurückzugreifen, ist die Herstellung des erfindungsgemäßen Arzneimittels möglich geworden.

Die Form und Dosis der Verabreichung des erfindungsgemäßen Arzneimittels bleibt dem behandelden Arzt überlassen, der insbesondere mit dem Krankheitszustand des Patienten vertraut ist. Weitere Faktoren, die die Form und Dosis der Verabreichung beeinflussen können, sind Alter, Geschlecht, Körperoberfläche und Gewicht des Patienten sowie die Verabreichungsroute. Pharmazeutisch verträgliche Träger sind im Stand der Technik bekannt und umfassen phosphatgepufferte Kochsalzlösungen, Wasser, Emulsionen wie Öl/Wasser-Emulsionen etc. Arzneimittelzusammensetzungen, die derartige Träger enthalten, sind nach konventionellen Verfahren formulierbar. Die Verabreichung des Arzneimittels kann systemisch oder lokal und in der Regel parenteral erfolgen. Übliche Wege der Verabreichung sind z.B. intraperitoneal, intravenös, subcutan, intramuskulär, topisch oder intradermal, wobei die intravenöse Verabreichung bevorzugt ist. Bevorzugte Dosierungen für die intravenöse Behandlung liegen im Bereich von 1ng wirksame Substanz pro kg Körpergewicht bis 500 µg/kg. Für die *ex vivo* Anwendung werden Dosierungen im Bereich von 1pg/ml bis 500 ng/ml bevorzugt eingesetzt. Diese Dosen werden vorzugsweise täglich verabreicht. Sofern die Behandlung eine kontinuierliche Infusion erforderlich macht, liegen die Dosierungen ebenfalls in den o.g. Bereichen.

Ferner betrifft die Erfindung also ein Arzneimittel, enthaltend
ein Fusionsprotein, das von einem erfindungsgemäßen Nukleinsäuremolekül codiert wird, wobei das Fusionsprotein ein Effektor-, Prozessierungs-, Targeting- und gegebenenfalls ein Affinitätsmodul und/oder ein Modulatormodul umfaßt oder einen Vektor, der das entsprechende Nukleinsäuremolekül enthält, wobei der
Modulator die Mistellektin B-Kette oder ein Fragment oder Derivat davon, das die biologische Aktivität der Mistellektin B-Kette besitzt, umfasst.

Das Modulatormodul kann im erfindungsgemäßen Arzneimittel also kovalent mit den anderen Modulen verknüpft sein und so vom selben Vektor codiert werden wie diese Module oder es kann als separate Einheit auftreten und wird dann beispielsweise von einen zweiten Vektor codiert, vorzugsweise jedoch zusammen mit den anderen Modulen durch in einem einzigen Vektor vorhandene Sequenzen.

In der Ausführungsform, in der das Arzneimittel die genannten Polypeptide enthält, werden diese vorzugsweise vor der Arzneimittelformulierung als kovalent verknüpftes Fusionsprotein dargestellt. Somit wird in besonderem Maße sichergestellt, daß der Polypeptidkomplex, der sowohl das Effektor-, Prozessierungs- und Targetingmodul wie auch vorzugsweise das Modulatormodul aufweist, in ein und dieselbe Zielzelle aufgenommen wird. Sofern das Arzneimittel den/die erfindungsgemässen Vektoren enthält, werden in der Regel 10⁶ bis 10²² Kopien pro Vektor nach den vorstehend dargestellten Verabreichungsschemata appliziert. Die erfindungsgemässen Vektoren können auch gentherapeutisch eingesetzt werden. Verfahren zum gentherapeutischen Einsatz von Vektoren sind im Stand der Technik ebenfalls bekannt.
Die Ausführungsform, in der das Arzneimittel die Vektoren enthält, ist besonders dann von Vorteil, wenn nicht eine sofortige Toxinwirkung beabsichtigt ist. Dies kann beispielsweise dann der Fall sein, wenn das Arzneimittel als begleitende Therapie verabreicht wird. In dieser Ausführungsform wird die Zielzellenspezifität dadurch erreicht, daß man einen geeigneten Vektor, beispielsweise einen retroviralen Vektor einsetzt. Im Stand der Technik sind eine Reihe retroviraler Vektoren bekannt, die beispielsweise spezifisch für T-Zellen sind. Die Expression der Nukleinsäuren kann beispielsweise über temperatursensitive Promotoren erfolgen. In der Praxis kann der Patient beispielsweise über einen geeigneten Zeitraum einer Wärmequelle ausgesetzt werden, wodurch die Expression der Nukleinsäuren angeschaltet wird und das Toxin in der Zielzelle die gewünschte Wirkung entfaltet.

In einer bevorzugten Ausführungsform des vorstehend erörterten erfindungsgemäßen Arzneimittels ist der Modulator bzw. umfaßt das Modulatormodul die Mistellektin B-Kette oder ein Fragment oder Derivat davon.

Insbesondere bevorzugt ist dabei aus den oben genannten Gründen, daß die Mistellektin B-Kette einen Austausch in Aminosäureposition 23, 38, 68, 70, 75, 79, 235 oder 249 (die Nomenklatur bezieht sich auf die Aminosäuresequenz der rMLB gemäß Fig. 11.b mit D1 als N-terminale Aminosäure) oder eine Kombination derartiger Austausche aufweist, wobei der Austausch in Position 23 vorzugsweise ein Austausch D23 zu A, in Position 38 vorzugsweise W38 zu A, in Position 235 vorzugsweise D235 zu A, in Position 249 vorzugsweise Y249 zu A, in Position 68 vorzugsweise Y68 zu S, in Position 70 vorzugsweise Y70 zu S, in Position 75 vorzugsweise Y75 zu S und in Position 79 vorzugsweise F79 zu S ist. Besoders bevorzugt ist hier, wie auch in den nachfolgend diskutierten Ausführungsformen, die diese Austausche betreffen, daß mindestens zwei, vorzugsweise mindestens drei, vier, fünf, sechs, sieben und am stärksten bevorzugt 8 derartige Austausche vorliegen.

Die Erfindung betrifft femer einen Kit, enthaltend
einen Vektor, der ein erfindungsgemäßes Nukleinsäuremolekül enthält.

Mit dem erfindungsgemäßen Kit kann insbesondere die Effizienz der verschiedenen Module in verschiedenen/auf verschiedene Zielzellen *in vitro* untersucht werden. Exemplarisch für die *in vivo* Situation werden z.B. neoplastisch transformierte Zellen *in vitro* gezüchtet und mit den Vektoren transfektiert. Die Auswirkung der Expression der verschiedenen Module auf die Viabilität der transfektierten Zellen kann beispielsweise unter dem Mikroskop verfolgt werden. Somit liefert der erfindungsgemäße Kit wertvolle Ergebnisse für die Entwicklung von Arzneimitteln beispielsweise zur Tumortherapie.

In einer bevorzugten Ausführungsform ist der Modulator im erfindungsgemäßen Kit die Mistellektin B-Kette oder ein Fragment oder Derivat davon.

Besonders bevorzugt ist dabei, daß die Mistellektin B-Kette einen Austausch in Aminosäureposition 23, 38, 68, 70, 75, 79, 235 oder 249 oder eine Kombination derartiger Austausche aufweist, wobei der Austausch in Position 23 vorzugsweise ein Austausch D23 zu A, in Position 38 vorzugsweise W38 zu A, in Position 235 vorzugsweise D235 zu A, in Position 249 vorzugsweise Y249 zu A, in Position 68 vorzugsweise Y68 zu S, in Position 70 vorzugsweise Y70 zu S, in Position 75 vorzugsweise Y75 zu S und in Position 79 vorzugsweise F79 zu S ist.

Ferner betrifft die Erfindung die Verwendung der Mistellektin B-Kette oder eines Fragments oder Derivats davon zur Modulierung der Wirksamkeit eines intrazellulär aktiven Toxins.
Wie bereits vorstehend dargestellt, wird durch die vorliegende Erfindung erstmalig gezeigt, daß die zuckerbindende Komponente eines Typ II-RIP in der Lage ist, die cytotoxische Wirkung eines Toxins intrazellulär zu modulieren und insbesondere zu erhöhen. Es wird erfindungsgemäß davon ausgegangen, daß die z.B. Mistellektin B-Kette nicht nur die Toxizität der Mistellektin A-Kette moduliert, sondern auch die anderer Toxine, insbesondere solcher vom Typ I- oder Typ II-RIP. Der Fachmann kann vermittels der erfindungsgemäßen Lehre leicht feststellen, ob der Modulator die Toxizität eines interessierenden Toxins tatsächlich verändert. Insofern sind von der erfindungsgemäßen Verwendung alle intrazellulären Toxine und nicht nur die Mistellektin A-Kette umfaßt.

Bevorzugt ist erfindungsgemäß eine Verwendung, wobei das Toxin intrazellulär ein Spaltprodukt eines Fusionsproteins ist, das die folgenden Komponenten aufweist:
(a) ein Effektormodul, das das Toxin umfaßt;
(b) ein Prozessierungsmodul, das kovalent mit dem Effektormodul verbunden ist, und das eine Erkennungssequenz für eine Protease aufweist; und
(c) ein Targetingmodul, das kovalent mit dem Prozessierungsmodul verbunden ist, und das spezifisch an die Oberfläche einer Zelle bindet, wodurch die Internalisierung des Fusionsproteins in die Zelle vermittelt wird; und gegebenenfalls
(d) ein Affinitätsmodul, das kovalent mit dem Effektormodul, dem Prozessierungsmodul, dem Targetingmodul und/oder dem Modulatormodul verbunden ist.
Diese bevorzugte Ausführungsform macht zudem Gebrauch von dem erfindungsgemäßen und eingangs beschriebenen modularen Konzept. insofern hat diese Ausführungsform besondere praktische Vorteile bei der Entwicklung von Arzneimitten.

Besonders bevorzugt ist dabei eine Verwendung, wobei die Mistellektin B-Kette einen Austausch in Aminosäureposition 23, 38, 68, 70, 75, 79, 235 oder 249 oder eine Kombination derartiger Austausche aufweist und wobei der Austausch in Position 23 vorzugsweise ein Austausch D23 zu A, in Position 38 vorzugsweise W38 zu A, in Position 235 vorzugsweise D235 zu A, in Position 249 vorzugsweise Y249 zu A, in Position 68 vorzugsweise Y68 zu S, in Position 70 vorzugsweise Y70 zu S, in Position 75 vorzugsweise Y75 zu S und in Position 79 vorzugsweise F79 zu S ist.

Bevorzugt ist ferner eine Verwendung, bei das Toxin die A-Kette von Typ II RIPs (Mistellektin, Ricin, Abrin, Ebulin, Modeccin und Volkensin) oder von Typ I RIPs (Saporin, Gelonin, Agrostin, Asparin, Bryodin, Colocin, Crotin, Curzin, Dianthin, Luffin, Trichosanthin und Trichokirin), oder ein intrazelluär toxisches Fragment oder Derivat davon ist.

Darüber hinaus betrifft die Erfindung ein Verfahren zur *in vitro* Testung eines prospektiven Modulators, wobei man folgende Schritte durchführt:
(a) Transfektion einer Zielzelle mit einem Vektor, der ein Nukleinsäuremolekül enthält, das ein Effektor-, Prozessierungs-, Targeting- und gegebenenfalls Affinitätsmodul codiert;
(b) Transfektion einer Zielzelle mit einem Vektor, der eine Nukleinsäure enthält, die einen prospektiven Modulator codiert;
(c) Expression der Nukleinsäuren in der Zielzelle; und
(d) Messung der modulierenden Aktivität des prospektiven Modulators auf die Toxizität des Toxins.
Mit dem erfindungsgemäßen Verfahren lassen sich eine Vielzahl von prospektiven Modulatoren testen, die unterschiedlichsten Ursprungs sein können. Vorzugsweise sind die Modulatoren pflanzlichen Ursprungs. Das Verfahren kann in einer bevorzugten Ausführungsform auch dazu eingesetzt werden, den Einfluß von Veränderungen an einem Modulator zu testen. So kann beispielsweise ein Modulator durch rekombinante Techniken dergestalt verändert werden, daß er eine weitere, im natürlichen Zustand nicht vorhandene Domäne aufweist, die eine gewünschte biologische Funktion erfüllt. Mit dem erfindungsgemäßen Verfahren kann getestet werden, ob und inwieweit diese Veränderung die modulierenden Eigenschaften des Modulators beeinflußt. Selbstverständlich können auch andere, dem Fachmann geläufige Modifikationen am Modulator mit diesem Verfahren getestet werden. Der Fachmann kann geeignete Zielzellen gemäß seinen experimentellen Vorgaben auswählen.

Dem Fachmann ist es möglich, ein Nukleinsäuremolekül, das ein Effektor-, Prozessierungs-, Targeting- und gegebenenfalls Affinitätsmodul codiert, stabil oder transient in eine gewünschte Zielzelle einzubringen. Demgemäß betrifft die Erfindung ferner ein Verfahren zur *in vitro* Testung eines prospektiven Modulators, wobei man folgende Schritte durchführt:
(a) Transfektion einer Zielzelle, die ein Nukleinsäuremolekül enthält, das ein Effektor-, Prozessierungs-, Targeting- und gegebenenfalls Affinitätsmodul codiert, mit einem Vektor, der eine Nukleinsäure enthält, die einen prospektiven Modulator codiert;
(b) Expression der Nukleinsäuren in der Zielzelle; und
(c) Messung der modulierenden Aktivität des prospektiven Modulators auf die Toxizität des Toxins.
Schließlich betrifft die Erfindung ein Verfahren zur Herstellung eines Modulators, in dem dieselben Schritte wie in den vorstehend beschriebenen *in vitro* Testverfahren durchgeführt werden und dazu noch folgender Schritt:
(e) bzw.(d) Isolierung des Modulators.
Die Isolierung kann vorzugsweise nach Standardverfahren durchgeführt werden.

Bevor die Erfindung durch die Beispiele erläutert wird, werden allgemeine Gesichtspunkte dargestellt, wie die Erfindung technisch auf der Basis des allgemeinen Fachwissens umgesetzt werden kann:
Der modulare Charakter der Komponenten Effektormodul (E), Modulatormodul (M), Targetingmodul (T), Prozessingmodul (P) und Affinitätsmodul (A) wird üblicherweise durch Einführung von geeigneten Restriktionsschnittstellen, jeweils am N- und C-Terminus der entsprechenden Nukleinsäuremoleküle bzw. Gene, realisiert. Die Nukleinsäuresequenz des Effektormoduls, in der hier diskutierten Ausführungsform von rMLA, enthält N-terminal eine Erkennungssequenz der Restriktionsendonuklease Ndel, was die Möglichkeit zur N-terminalen Fusion des Effektormoduls mit Prozessierungsmodulen ermöglicht (*Beispiel 1*). C-terminale Fusionen werden z. B. durch eine Aval-Restriktionsschnittstelle ermöglicht (*Fig. 11.a*). In der für das Modulatormodul (vorzugsweise rMLB) codierenden Sequenz können beispielsweise die N-terminale Restriktionsschnittstellen Stul oder BspLU11l und die C-terminale Restriktionsschnittstelle EcoRV zur Genfusion mit weiteren Modulen genutzt werden (*Fig. 11.b*). Prozessierungsmodule, die z. B. aus dem rekombinanten Propeptid des Mistellektins erhalten werden können (*Fig. 11.c*), können aufgrund ihrer kurzen Sequenz in Form chemisch synthetisierter Genkassetten an die jeweils benötigten Restriktionsschnittstellen und an das jeweilige zielzellspezifische Proteaseprofil angepaßt werden, wobei letzteres die selektive Wirkung der erfindungsgemäßen Fusionsproteine noch erhöhen kann.

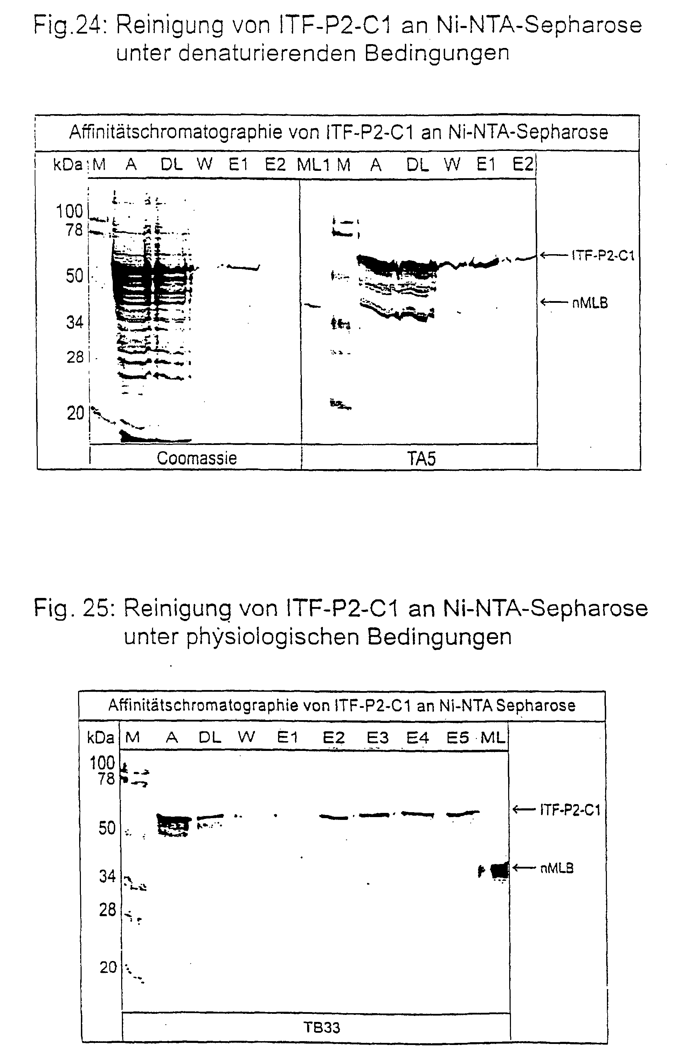

Die Bereitstellung der Fusionsproteine in hohen Reinheitsgraden erfolgt vorzugsweise durch einen oder mehrere Chromatographieschritte, vorzugsweise durch Affinitätschromatographien, die eine Anreicherung der Fusionsproteine beispielsweise unter Verwendung der Affinitätsmodule erlauben. Femer kann eine Selektion auf ein funktionelles Targetingmodul die weitere Aufreinigung ermöglichen. Die Reihenfolge der Aufreinigungsschritte kann dabei beliebig sein. In *Beispiel 3* wird die Anwendung eines Zweischritt-Reinigungsverfahrens ohne die Verwendung eines Affinitätsmoduls gezeigt. Es erfolgt im ersten Schritt eine Reinigung des erfindungsgemäßen Fusionsproteins über seine Targetingmodul vermittelte Heparinaffinität und im zweiten Schritt eine weitere Aufreinigung über einen immobilisierten Antikörper, der eine Affinität zum Effektormodul aufweist. Zu den effektivsten Methoden, Proteine aus Zellextrakten anzureichern, zählt die Affinitätschromatographie. Von besonderem Vorteil für die Anreicherung von ITFs ist die Verwendung des *His-Tag's* als Affinitätsmodul (Hexahistidinsequenz mit Affinität an Nickel-NTA-Sepharose), da selbst die Anwesenheit von chaotrophen Salzen keinen nachteiligen Einfluß auf das Bindeverhalten zeigt. Die Verwendung des Affinitätsmoduls "His-Tag" zur Darstellung von ITF's in nativer Form wird in *Beispiel 12.b*, die in denaturierter Form in *Beispiel 12.c* am Beispiel von ITF-P2-C1 exemplarisch beschrieben. Somit kann die Anreicherung und Reinigung von Proteinen in nativer (*Fig. 25*) sowie denaturierter Form *(Fig. 24)* erfolgen, so daß je nach dem spezifischen Verhalten der jeweiligen ITF-Variante die vorteilhaftere Methode zur Anwendung kommen kann. Interessanter Weise erfolgt auch bei der Reinigung unter denaturierenden Bedingungen neben der nahezu vollständigen Abreicherung von Fremdprotein auch die von proteolytischen Abbauprodukten (*Fig. 24*), was den Vorteil dieser Methode zusätzlich unterstreicht. Ein Verfahren zur Darstellung von löslichen ITF, ausgehend von in GuHCl gelösten, ITF-haltigen Einschlußkörpern ist in *Beispiel 12.c* beschrieben.

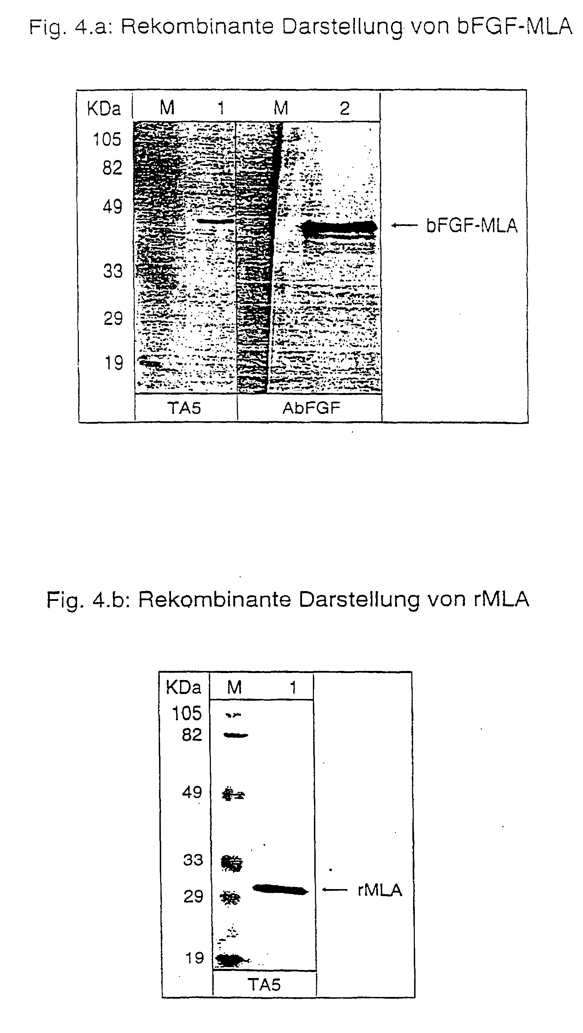

Als Beispiel eines erfindungsgemäßen Fusionsproteins des Typs TPE (Targeting-, Processierungs-, Effektormodul) wurde der "basic fibroblast growth factor" (bFGF) als Targetingmodul über ein Processierungsmodul an den N-Terminus von rMLA fusioniert. Als Processierungsmodul dient hierbei eine Protease-sensitive Domäne entsprechend einem C-terminal gelegenen Sequenzabschnitt des bFGF. Die Domäne ist durch das Vorliegen wenig ausgeprägter Sekundärstrukturelemente von dem N-terminal gelegenen Sequenzabschnitt von bFGF abgegrenzt. Aufgrund dieser Eigenschaft sind die in diesem Abschnitt gelegenen Protease-Erkennungssequenzen für Proteasen der Zielzellen erkennbar. Die Bereitstellung der Substanz kann durch heterologe Expression des Fusionsgens in *E. coli* gemäß *Beispiel 3* durchgeführt werden. *Fig. 4.a* zeigt die ldentität der auf diesem Wege gewonnenen Substanz durch immunologische Detektion mit den monoklonalen anti-bFGF- und anti-nMLA Antikörpern in einer Western-Blot-Analyse.

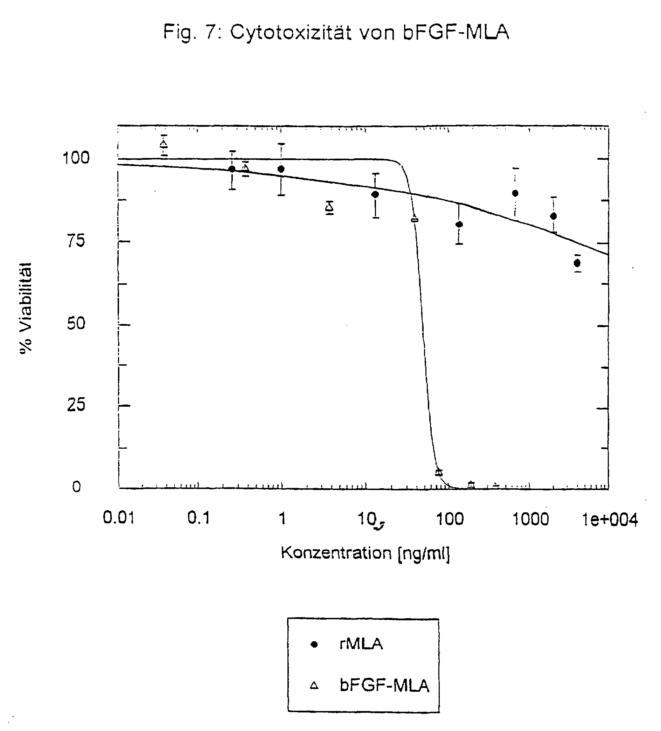

Die Funktionalität eines derartigen bFGF-MLA-Fusionsproteins wurde gegenüber B16-Zellen gemäß *Beispiel 5* dargestellt. Der Vorteil der Verwendung von B16-Zellen liegt darin begründet, daß bekannt ist, daß sie vermehrt bFGF-Rezeptoren auf ihrer Zelloberfläche präsentieren. Der Vergleich der zellabtötenden Wirkung von bFGFrMLA (*Fig. 4.a*) mit der Wirkung des Effektormoduls, in Form von rMLA *(Fig. 4.b)* alleine, demonstriert eindrucksvoll die Realisierung des erfindungsgemäßen Konzepts der Verwendung eines Targetingmoduls. Während rMLA in dem untersuchten Konzentrationsbereich von 200 pg/ml bis 4 µg/ml keine toxische Wirkung auf die B16-Zellen ausübt, entfaltet bFGF-MLA eine potente cytotoxische Wirkung, mit einer halbmaximalen Viabilität (I_{C}-50-Wert) der B16-Zellen bei einer Konzentration von 48 ng/ml (*Fig. 7.a*). Dadurch konnte erfindungsgemäß gezeigt werden, daß das ansonsten nicht wirksame Effektormodul rMLA durch kovalente Verknüpfung über ein Prozessierungsmodul mit einem Targetingmodul, z.B. bFGF, selektiv zur Abtötung gegen B16-Zellen eingestzt werden kann.

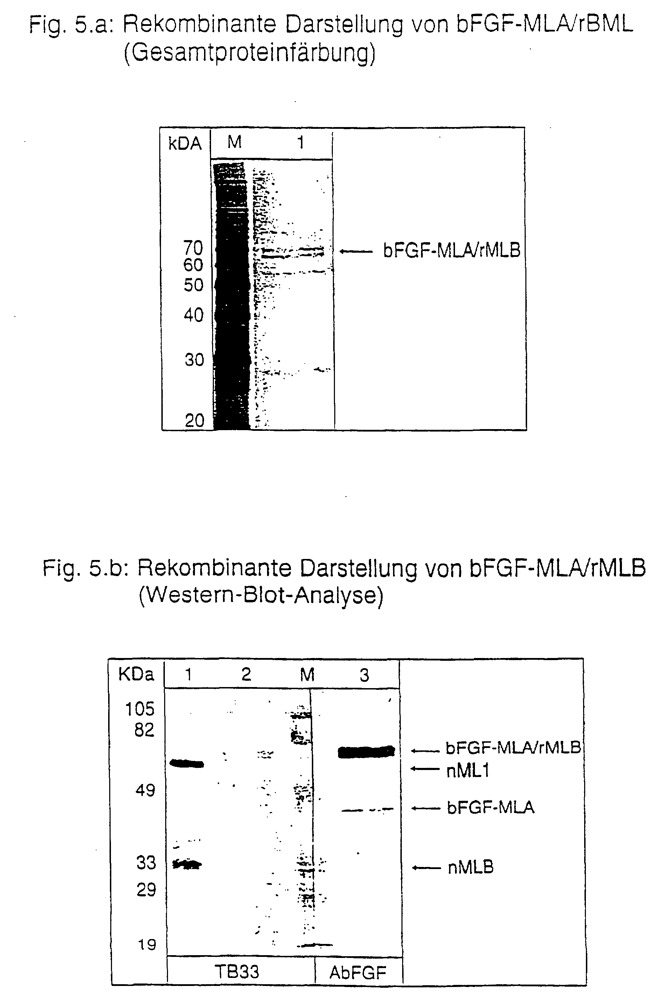

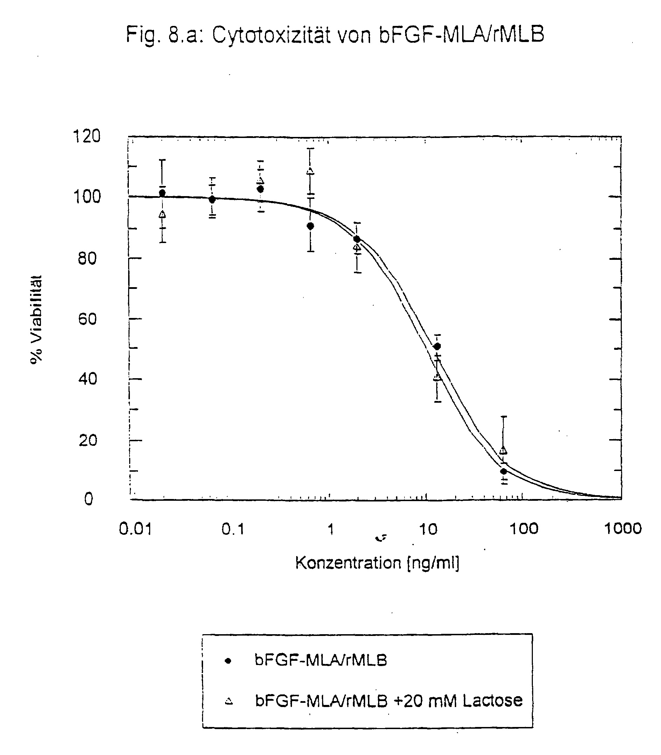

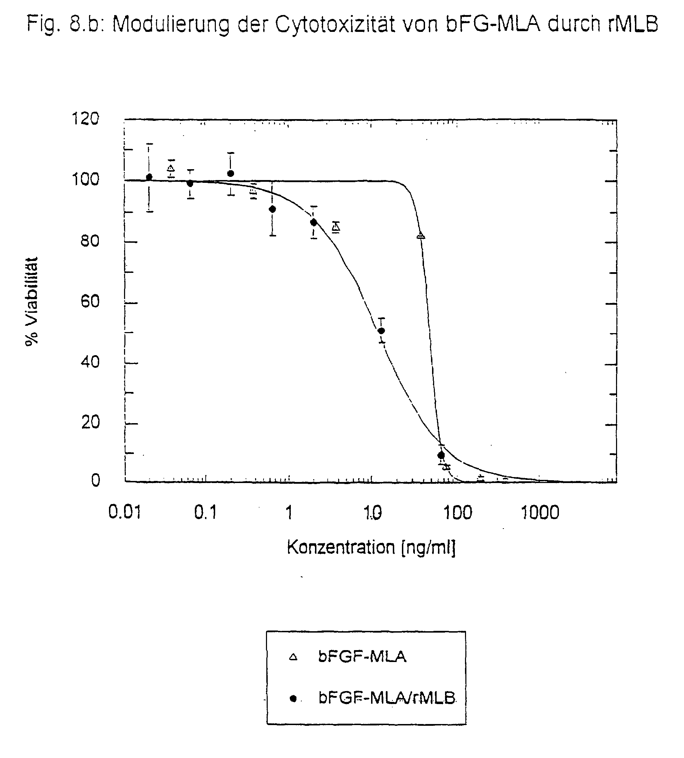

In einem weiteren Ausführungsbeispiel wird die Wirkung eines Modulatormoduls (rMLB) auf ein Effektormodul (rMLA) demonstriert. Ein Fusionsprotein von Typ TPE, hier bFGF-MLA (s. o.), wird gemäß *Beispiel 4* durch einen zusammen mit rMLB durchgeführten *in vitro* Renaturierungsvorgang mit rMLB assoziiert (*Fig. 5.a - 5.b*). Die Assoziation während des Renaturierungsvorganges nutzt die spezifischen Eigenschaften von rMLB zur kovalenten Assoziation mit rMLA durch Ausbildung einer Disulfidbrücke. Das dazu benötigte Ausgangsmaterial in Form der beiden Polypeptidketten kann durch Expression in *E. coli* in Form cytoplasmatischer Einschlußkörper gemäß *Beispiel 2* erfolgen. Die toxizitätssteigernde Wirkung des Modulatormoduls (rMLB) konnte in einem *in vitro* Modell gemäß *Beispiel 6* nachgewiesen werden. Ein Vergleich der Cytotoxizität von bFGF-MLA/rMLB mit der Cytotoxizität des nicht modulierten TPE-Konstrukts (bFGF-MLA) zeigt eine Verbesserung des IC₅₀-Wertes um den Faktor 5, von 48 ng/ml auf 10 ng/ml (*Fig. 8.b*). Dieses Ergebnis bestätigt in eindrucksvoller Weise die Funktionalität von rMLB als Modulatormodul. Die in dem hier demonstrierten modulierten rML-ITF vorhandene Carbohydrat-Bindeaktivität des Modulatormoduls (rMLB) hat dabei keinen Einfluß auf die Aufnahme in die Zellen, was dadurch nachgewiesen werden kann, daß die Zugabe von Lactose, einem kompetitiven Inhibitor der Carbohydratbindung von rMLB, keine Inhibition der Funktionalität der assoziierten Polypeptide TPE/M bewirkt (*Fig. 8.a*).

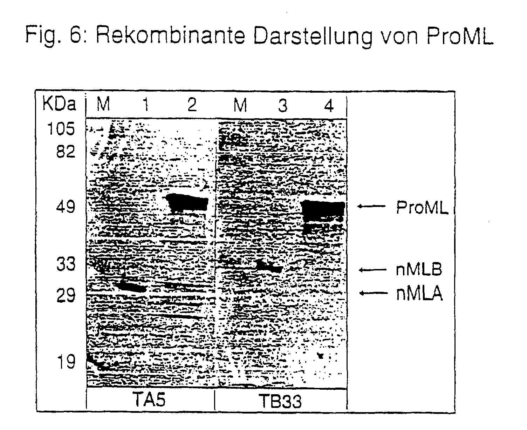

Das *Referenzbeispiel 1* zeigt die Anwendung eines Polypeptids mit der Kombination der Module EPMT zur Untersuchung der Funktionalität des ProML-Propeptids als Processierungsmodul. In diesem speziellen Beispiel wird als Modulator- und Targetingmodul (M^{T}) eine Wildtyp/rMLB-Kette verwendet, in deren Subdomänen 1α und 2γ eine intrinsische Carbohydrat-Bindeaktivität belassen wurde, die im vorliegenden Beispiel zur wenig spezifischen Bindung an Glykosyl-Oberflächenstrukturen der MOLT4-Zielzellen und somit zur Targetierung des Konstruktes vorteilhaft ausgenutzt werden kann. Diese Targetierungsfunktion läßt sich auf Strukturebene den genannten Subdomänen zuweisen und damit deutlich von den modulierenden Domänen funktionell unterscheiden. Dieses Minimalmodell nutzt die neuartigen Eigenschaften des rekombinant dargestellten ProMLs, insbesondere von dessen Propeptid aus. Hier wird das Effektormodul (rMLA) über das Propeptid des Mistellektins gemäß *Bei spiel 3* an das Modulatormodul (rMLB) gekoppelt. Die Gewinnung dieses rML-ITF, in Form von ProML *(Fig.* 6), kann über die Expression in *E. coli* und die Akkumulation cytoplasmatischer Einschlußkörper, wie in *Referenzbeispiel 2* ausgeführt wird, erfolgen.

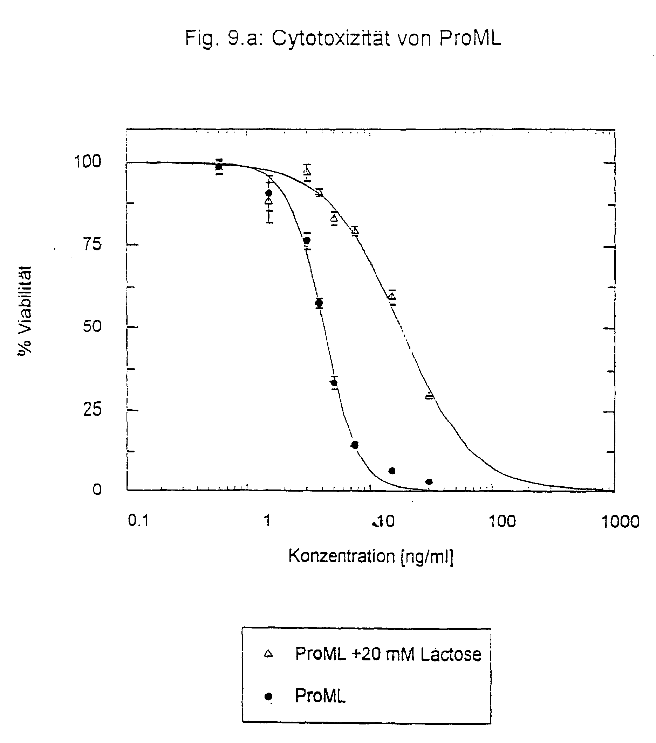

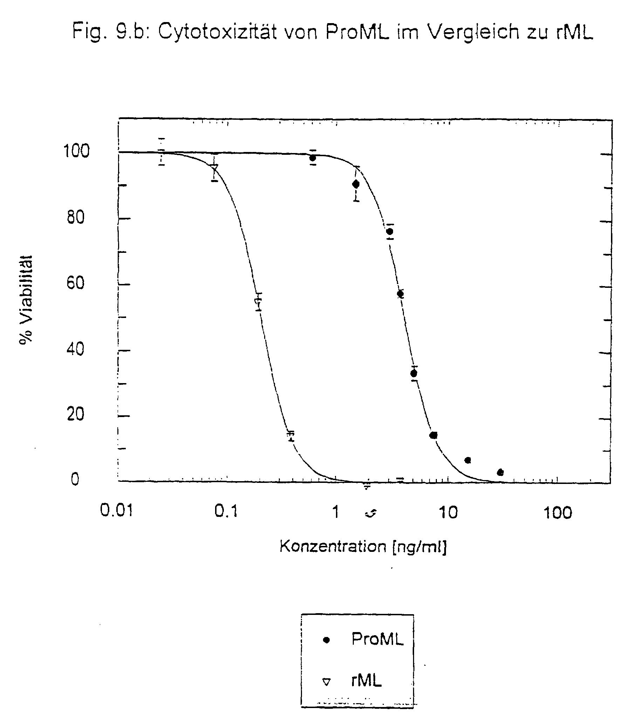

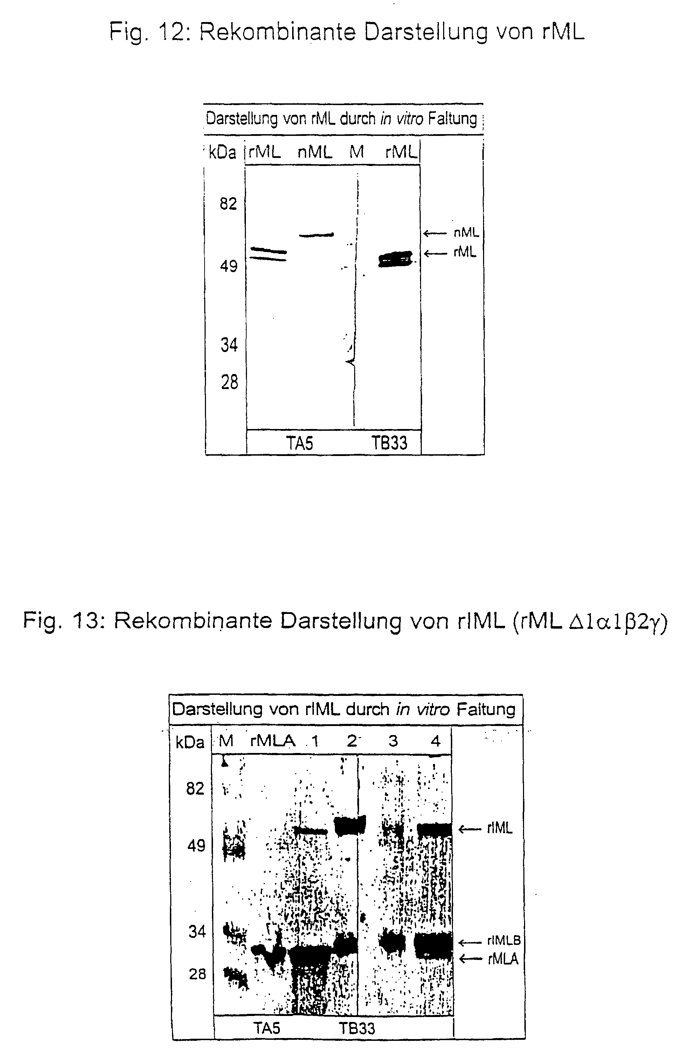

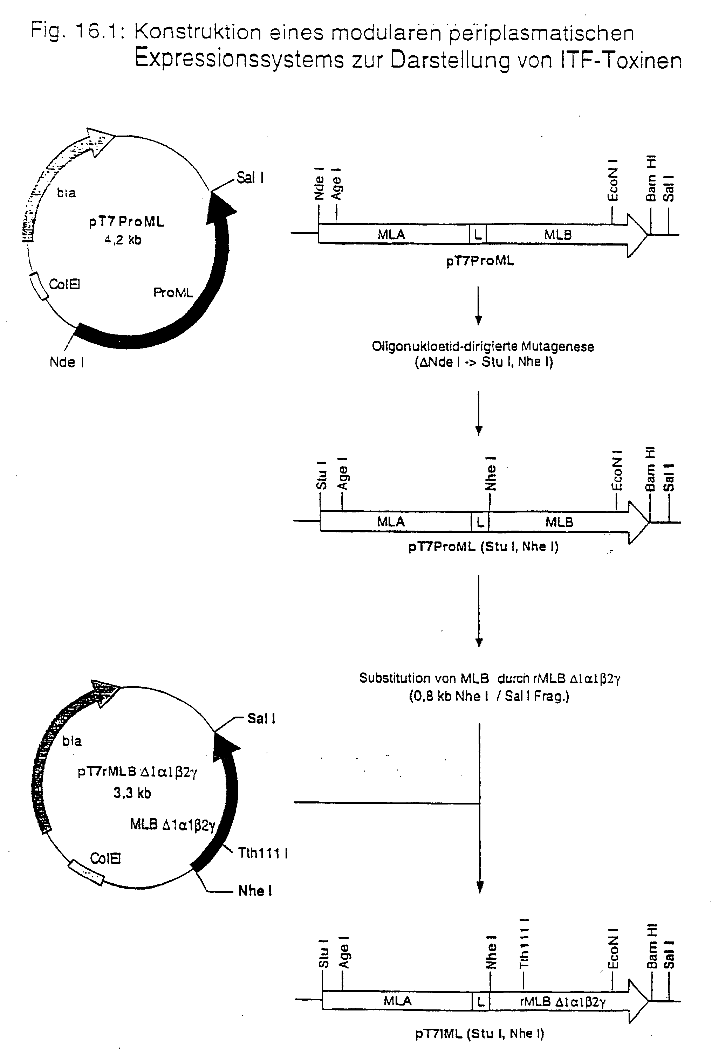

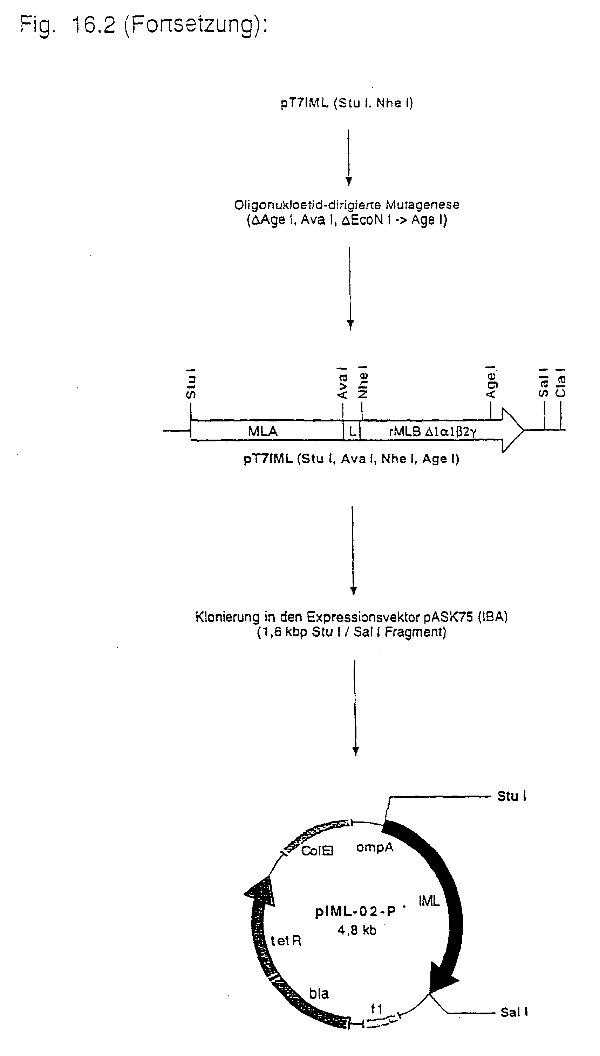

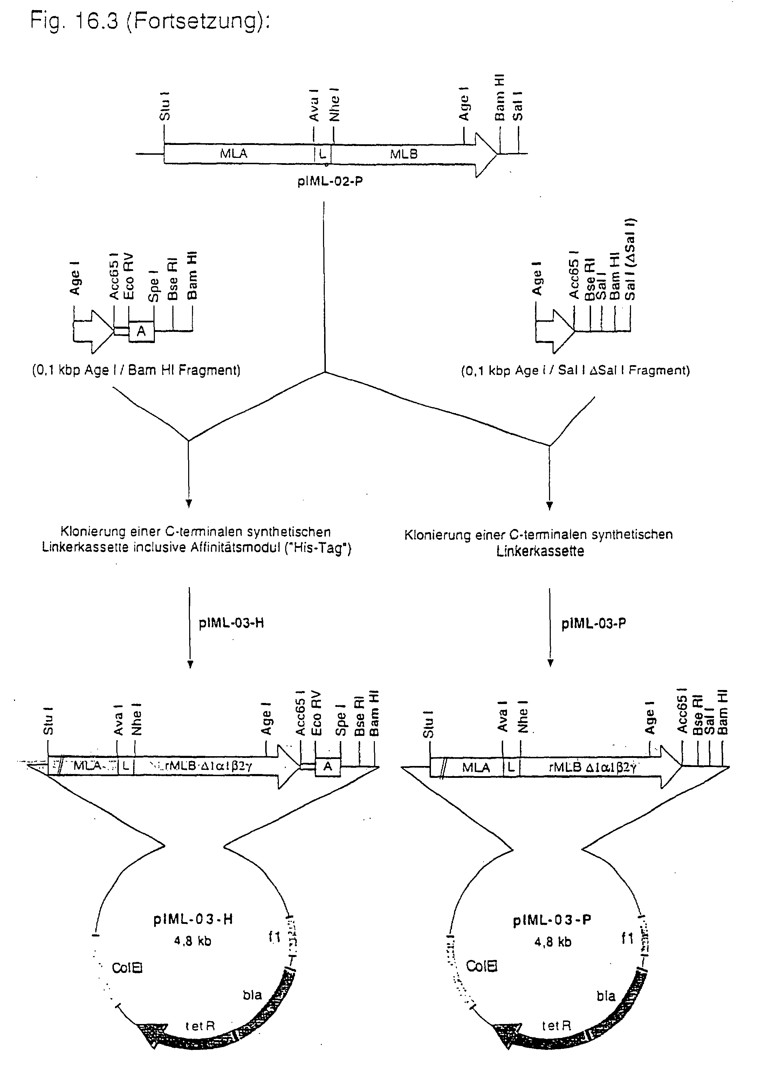

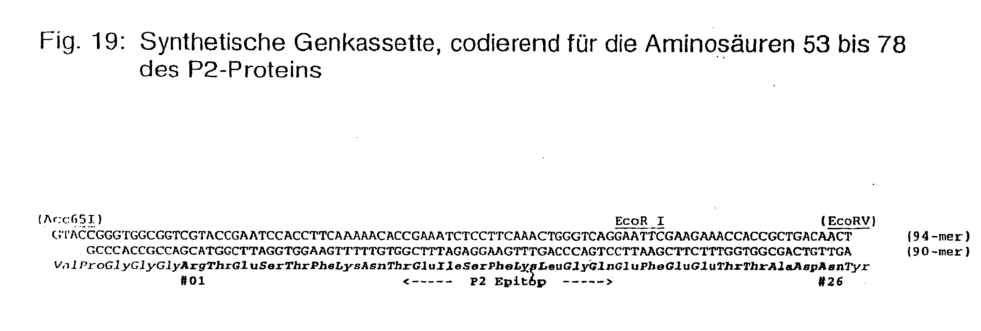

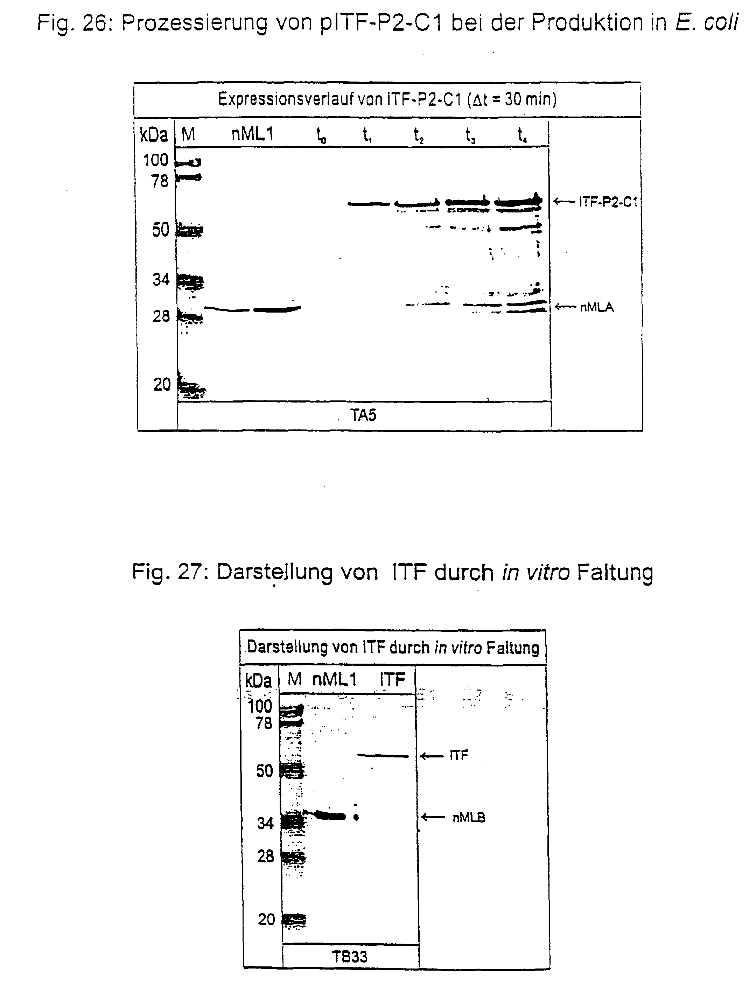

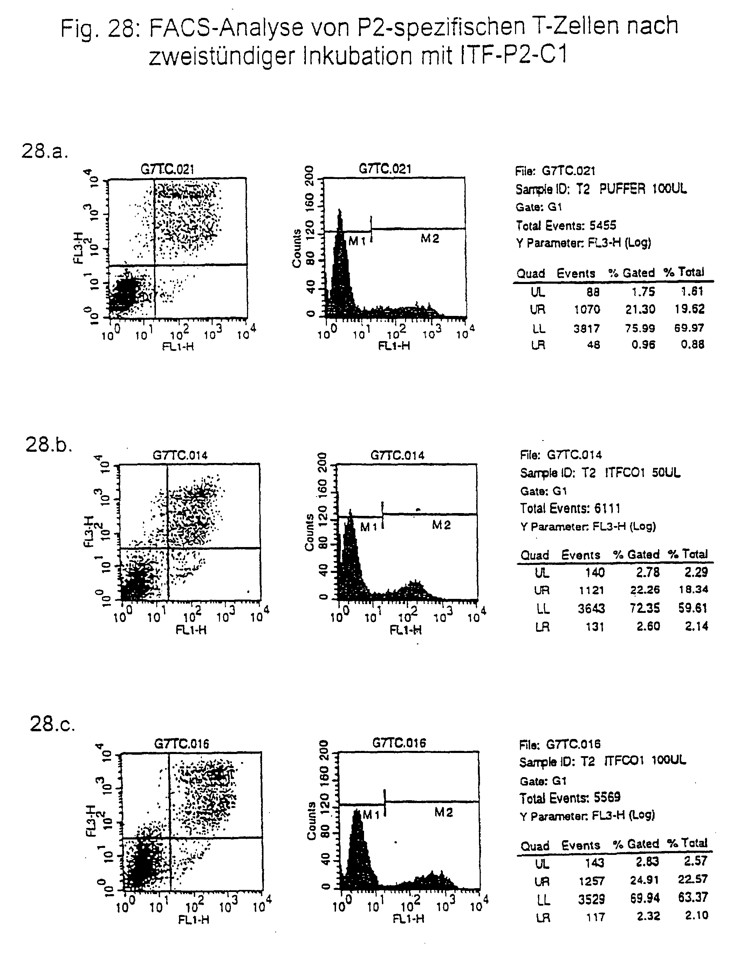

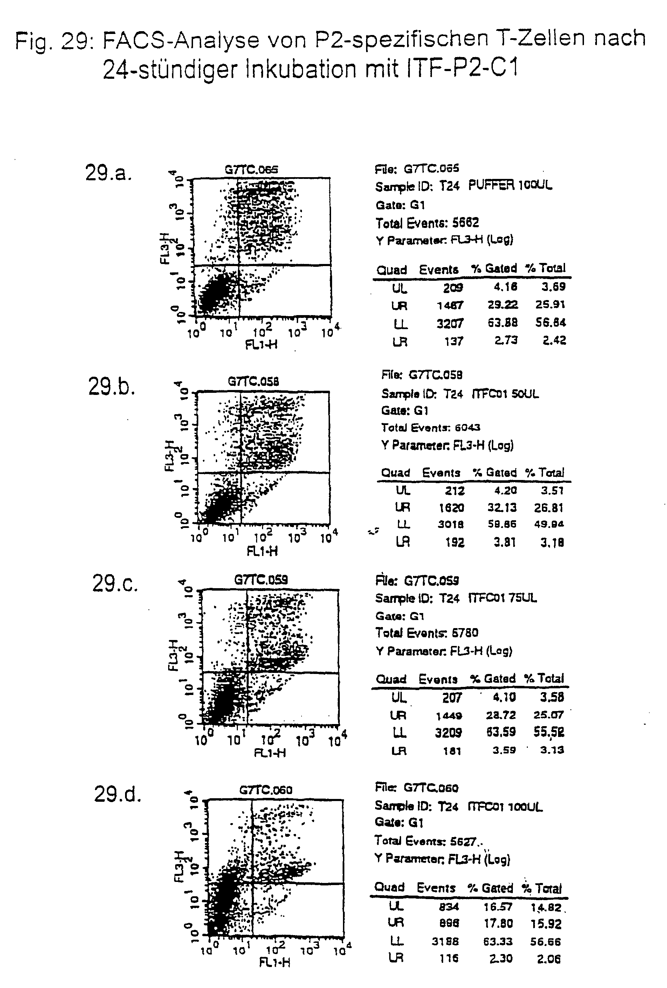

Die Eignung von ProML, das in Referenzbeispielen dargestellt ist und nicht zur Erfindung gehört, als EPMT-Modul zeigt der Funktionalitätstest gegenüber Immunzellen des Blutes, wie z. B. der humanen leukämischen Zellinie MOLT-4 gemäß *Beispiel 9* (*Fig. 9.a*). Die zu beobachtende Wirksamkeit von ProML, mit einem IC₅₀-Wert von 5 ng/ml, zeigt die bisher nicht bekannte und überraschende Eigenschaft eines Typ II-RIP-Propeptids, ein funktionelles Prozessierungsmodul in Form einer proteasesensitiven Sequenz bereitstellen zu können. Darüber hinaus wird das Effektormodul (rMLA) durch das intakte Propeptid außerhalb der Zelle inaktiviert gehalten. Dies konnte bisher nicht für weitere bekannte Pro-Formen anderer Typ II-RIPs gezeigt werden. Um ein spezifisches Zell-Targeting durchführen zu können, ist das Ausschalten der unspezifischen Bindeaktivität der Modulatordomäne vorteilhaft. Dazu ist es wichtig, die Carbohydrat-Bindestellen sowie die an der Bindung beteiligten Aminosäuren zu kennen. Diese wurden wie im Beispiel 7 beschrieben im Falle der B-Kette des Mistellektins durch Mutation auf Nukleinsäureebene ausgetauscht. Anschließend wurde das Carbohydratbindungs-inaktivierte rIML gemäß den Ausführungen in *Beispiel 8 (a. - c.)* durch Expression der Einzelketten und *in vitro* co-Faltung dargestellt *(Fig. 13)*. Die Cytotoxizität dieser rML-Variante ist, wie aus *Beispiel 9* zu ersehen drastisch reduziert, so daß im angestrebten Niedrig-Dosierungbereich einer potentiellen ITF-Therapie, mit einer drastischen Abmilderung des Risikos von Nebenwirkungen im Vergleich zu bisher bekannten Immun- und Mitotoxinen ausgegangen werden kann *(Fig. 14)*.
*Beispiel 10* beschreibt das Vorgehen zur Konstruktion von Vektoren, die als Ausgangspunkt zur Konstruktion beliebiger ITF-Toxine durch modulare Insertion von Targetingmodulen dienen, sowie außerdem die Möglichkeit zur Realisierung unterschiedlicher Anordnungen und Kombinationen der einzelnen ITF-Module eröffnen (*Fig. 16* u. *Fig. 17*).
Um die Funktionalität eines ITF-Toxins mit einem spezifischen Targeting-Modul zu demonstrieren, wurde die Sequenz des neuritogenen P2-Peptids (Weishaupt et al., 1995) in Form eines synthetischen Genfragments (*Fig. 19*) in den Vektor pIML-03-H eingefügt (*Beispiel 11*, *Fig. 17* u. *18*) und zur Expression gebracht (*Beispiel 12.a*). Die Reinigung dieser ITF-Variante kann anschließend über das Affinitätsmodul, sowohl unter nativen (*Beispiel 12.b, Fig. 24*) als auch unter denaturierenden Bedingungen (*Beispiel 12.b, Fig. 25*) gereinigt, bzw. *in vitro* renaturiert werden (*Beispiel 12.c; Fig. 27*). Die Wirksamkeit eines solchen ITF-Toxins wird weiter unten näher beschreiben. Eine Voraussetzung und zugleich ein Hauptproblem der Entwicklung von cytotoxisch wirksamen Substanzen auf Basis von ribosomeninaktivierenden Proteinen ist die Verknüpfung von Toxin-, Modulator- und Targetingmodulen in der Art, daß sie außerhalb von Zielzellen und unter physiologischen Bedingungen stabil verbunden bleiben, intrazellulär jedoch von einander abgespalten werden, so daß sich die Toxinwirkung entfalten kann. Diese Anforderung ist durch die Verwendung von Polypeptid-Linkem (Processierungsmodulen) zu erfüllen, die außerhalb der Zellen eine stabile Verknüpfung gewährleisten, intrazellulär jedoch von speziellen Enzymen - i. d. R. Proteasen - hydrolytisch gespalten werden. In den Mistellektin-basierten ITF-Toxinen konnte ein solcher Linker - bzw. Prozessierungsmodul im Sinne der Erfindung - der die geforderte Funktionalität des Toxins ermöglicht, erstmals erfolgreich eingesetzt werden. Als eine Konsequenz der Proteasesensitivität des verwendeten Prozessierungsmoduls ergibt sich allerdings, daß bereits während der heterologen Expression der entsprechenden ITF-Gene in *E. coli* hydrolytisch gespaltene Effektormodule als Nebenprodukt anfallen (Bsp. *12, Fig. 26*), die dann in der anschließenden Aufarbeitung und Reinigung der ITFs abgetrennt werden müssen. Durch Verwendung von *E. coli* Stämmen mit geeigneter Proteasedefizienz läßt sich der Anteil an Abbauprodukten zusätzlich begrenzen.
Die Wirkung des ITF mit dem neuritogenen P2-Peptid als Targetingdomäne auf P2-spezifische autoreaktive T-Zellen *in vitro* wird beispielsweise mittels Durchflusscytometrie in einem FACS (fluorescence activated cell sorter) analysiert (*Beispiel 13*). Durch die verwendete Färbemethode (Annexin-V/Propidiumjodid) können apoptotische von nekrotischen Zellen unterschieden werden. Die Messungen nach 2h *(Fig. 28*) und nach 24h (*Fig. 29*) zeigen (nähere Erläuterung in *Beispiel 13*), daß je nach Dauer der Behandlung und Konzentration beide Arten des Zelltodes durch das ITF induziert werden.

Die Figuren zeigen:

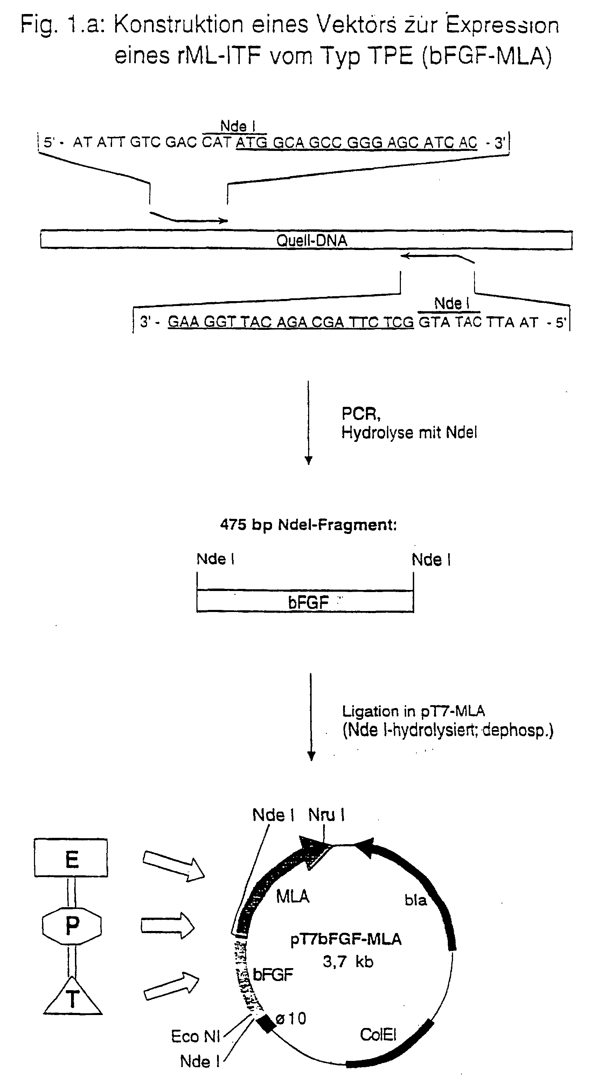

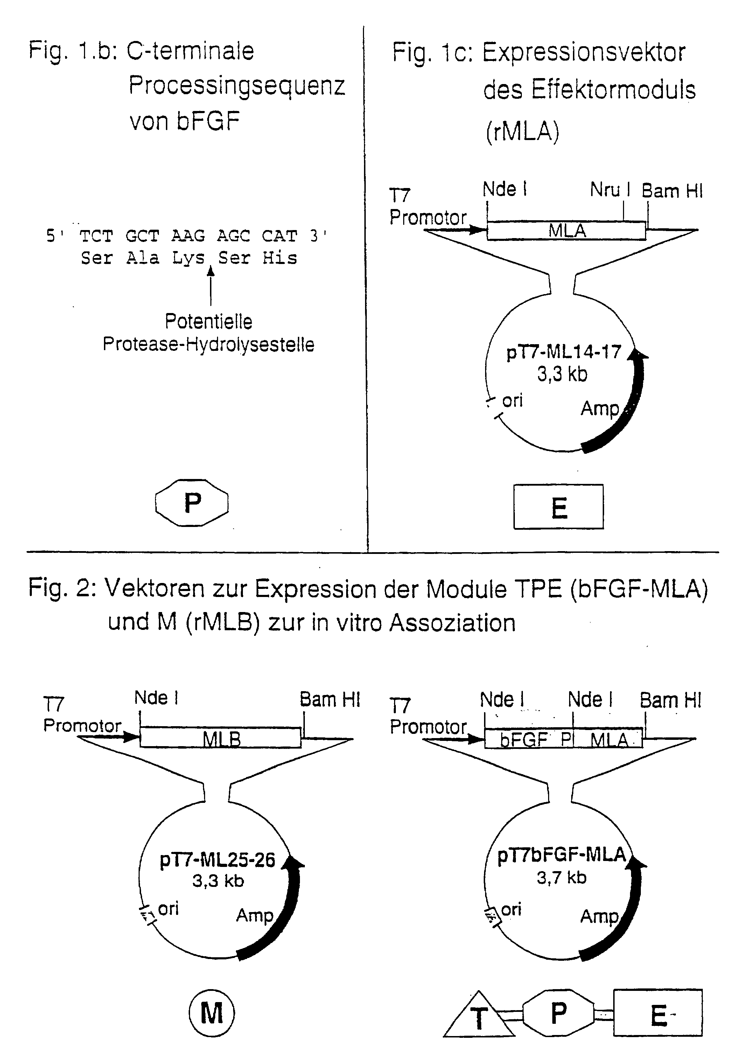

- Fig. 1.a:: Konstruktion eines Vektors zur Expression eines rML-ITF vom Typ TPE (bFGF-MLA).
- Fig. 1.b:: C-terminale Processingsequenz von bFGF.
- Fig. 1.c:: Expressionsvektor des Effektormoduls (rMLA).
- Fig. 2:: Vektoren zur Expression der Module TPE (bFGF-MLA) und M (rMLB) zur *in vitro* Assoziation.
- Fig. 3:: Konstruktion eines Vektors zur Expression eines rML-ITF vom Typ EPM^{T} (ProML).
- Fig. 4.a:: Rekombinante Darstellung von bFGF-MLA.
- Fig. 4.b:: Rekombinante Darstellung von rMLA.
- Fig. 5.a:: Rekombinante Darstellung von bFGF-MLA/rMLB (Gesamtproteinfärbung)
- Fig. 5.b:: Rekombinante Darstellung von bFGF-MLA/rMLB (Western-Blot-Analyse).
- Fig. 6:: Rekombinante Darstellung von ProML.
- Fig. 7:: Cytotoxizität von bFGF-MLA.
- Fig. 8.a:: Cytotoxizität von bFGF-MLA/rMLB.
- Fig. 8.b:: Modulierung der Cytotoxizität von bFGF-MLA durch rMLB.
- Fig. 9.a:: Cytotoxizität von ProML.
- Fig. 9.b:: Cytotoxizität von ProML im Vergleich zu rML.
- Fig. 10:: Beispielhafte Auswahl von Kombinationsmöglichkeiten der rML-ITF-Module.
- Fig. 11.a:: Nukleotidsequenz und abgeleitete Aminosäuresequenz von rMLA.
- Fig. 11.b:: Nukleotidsequenz und abgeleitete Aminosäuresequenz von rMLB.
- Fig. 11.c:: Nukleotidsequenz und abgeleitete Aminosäuresequenz des rML-Propeptids.
Die Nukleotidsequenz der Fig. 11 zeigt verschiedene Restriktionsspaltstellen, Start- und Stoppcodons, die vom Fachmann bei Bedarf zum erfindungsgemäßen Zwecke entfernt oder verändert werden können.
Derartige Ausführungsformen sind in den Figuren 11a' - 11c' gezeigt.
- Fig. 11.d:: Flankierende Bereiche der ProML-Genkassette im Expressionsvektor pT7ProML
- Fig. 11.e:: Flankierende Bereiche der IML-Genkassette im Expressionsvektor pIML-02-P
- Fig. 12:: Rekombinante Darstellung von rML
- Fig. 13:: Rekombinante Darstellung von rIML (rML Δ1α1β2γ)
- Fig. 14:: Cytotoxizität von rIML mit inaktivierten Carbohydratbindestellen im Vergleich zu rML (Wildtyp)
- Fig. 15:: Konstruktion eines Vektors zur Expression eines rML-Derivats ohne Carbohyd rataffinität.
- Fig. 16:: Konstruktion eines modularen periplasmatischen Expressionssytems zur Darstellung von ITF-Toxinen.
- Fig. 17:: Assemblierung von ITF-Toxinen auf Basis der Vektoren pIML-03-H bzw. pIML-03-P mit spezifischer Aktivität gegnüber Zielzellen.

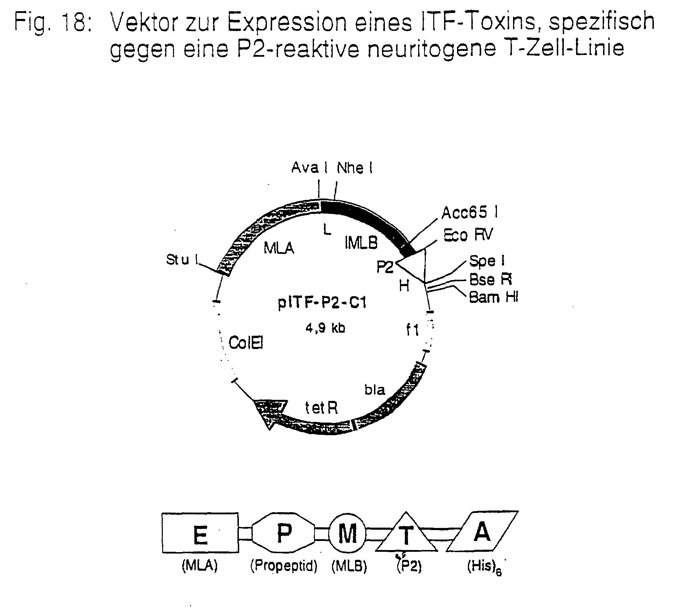

- Fig. 18:: Vektor zur Expression eines ITF-Toxins. spezifisch gegen eine P2-reaktive neuritogene T-Zellinie.
- Fig. 19:: Synthetische Genkassette, codierend für die Aminosäuren 53 bis 78 des P2-Proteins.

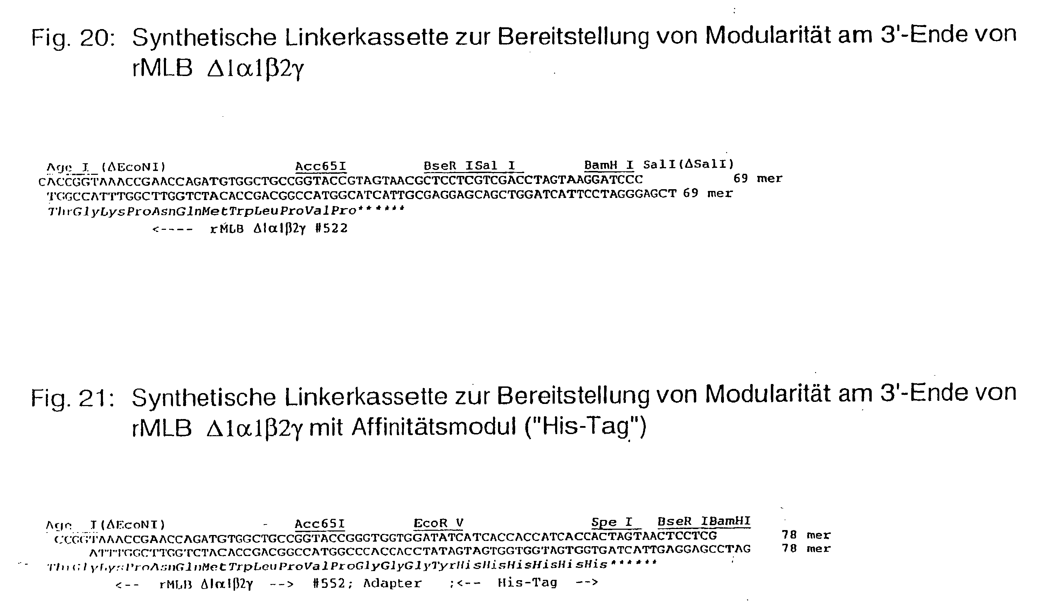

- Fig. 20:: Synthetische Linkerkassette zur Bereitstellung von Modularität am 3'-Ende von rMLB Δ1α1β2γ.
- Fig. 21:: Synthetische Linkerkassette zur Bereitstellung von Modularität am 3'-Ende von rMLB Δ1α1β2γ mit Affinitätsmodul ("His-Tag").

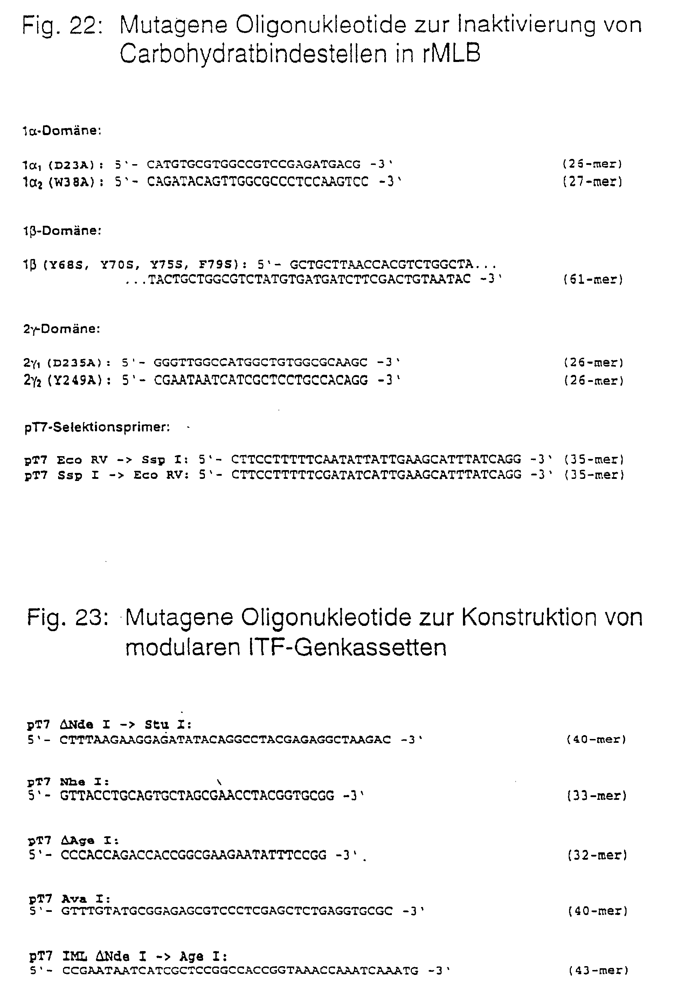

- Fig. 22:: Mutagene Oligonukleotide zur Inaktivierung von Carbohydratbindestellen in rMLB.
- Fig. 23:: Mutagene Oligonukleotide zur Konstruktion von modularen ITF-Genkassetten.
- Fig. 24:: Reinigung von ITF-P2-C1 an Ni-NTA-Sepharose unter denaturierenden Bedingungen.
- Fig. 25:: Reinigung von ITF-P2-C1 an Ni-NTA-Sepharose unter physiologischen Bedingungen.
- Fig. 26:: Prozessierung von pITF-P2-C1 bei der Produktion in E. coli.
- Fig. 27:: Darstellung von ITF durch *in vitro* Faltung.
- Fig. 28:: FACS-Analyse von P2-spezifischen T-Zellen nach zweistündiger Inkubation mit ITF-P2-C1.
- Fig. 29:: FACS-Analyse von P2-spezifischen T-Zellen nach 24-stündiger Inkubation mit ITF-P2-C1

Die Beispiele erläutern die Erfindung.

### Beispiel 1

### Konstruktion eines Vektors zur heterologen Expression eines Fusionsproteins des Typs TPE (bFGF-MLA) in E. coli

Als Beispiel für einen zielzellspezifischen Einsatz der ribosomeninaktivierenden Aktivität der Mistellektin-A-Kette (rMLA), wurde ein Fusionsgen konstruiert, das in einer geeigneten Wirtszelle (*E. coli* BL21) zur cytoplasmatischen Akkumulation eines Fusionsproteins, bestehend aus dem basischen Fibroblasten Wachstumsfaktor (bFGF) und rMLA führt. Das Fusionsprotein besitzt somit den bFGF-Anteil als Targetingmodul und die rMLA-Domäne als Effektormodul. Die C-terminale Sequenz des bFGF enthält eine Trypsinspaltstelle (Lappi et al., 1994) und dient als Processingmodul (*Fig. 1.b*).

Ausgehend von einer Plasmid-DNA-Präparation (Plasmid-Minipräparation, Qiagen) des mittels *E. coli* XL1 -Blue propagierten Plasmids pUC-bFGF (R&D Systems, Wiesbaden) wurde das bFGF-Gen (Abraham et al., 1986) mittels Polymerase Kettenreaktion (PCR) unter Verwendung bFGF-spezifischer Primer amplifiziert (*Fig. 1.a*). Nach Hydrolyse des Amplifikationsprodukts mit der Restriktionsendonuklease Ndel und anschließender Aufreinigung (PCR Purification Kit, Fa. Qiagen) wurde das DNA-Fragment mit dem ebenfalls Ndel-hydrolysierten und dephosphorylierten Vektor pT7-ML14-17 (*Fig. 1.c*), dessen Konstruktion im Detail in der EP Anmeldung Nr, 95109949.8 dargestellt ist, in einer T4-Ligase-Reaktion kovalent verknüpft. Nach Transformation des Ligationsansatzes in *E. coli* XL1-Blue wurde durch Ausplattieren auf Ampicillin-Agar auf Klone selektioniert, die das gewünschte Plasmid pT7bFGF-MLA intrazellulär etabliert haben. Die Plasmid-DNA ausgewählter Klone wurde durch Hydrolyse mit geeigneten Restriktionsendonukleasen auf das elektrophoretische Auftreten vorhergesagter charakteristischer Fragmentgrößen hin getestet. Die korrekte Sequenz des bFGF-Gens aus einem ausgewählten positiven Klon wurde durch Nukleotidsequenz-Analyse verifiziert.

Der erhaltene Expressionsvektor pT7bFGF-MLA (*Fig. 1.a*) enthält das für bFGF-MLA codierende Fusionsgen unter Kontrolle des phi10-Promoters. Nach Induktion mit IPTG kommt es in *E. coli* BL21 zur Bildung von T7-Polymerase, wodurch es zu einer hohen Transkriptionsrate des bFGF-MLA-Gens kommt. Das gebildete Genprodukt kann anschließend aus der löslichen- oder der Einschlußkörper-Fraktion der Zellen isoliert werden.

### Beispiel 2

### Konstruktion der Vektoren zur heterologen Darstellung eines assoziierten Fusionsproteins des Typs TPE/M (bFGF-MLA/rMLB)

Zur Darstellung eines assoziierten Fusionsproteins: Typ TPE/M, bestehend aus *in vitro* gekoppeltem bFGF-MLA und rMLB wird zum einen ein Vektor zur Expression von bFGF-MLA (pT7bFGF-MLA) und zum anderen ein Vektor zur Expression von rMLB (pT7-ML25-26) benötigt *(Fig.* 2). Die Konstruktion des Vektors pT7bFGF-MLA ist in *Beispiel 1* beschrieben. Zur Konstruktion des Vektors pT7-ML25-26 wurde die vollständige, rMLB codierende Sequenz durch spezifische PCR aus komplexer genomischer *Viscum album* DNA amplifiziert, wobei über nicht-komplementäre Bereiche der eingesetzten Primer-Oligonukleotide-Translationskontrollelemente sowie Erkennungssequenzen für Restriktionsendonukleasen eingeführt wurden, über die das Gen für rMLB in den Expressionsvektor kloniert worden ist (Detaillierte Beschreibung in: EP Anmeldung Nr. 95109949.8).

### Referenzbeispiel 1

### Konstruktion eines Vektors zur heterologen Expression eines Polypeptids des Typs EPM^{T} (ProML) in E. coli

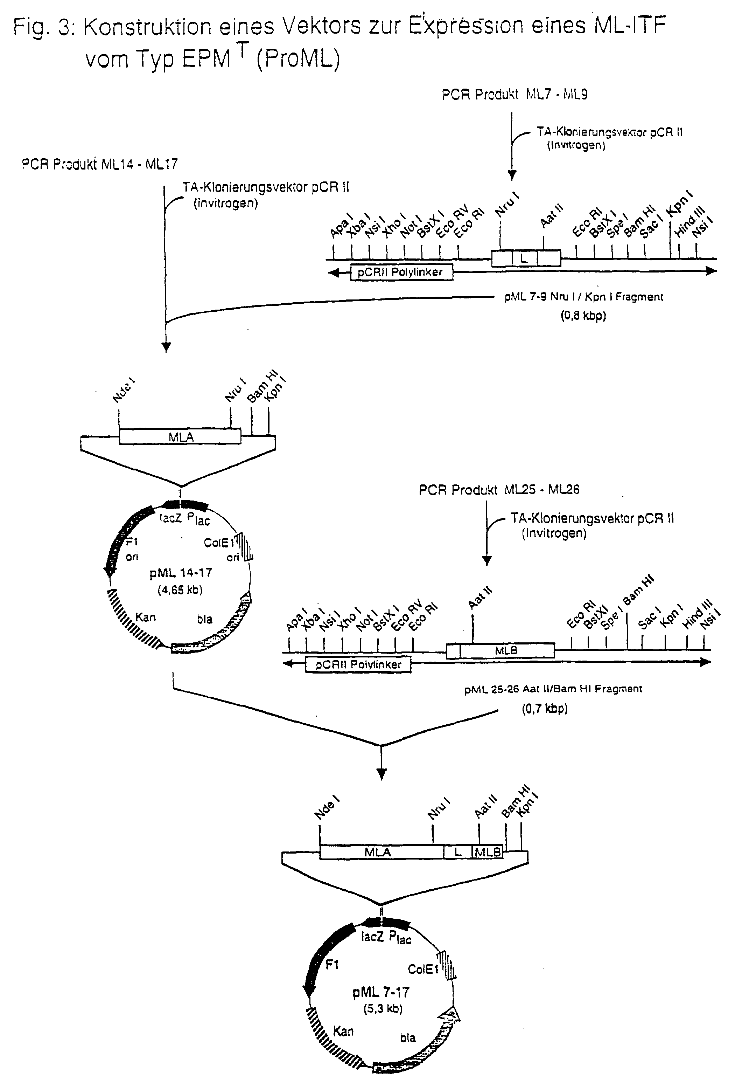

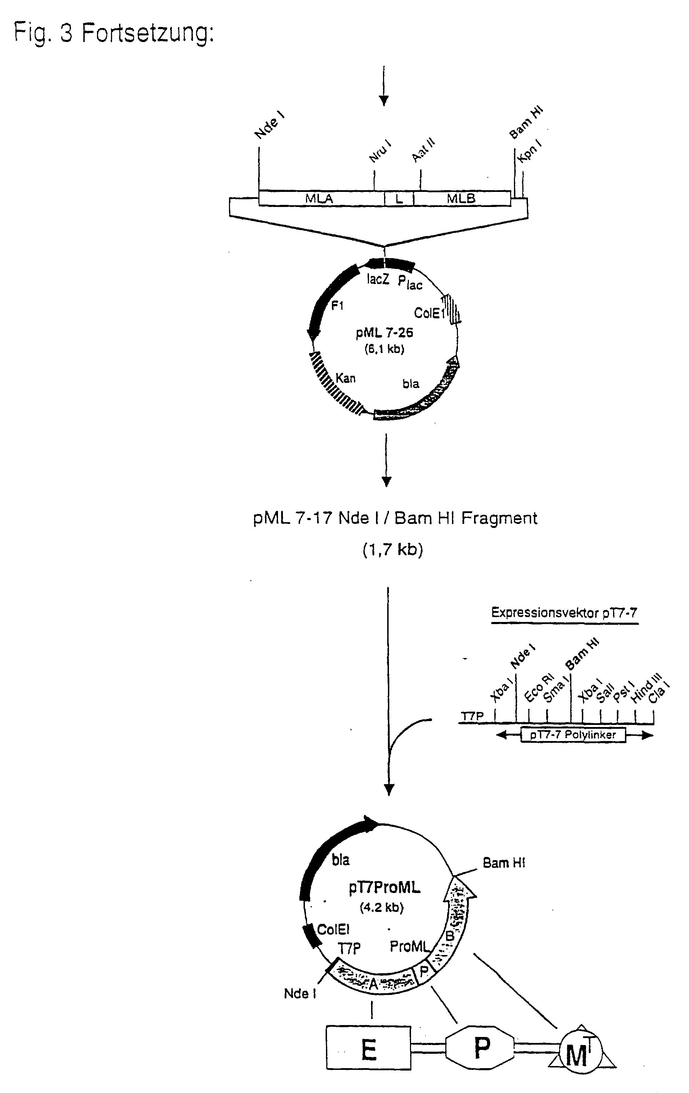

Zur rekombinanten Darstellung von ProML - dem in der Mistel synthetisierten, RIPinaktiven ML-Vorläuferprotein - wurden die aus der Mistel durch PCR isolierten und klonierten Genfragmente für die rMLA (pML14-17), das Propeptid (pML7-9) und die rMLB (pML25-26) (Detaillierte Beschreibung in: EP Anmeldung Nr. 95109949.8) in zwei sequentiellen Ligasereaktionen kombiniert und anschließend in den Expressionsvektor pT7-7 kloniert (*Fig. 3*).

Dazu wurde nach Nrul/Kpnl-Hydrolyse des Vektors pML7-9 die Pro-Sequenz über Agarosegel-Elektrophorese präpariert und in den Nrul/Kpnl-hydrolysierten, dephosphorylierten Vektor pML14-17 kloniert (*Fig. 3*). Nach Transformation von E.coli XL1blue wurde die Plasmid-DNA ampicillinresistenter Klone durch Hydrolyse mit Nrul/Kpnl auf Insertion der Pro-Sequenz validiert. In den so erhaltenen Vektor pML7-17 wurde nach der gleichen Strategie, jedoch unter Verwendung der Restriktionsendonukleasen Aatll und BamHl, die Sequenz der rMLB-Kette mit der Pro-Sequenz fusioniert, was zu dem Vektor pML7-26 führte. Der Expressionsvektor pT7proML wurde nach dem gleichen Vorgehen, durch Umklonieren der ProML-Sequenz in den Vektor pT7-7 über die Restriktionsschnittstellen Ndel und BamHI erhalten. Fig. 11.d zeigt die Lage von Erkennungssequenzen der Restriktionsnukleasen, die eine Insertion der modularen Genkassette in einen entsprechenden Vektor ermöglicht. In Fig. 11.d. ist zudem die Anordnung von Translationskontrollelementen, hier des Startcodons ATG sowie der Stoppcodons TGA und TAA, zur beispielhaften cytoplasmatischen Expression eines Polypeptids des Typs EPM^{T} (ProML) in E.coli gezeigt. Das ProML-Gen steht unter Kontrolle des phi10-T7-Promotors. Bei Transformation des Plasmids in *E. coli* BL21, der das T7-Polymerasegen in trans zur Verfügung stellt, kommt es nach Induktion mit IPTG zur Bildung von T7-RNA-Polymerase und im Sinne einer Verstärkungskaskade zur Transkription des T7-Promoter kontrollierten Gens. Als Folge der einsetzenden massiven Bildung spezifischer mRNA kommt es, je nach Translationseffizienz und Proteineigenschaften, in unterschiedlichem Ausmaß zur Akkumulation des Genprodukts in der löslichen Phase oder in cytoplasmatischen Einschlußkörpern.

### Beispiel 3

### Verfahren zur Produktion eines Fusionsproteins (bFGF-MLA) durch lösliche Expression in E. coli

Die hier und in Beispiel 6 beschriebene heterologe Expression der jeweiligen rML-ITF-Gene erfolgt in *E. coli* BL21, das über ein chromosomal integriertes T7-Gen unter Kontrolle des Lac-Promotors verfügt. Nach Zugabe von IPTG kommt es zur T7-RNA-Polymerase vermittelten Expression der das Fusionsprotein codierenden Nukleinsäure. Das Genprodukt kann aus der löslichen (*dieses Beispiel*) oder der nicht-löslichen Fraktion (*Beispiel* 6) des Zellaufschlußes gewonnen werden, wobei sich die Anreicherung der Fusionsproteine in der gewünschten Fraktion durch die zur Induktion verwendete IPTG-Menge in die eine oder andere Richtung dirigieren läßt.

Zur Produktion von rekombinantem bFGF-MLA Fusionsprotein wurden 10 ml einer in LB-Amp-Medium stationär gewachsenen *E. coli* BL21-(pT7bFGF-MLA; *Fig. 1.a*)-Vorkultur in 1000 ml LB-Amp-Medium in 2000 ml Kolben überführt und bei 37° C bei 190 UpM inkubiert. Bei Erreichen einer Zelldichte entsprechend einer OD₅₇₈ von 0,9 wurde die Expression des Fusionsgens durch Zugabe von 500 µM IPTG induziert. Drei Stunden nach der Induktion wurden die Zellen durch Zentrifugation (10 min, 6000 UpM, 4° C, Sorvall GS3 Rotor) geerntet. Das Zellsediment wurde in Puffer A (600 mM NaCl; 10 mM Tris-HCl, pH 7,4; 4° C) resuspendiert und durch zweimaliges Passagieren einer "French-Press" Druckzelle (Fa. SLM Instruments) bei 1500 psi aufgeschlossen. Die Abtrennung der unlöslichen Zellbestandteile erfolgte durch Zentrifugation (17000 UpM, 30 min, 4° C, Sorvall SS34 Rotor).

Die Anreicherung von löslichem bFGF-MLA-Fusionsprotein mit funktionellem bFGF-Anteil erfolgte durch Bindung an eine immobilisierte Heparin-Affinitätsmatrix (1 ml HiTrap Heparin-Sepharose; Fa. Pharmacia) bei einem konstanten Fluß von 1 ml pro min (Äkta Chromatographieanlage; Fa. Pharmacia). An die Affinitätsmatrix gebundenes Protein wurde mit Puffer B (2M NaCl; 10 mM Tris-HCl; pH7,4) eluiert und zur Vorbereitung der weiteren Reinigung gegen Puffer C (50 mM NaH₂PO₄, 300 mM NaCl, 1 mM EDTA, 10% (v/v) Glyzerin, 0,05% (v/v) Tween-20) dialysiert. Die Abtrennung von bFGF-haltigen Abbauprodukten sowie co-gereinigten *E. coli*-Proteinen erfolgte durch Bindung des bFGF-MLA-Fusionsproteins an eine anti-rMLA-Immunaffinitätsmatrix (260 µg anti-nMLA-IgG (TA5), immobilisiert an Protein A-Sepharose CL4B (Fa. Sigma, Deisenhofen) nach der Methode von Harlow & Spur, 1988). Der monoklonale Antikörper anti-nMLA-IgG TA5 (Tonevitsky et al., 1995) wurde vom Autor zur Verfügung gestellt. Wie auch die anderen hier verwendeten Antikörper sind sie durch Standardverfahren unter Verwendung des entsprechenden Immunogens (im Falle von TA5 ist dies ML-1 oder MLA) herstellbar. Nach 2 stündiger Inkubation der Affinitätsmatrix in der Proteinlösung unter Schwenken bei 4° C wurden die nicht gebundenen Proteine mit Puffer D (1 M NaCl; 50 mM NaH₂PO₄; pH 7,4) ausgewaschen. Gebundenes Protein wurde mit Puffer E (0,1 M Glycin; 300 mM NaCl; 1 mM EDTA; 10% (vlv); Glyzerin 0,05% (v/v) Tween-20; pH 3,0) direkt in Rückstellpuffer (1M NaH₂PO₄; pH 8,0) eluiert. Die Identität des Proteins wurde durch Western-Blot Analyse mit den monoklonalen Antikörpern anti-nMLA (TA5) (Tonevitsky et al., 1995) sowie anti-bFGF (F-6162, Fa. Sigma, Deisenhofen) und einen zweiten, alkalische Phosphatase konjugierten Detektionsantikörper anti-Maus IgG-IgG (Fa. Sigma, Deisenhofen) nachgewiesen (*Fig. 4.a*).

### Beispiel 4

### Darstellung eines assoziierten Fusionsproteins: Typ TEP/M (bFGF-MLA/rMLB)

Die Bereitstellung von bFGF-MLA und rMLB kann unter Verwendung der Expressionsvektoren pT7bFGF-MLA und pT7-ML25-26 (Fig. 2) durchgeführt werden. Dazu wurden jeweils 10 ml von in LB-Amp-Medium stationär gewachsenen *E. coli*-BL21/pT7bFGF-MLA- bzw. *E. coli-* BL21/pT7-ML25-26-Vorkulturen in jeweils 1000 ml LB-Amp-Medium in 2000 ml Kolben überführt und bei 37° C mit 190 UpM geschüttelt. Bei Erreichen einer Zelldichte entsprechend einer OD₅₇₈ von 0,9 wurden die Expressionen durch Zugabe von 500 µM IPTG induziert. Drei Stunden nach der Induktion wurden die Zellen durch Zentrifugation (10 min, 6000 UpM, 4° C, Sorvall GS3 Rotor) geerntet.

Das bFGF-MLA-haltige Zellsediment A und das rMLB-haltige Zellsediment B wurden jeweils in 20 ml Aufschlußpuffer (20 mM NaH₂PO₄; 50 mM NaCl; 1 mM EDTA; pH 7,4; 4° C) resuspendiert und durch zweimaliges Passagieren in einer "French-Press" Druckzelle (SLM Instruments) bei 1500 psi aufgeschlossen. Durch Zentrifugieren (30 min, 10000 UpM, 4° C, SS34-Rotor) wurden jeweils die unlöslischen Zellbestandteile sedimentiert. Die Einschlußkörper enthaltenden Sedimente A und B wurden jeweils mit STET-Puffer (8% (w/v) Saccarose; 50 mM EDTA; 0,05% (v/v) Tween-20; 50 mM Tris-HCl; pH 7,4) gewaschen und anschließend für 4 Stunden in 15 ml Denaturierungspuffer (6 M Guanidiniumchlorid; 20 mM DTT; 50 mM Tris-HCl; pH 8,0; RT), unter Rühren gelöst. Durch Zentrifugieren (17000 UpM, 30 min, 4° c, Sorvall SS34 Rotor) wurden jeweils die unlöslichen Zellbestandteile entfernt. Der bFGF-MLA-Gehalt von Lösung A wurde durch Western-Blot-Analyse mit immunochemischer Detektion durch monoklonalen anti-bFGF-Antikörper (F-6162, Fa. Sigma), anhand eines bFGF-Standards (F-0291, Fa. Sigma, Deisenhofen) bestimmt. Der rMLB-Gehalt von Lösung B wurde durch Western-Blot-Analyse mit immunochemischer Detektion durch monoklonalen anti-rMLB-Antikörper (TB33, Tonevitsky et al, 1995) und einem alkalische Phosphatase konjugierten anti-Maus IgG-IgG Detektionsantikörper (Sigma, Deisenhofen), anhand eines ML1-Mengenstandards (Fa. MADAUS AG, Köln; Charge Nr. 220793) bestimmt. Der verwendete monoklonale Antikörper anti-nMLB-IgG TB33 wurde vom Autor zur Verfügung gestellt. Wie auch die anderen hier verwendeten Antikörper sind sie durch Standardverfahren unter Verwendung des entsprechenden Immunogens (im Falle von TB33 ist dies ML-1 oder MLB) herstellbar.

Zur *in vitro* Assoziation von bFGF-MLA mit rMLB wurde eine Proteinlösung (6 M Guanidiniumchlorid; 2 mM DTT; 50 mM Tris-HCl; pH 6,0) mit einem Gehalt an Kopplungspartnern von jeweils 0,5 mg, mit einer Geschwindigkeit von ca. 1 ml/h bei 4°C unter Rühren in Faltungs- bzw. Kopplungspuffer (50 mM NaH₂PO₄; 50 mM KCl; 1 mM EDTA; 10% (v/v) Glyzerin; 100 mM Glucose; 20 mM Lactose; 1 mM reduziertes Glutathion; 1 mM oxidiertes Glutathion; pH 8,0) des 28-fachen Volumens der Proteinlösung zu einer theoretischen Zielkonzentration an bFGF-MLA/rMLB von 7,5 µg/ml eingetropft. Nach 24 stündigem Rühren bei 4° C wurden unlösliche Bestandteile sedimentiert (17000 UpM, 30 min, 4° C, Sorvall SS34 Rotor). Die löslichen Proteine wurden um Faktor fünf konzentriert (N₂-Überdruck-Rührzelle mit Diaflo Ultrafiltrationsmembran YM30, Fa. Amicon) und gegen Chromatographiepuffer (20 mM NaH₂PO₄; 300 mM NaCl; 1 mM EDTA; 0,1 g/l PVP K17; pH 8,0) dialysiert.

Die Anreicherung von löslichem und lactosebindendem bFGF-MLA/rMLB erfolgte durch Affinitätschromatographie an einer β-Lactosyl-Agarose Affinitätsmatrix (Nr. 20364; Fa. Pierce) mit einer konstanten Flußrate von 0,3 ml/min. Gebundenes Protein wurde mit 400 mM Lactose enthaltendem Chromatographiepuffer eluiert. Die erhaltene Eluatfraktion wurde gegen Aufbewahrungspuffer (20 mM NaH₂PO₄; 300 mM NaCl; 1 mM EDTA; 0,1 g/l PVP K17; pH 7,0) dialysiert. Die Reinheit der eingesetzten bFGF-MLA/rMLB-Probe wurde durch PAGE mit anschließender Silberfärbung dokumentiert (*Fig. 5.a*). Die Identität der Probenbande wurde durch Western-Blot Analyse mit den monoklonalen Antikörpern anti-bFGF (F-6162, Fa. Sigma) und anti-rMLB (TB33, Tonevitsky et al., 1995) sowie einem alkalische Phosphatase konjugierten anti-Maus IgG-IgG Detektionsantikörper (Sigma, Deisenhofen) nachgewiesen (*Fig. 5.b*).

### Referenzbeispiel 2

### Bereitstellung eines rML-ITF vom Typ EPM^{T} (ProML) durch Expression in E. coli in Form von cytoplasmatischen Einschlußkörpern

Zur Produktion von rekombinantem proML wurden 10 ml einer in LB-Amp-Medium stationär gewachsenen *E. coli*- BL21/pT7proML-Vorkultur in 1000 ml LB-Amp-Medium in 2000 ml Kolben überführt und bei 37° C mit 190 UpM geschüttelt. Bei Erreichen einer Zelldichte entsprechend einer OD₅₇₈ von 0,9 wurde die Expression durch Zugabe von 500 µM IPTG induziert. Drei Stunden nach der Induktion wurden die Zellen durch Zentrifugation (10 min, 6000 UpM, 4° C, Sorvall GS3 Rotor) geerntet.

Das Zellsediment wurde in 20 ml Aufschlußpuffer (20 mM NaH₂PO₄; 50 mM NaCl; 1 mM EDTA; pH 7,4; 4° C) resuspendiert und durch zweimaliges Passagieren in einer "French-Press" Druckzelle (SLM Instruments) bei 1500 psi aufgeschlossen. Durch Zentrifugieren (30 min, 10000 UpM, 4° C, SS34-Rotor) wurden die unlöslischen Zellbestandteile sedimentiert. Das Einschlußkörper enthaltende Sediment wurde fünf mal mit STET-Puffer (8% (w/v) Saccharose; 50 mM EDTA; 0,05% (v/v) Tween-20; 50 mM Tris-HCl; pH 7,4) gewaschen und anschließend für 4 Stunden in 15 ml Denaturierungspuffer (6 M Guanidiniumchlorid; 20 mM DTT; 50 mM Tris-HCl; pH 8,0; RT), unter Rühren gelöst. Durch Zentrifugieren (17000 UpM, 30 min, 4° c, Sorvall SS34 Rotor) wurden unlösliche Zellbestandteile entfernt.

Der ProML-Gehalt dieser Lösung wurde durch Western-Blot-Analyse mit immunochemischer Detektion durch monoklonalen anti-rMLA-Antikörper (TA5, Tonevitsky et al., 1995) anhand eines ML 1-Mengenstandards (Fa. MADAUS AG, Köln; Charge Nr. 220793) bestimmt. Die Proteinlösung wurde nun durch Gelfiltration (PD10, Fa. Pharmacia) auf Renaturierungsbedingungen umgepuffert (6 M Guanidiniumchlorid; 10 mM NaH₂PO₄; pH 4,5) und auf eine ProML-Konzentration von 400 µg/ml eingestellt. Renaturiertes ProML wurde durch Eintropfen (ca. 1 ml/h) der Proteinlösung in das 20-fache Volumen Faltungspuffer (50 mM KCl; 1 mM EDTA; 100 mM Glucose; 10 mM Lactose; 10%· (v/v) Glyzerin; 3 mM oxidiertes Glutathion; 0,6 mM red. Glutathion; 50 mM Tris-HCl; pH 8,5; 4° C) unter ständigem Rühren erhalten. Der nach Zentrifugation (17000 UpM, 30 min, 4° C) erhaltene Überstand wurde bei 4° C um Faktor 4 einkonzentriert (N₂-Überdruck-Rührzelle mit Diaflo Ultrafiltrationsmembran YM30, Fa. Amicon) und nochmals zentrifugiert (17000 UpM, 30 min, 4° C). Anschließend wurde die Probe gegen Lagerungspuffer dialysiert (300 mM NaCl; 1 mM EDTA; 100 mg/l PVP-K17; 20 mM NaH₂PO₄; ph 8,0; 4° C). Ausbeute und Identität des renaturierten ProMLs wurde durch Western-Blot-Analyse, einer unter reduzierenden Bedingungen durchgeführten PAGE unter Verwendung der MLA- und MLBspezifischen monoklonalen Antikörper TA5 und TB33 (Tonevitsky et al., 1995) sowie einem alkalischen Phosphatase konjugierten anti-Maus IgG-IgG Detektionsantikörper (Sigma, Deisenhofen) nachgewiesen (*Fig. 6*).

Zur selektiven Anreicherung von ProML mit funktionell renaturiertem B-Ketten-Anteil wurde die Proteinlösung, 1/10 in Chromatographiepuffer (100 mM NaCl; 1 mM EDTA; 100 mg/l PVP-K17; 0,05 % (w/v) BSA; 50 mM NaAcetat/Eisessig; pH 5,6; 4° C) verdünnt, an einer β-Lactosyl-Agarose Affinitätsmatrix (Nr. 20364, Fa. Pierce) mit einer konstanten Flußrate von 0,3 ml/min gebunden und mit 400 mM Lactose enthaltendem Chromatographiepuffer eluiert. Die erhaltene Eluatfraktion wurde gegen Lagerungspuffer (20 mM NaH₂PO₄; 300 mM NaCl; 1 mM EDTA; 0,1 g/l PVP K17; pH 7,0) dialysiert.

### Beispiel 5

### Funktionalität eines Fusionsproteins vom Typ TPE (bFGF-MLA) gegenüber Zielzellen.

Die Cytotoxizität des Fusionsproteins bFGF-MLA wurde gegenüber der Mauszellinie B16 (DKFZ Heidelberg, TZB-Nr.: 630083) in einem Konzentrationsbereich von 375 ng/ml bis 37,5 pg/ml bestimmt. Dazu wurde eine 96-Loch Mikrotiterplatte (Fa. Nunc, Wiesbaden) mit je 1500 B16-Zellen in jeweils 100 µl Kulturmedium (RPMI-1640 (R-7880, Fa. Sigma) plus 5% FKS) beimpft. Die Konzentration der dazu eingesetzten bFGF-MLA-Lösung wurde durch Western-Blot Analyse mit immunologischer Detektion mit monoklonalem anti-bFGF-Antikörper (F-6162, Fa. Sigma) anhand einer bFGF-haltigen Lösung mit bekanntem bFGF-Gehalt (F-0291, Fa. Sigma) bestimmt.

Nach 24 stündiger Inkubation im Brutschrank (37° C; 5% CO₂) wurde die Anheftung der Zellen mikroskopisch verifiziert. 10 µl des Überstandes würden gegen - in steigenden Verdünnungen bFGF-MLA Fusionsprotein enthaltendes - Kulturmedium ausgetauscht, wobei pro bFGF-MLA Verdünnungsstufe sechs Replikas angefertigt wurden. Nach weiteren 72 Stunden Inkubation erfolgte die Quantifizierung des cytotoxischen Effekts durch Bestimmung der Viabilität der Zellen nach der WST-1 Methode (Scudiero et al., 1988). Die Auswertung der Farbreaktion erfolgte durch Bestimmung der optischen Dichte bei einer Wellenlänge von 490 nm (Referenzwellenlänge: 690 nm) mit einem Mikrotiterplattenphotometer (Fa. MWG-Biotech, Ebersberg). Der IC₅₀-Wert (die bFGF-MLA-Konzentration, die zu einer Herabsetzung der Viabilität gegenüber der Positivkontrolle um 50% führt), wurde durch eine 4-Parameter-Kurvenanpassung an die Meßwerte erhalten. Das bFGF-MLA-Fusionsprotein zeigte eine cytotoxische Aktivität mit einem IC₅₀-Wert von 48 ng/ml (*Fig. 7*).

Zur Verifizierung des cytotoxischen Effekts des bFGF-MLA-Fusionsproteins durch bFGF-vermittelte Internalisierung über eine spezifische Bindung an auf der Oberfläche der B16-Zellen vorhandene bFGF-Rezeptormoleküle wurde nach dem gleichen Vorgehen die cytotoxische Wirkung von rMLA auf B16-Zellen in einem Konzentrationsbereich von 4 µg/ml bis 200 pg/ml bestimmt (Fig. 7). Dabei zeigt sich in dem Konzentrationsbereich des IC₅₀-Wertes des bFGF-MLA-Fusionsproteins von 48 ng/ml keinerlei cytotoxischer Effekt von rMLA. In der höchsten eingesetzten Konzentration von 4 µg/ml zeigt sich eine Viabilität der B16-Zellen von über 60%, was als eine beginnende Cytotoxizität von rMLA über unspezifische Aufnahme gewertet werden kann.

Durch Fusion des Effektormoduls mit dem Prozessierungsmodul und dem Targetingmodul konnte ein Wirkstoff (bFGF-MLA) erhalten werden, der in der Lage ist, Zielzellen mit einem IC₅₀-Wert von 48 ng/ml abzutöten. Im Gegensatz dazu zeigt das Effektormodul (rMLA) keine unspezifische Toxizität bis zu einer untersuchten Konzentration von 4 µg/ml. Durch die Fusion mit dem Prozessierungs- und dem Targetingmodul konnte die Toxizität des Effektormoduls um mindestens den Faktor 100 gesteigert werden.

### Beispiel 6

### Funktionalität eines assoziierten Fusionsproteins vom Typ TPE/M (bFGF-MLA/rMLB) gegenüber Zielzellen

Die Cytotoxizität des *in vitro* assozierten Fusionsproteins (bFGF-MLA unter Cofaltung mit rMLB gekoppelt) wurde gegenüber der Mauszellinie B16 (DKFZ Heidelberg, TZB-Nr.: 630083) in einem Konzentrationsbereich von 65 ng/ml bis 1 pg/ml bestimmt, deren Konzentration durch einen "Enzyme-Linked-Lectin-Assay" (ELLA) (Vang et al., 1986) bestimmt worden war.

Dazu wurde eine 96-Loch Mikrotiterplatte (Fa. Nunc, Wiesbaden) mit je 1500 B16 Zellen in jeweils 100 µl Kulturmedium (RPMI-1640 (R-7880, Fa. Sigma) plus 5% FKS) beimpft. Nach 24 stündiger Inkubation im Brutschrank (37° C, 5% CO₂) wurde die Anheftung der Zellen mikroskopisch verifiziert. 10 µl des Überstandes wurden gegen - in steigenden Verdünnungen bFGF-MLA/rMLB Fusionsprotein enthaltendes - Kulturmedium ausgetauscht, wobei pro bFGF-MLA Verdünnungsstufe sechs Replikas angefertigt wurden. Nach weiteren 72 Stunden Inkubation erfolgte die Quantifizierung des cytotoxischen Effekts durch Bestimmung der Viabilität der Zellen nach der MTT Methode (M-5655, Fa. Boehringer; Mossmann, 1983).

Die Auswertung der Farbreaktion erfolgte durch Bestimmung der optischen Dichte bei einer Wellenlänge von 562 nm (Referenzwellenlänge: 690 nm) mit einem Mikrotiterplattenphotometer (Fa. MWG-Biotech, Ebersberg). Der IC₅₀-Wert (die bFGF-MLA/rMLB-Konzentration, die zu einer Herabsetzung der Viabilität gegenüber der Positivkontrolle um 50% führt), wurde durch eine 4-Parameter-Kurvenanpassung an die Meßwerte erhalten.

Das mit rMLB assoziierte Fusionsprotein bFGF-MLA zeigt eine cytotoxische Wirkung mit einem IC₅₀-Wert von 10 ng/ml *(Fig. 8.a*). Die zelltypspezifische Aufnahme über Bindung an bFGF-spezifische Zelloberflächenrezeptoren wurde durch einen, mit Ausnahme der Anwesenheit von 20 mM Lactose im Medium ansonsten identischen Parallelversuch nachgewiesen, wobei der cytotoxische Effekt im Falle von bFGF-MLA/rMLB nicht abgeschwächt wird (*Fig. 8.a*).

Durch Addition des Modulators konnte der IC₅₀-Wert als Maß für die spezifische Toxizität des TPE-Fusionsproteins (bFGF-MLA) von 48 ng/ml auf 10 ng/ml im Falle von bFGF-MLA/rMLB erhöht werden (*Fig. 8.b*). Damit konnte gezeigt werden, daß die über ein Targetingmodul (bFGF) spezifizierte Toxizität des Effektormoduls (rMLA) durch ein Modulatormodul (rMLB) um ein mehrfaches erhöht werden kann.

### Referenzbeispiel 3

### Cytotoxizität eines Polypeptids des Typs EPM^{T} (ProML) gegenüber humanen lymphatischen Leukämiezellen

Die Entfaltung der cytotoxischen Aktivität von ProML wurde mit der humanen mononucleären lymphatischen Leukämiezellinie MOLT-4 (European Collection of Animal Cell Cultures No. 85011413) in einem Konzentrationsbereich von 0,6 ng/ml - 30 ng/ml gemessen.
MOLT-4-Zellen wurden in serumfreiem MDC-1-Medium (Fa. PAN SYSTEMS, Aidenbach) kultiviert und für den Test auf eine Zelldichte von 1,6 x 10⁵ Zellen / ml bei einer Viabilität > 98% eingestellt. Pro Kavität einer 96-Loch Mikrotiterplatte (Fa. Nunc, Wiesbaden) wurden 90 µl (entsprechend 18000 MOLT-4-Zellen) eingesät und mit je 10 µl, in steigender Verdünnung ProML-haltigem MDC-1-Medium versetzt. Der ProML-Gehalt der eingesetzten Lösung wurde zuvor durch ELLA-Analyse (Vang et al., 1986) anhand eines ML1-Mengenstandards (Fa. MADAUS AG, Köln, Charge Nr. 220793) quantifiziert. Als Kontrollen dienten Ansätze mit purem Medium und unter Zugabe von ProML-Lagerungspuffer. Für jede ProML-Konzentration sowie die Kontrollen wurden sechs Replikas erstellt. Die Zellen wurden 72 Stunden bei 37° C und 5% CO₂ im Brutschrank inkubiert.

Die Quantifizierung des cytotoxischen Effekts erfolgte durch die Bestimmung der Viabilität der Zellen nach der WST-1-Methode. (Scudiero et al., 1988). Die Auswertung der Farbreaktion erfolgte durch Bestimmung der optischen Dichte bei einer Wellenlänge von 490 nm (Referenzwellenlänge: 690 nm) mit einem Mikrotiterplattenphotometer (Fa. MWG-Biotech, Ebersberg). Der IC₅₀-Wert (die ProML-Konzentration, die zu einer Herabsetzung der Viabilität (bzw. der optischen Dichte) gegenüber der Positivkontrolle um 50% führt), wurde durch eine 4-Parameter-Kurvenanpassung an die erhaltenen Meßwerte erhalten. ProML entfaltet Cytotoxizität gegenüber MOLT-4 Zellen mit einem IC₅₀-Wert von 5 ng/ml. Daß dieser Effekt auf einer spezifischen rMLB vermittelten Endocytose beruht, zeigt sich durch eine Erhöhung des IC₅₀-Wertes auf 26 ng/ml in Gegenwart von 20 mM Lactose (*Fig. 9.a*).

Das Ergebnis zeigt in überraschender Weise die Potenz der natürlichen Pro-Sequenz, als effektives Processingmodul zu funktionieren. Die Toxizität von ProML ist dabei mit einem IC₅₀-Wert von 5 ng/ml gegenüber dem RIP-aktiven rML mit einem IC₅₀-Wert von 200 pg/ml um den Faktor 25 geschwächt worden *(Fig. 9.b*). Zusammen mit seiner Eigenschaft, die Effektordomäne im nicht-prozessierten Zustand inaktiv zu hatten, ergeben sich für ProML ideale Eigenschaften bei Verwendung als EPM-Komponente in Arzneimitteln.

### Beispiel 7

### Konstruktion eines von rMLB abgeleiteten Modulatormoduls mit verringerter Carbohydrat-Affinität

Aufgrund von Literaturangaben über Ricin sowie durch zusätzliche computergestützte Kraftfeldberechnungen wurden insgesamt acht Aminosäuren in der Mistellektin B-Kette identifiziert, für die eine funktionelle Beteiligung an der Carbohydratbindung als wahrscheinlich angenommen werden konnte. Von daher wurden die Codons für diese Aminosäuren durch sukzessiv durchgeführte Oligonukleotid-dirigierte Mutagenesen nach Deng *et. al*., 1992 (Chamäleon Mutagenese Kit, Stratagene) gegen Alanin- (D23 zu A, W38 zu A, D235 zu A, Y249 zu A) bzw. Serincodons (Y68 zu S, Y70 zu S, Y75 zu S, F79 zu S) ausgetauscht (*Fig. 15, Fig. 22.a*). Als Selektionsprimer wurden abwechselnd die Primer "pT7 Ssp l → Eco RV" und "pT7 Eco RV → Ssp I" (*Fig. 22.b*) eingesetzt. Die Plasmid-DNA von jeweils ausgewählten, durch die Mutagenesen erhaltenen Klonen (*E. coli* XI1Blue), wurde durch Nukleotid-Sequenz Analyse auf die Anwesenheit der jeweils erwarteten mutierten DNA-Sequenz hin überprüft.

### Beispiel 8

### Darstellung von rekombinantem Mistellektin-Varianten (8.a - 8.c)

### (8.a) Expression von rMLA in E. coli in Form unlöslicher Einschlußkörper und Präparation einer rMLA-haltigen Guanidiniumchlorid-Lösung

Zur Expression von rekombinanter Mistellektin A-Kette wurden 1000 ml LB/Amp-Medium (in 2 I Schikanekolben) mit 10 ml einer stationär gewachsenen Vorkultur (50 ml) beimpft und bei 37° C und 190 UpM kultiviert, wobei das Wachstum durch Trübungsmessung bei 578 nm verfolgt wurde. Bei erreichen einer OD578 von 1,0 wurde die Expression des rML-Gens durch Zugabe von 0,5 mM IPTG induziert. Zwei Stunden später wurden die Zellen geerntet (20 min, 6000 UpM, 4° C, Beckmann JA10 Rotor). Das erhaltene Zellsediment wurde in 20 ml Aufschlußpuffer resuspendiert (100 mM NaCl, 1 mM EDTA, 5 mM DTT, 1mM PMSF, 50 mM Tris/HCl pH 8,0) und zweimal mit einem N2-Gasdruckhomogenisator bei 1500 psi aufgeschlossen. Die rMLA-haltigen Einschlußkörper, sog. "inclusion bodies", wurden durch anschließende Zentrifugation (30 min, 10000 UpM, 4° C, Beckmann JA20) sedimentiert. Das Sediment wurde zur Abreicherung von *E. coli* Proteinen 3 x mit je 30 ml STET-Puffer (50 mM EDTA, 8 % (w/v) Glucose, 0,05 % (v/v) Tween-20, 50 mM Tris/HCl, pH 7,4 nach Babbitt *et al*., 1990) gewaschen. Nach Lösen des verbleibenden Zellsediments in Guanidiniumchlorid (6 M GuHCl, 100 mM DTT, 50 mm Tris/HCl, pH 8,0) für 12 Stunden bei Raumtemperatur wurden nicht-lösliche Bestandteile durch Zentrifugieren (17000 UpM, 30 min, 4° C, JA20 Rotor) sedimentiert und verworfen. Der rMLA-Gehalt der erhaltenen Lösung wurde durch Western-Blot Analyse unter Verwendung des nMLA- und rMLA-spezifischen monoklonalen Antikörper (TA5) anhand eines standardisierten nML1-Probe bestimmt.

### (8.b) Expression von rMLB Δ1α1β2γ In E. coli in Form von Einschlußkörpern und Zubereitung einer rMLB Δ1α1β2γ-haltigen Guanidiniumchlorid-Lösung

Zur Expression von rekombinanter Mistellektin B-Kette (rMLB) bzw. der nicht-carbohydratbindenden rMLB Δ1α1β2γ Variante wurden je 1000 ml LB/Amp-Medium (in 2 I Schikanekolben) mit 10 ml einer stationär gewachsenen Vorkultur (50 ml) beimpft und bei 37° C und 190 UpM kultiviert, wobei das Wachstum durch Trübungsmessung bei 578 nm verfolgt wurde. Bei erreichen einer OD₅₇₈ von 1,0 wurde die Expression des rML B- bzw. des rMLB D1a1b2g-Gens durch Zugabe von 0,5 mM IPTG induziert. Vier Stunden nach der Induktion wurden die Zellen geerntet (20 min, 6000 UpM, 4° C, Beckmann JA10 Rotor). Das erhaltene Zellsediment wurde in 20 ml Aufschlußpuffer B resuspendiert (50 mM NaCl, 1 mM EDTA, 5 mM DTT, 1mM PMSF, 20 mM NaH₂PO₄, pH 7,2) resuspendiert und zweimal mit einem N2-Gasdruckhomogenisator bei 1500 psi aufgeschlossen. Die rMLB-haltigen Einschlußkörper wurden durch anschließende Zentrifugation (30 min, 10000 UpM, 4° C, Beckmann JA20) sedimentiert. Das Sediment wurde zur Abreicherung von E. coli Proteinen 3 x mit je 30 ml STET-Puffer (50 mM EDTA, 8 % (w/v) Glucose, 0,05 % (v/v) Tween-20, 50 mM Tris/HCl, pH 7,4 nach Babbitt *et al*., 1990) gewaschen. Nach Lösen des verbleibenden Zellsediments in Guanidiniumchlorid (6 M GuHCl, 100 mM DTT, 50 mm Tris/HCl, pH 8,0) für 12 h bei Raumtemperatur wurden nicht-lösliche Bestandteile durch Zentrifugieren (17000 UpM, 30 min, 4° C, JA20 Rotor) sedimentiert und verworfen. Der rMLB-Gehalt der erhaltenen Lösung wurde durch Western-Blot Analyse unter Verwendung des nMLB und rMLB-spezifischen monoklonalen Antikörpers (TB33) anhand einer Referenzrobe mit bekanntem nML1-Gehalt bestimmt. Das gleiche Verfahren kann zur Gewinnung von rMLB (identische Aminosäuresequenz wie das natürliche Mistellektin) angewendet werden.

### (8.c) Verfahren zur Darstellung von rIML-Holotoxin durch in vitro Faltung

Das Verfahren dient zur Faltung und gleichzeitigen Kopplung der nicht-carbohydratbindenden rMLB-Variante (rMLB Δ1α1β2γ) mit rMLA zur Gewinnung von rekombinantem (Holo)-Mistellektin mit reduzierter Carbohydrataffinität (rIML).
Die in GuHCl gelösten denaturierten Komponenten von rIML, rMLA und rMLB Δ1α1β2γ, (siehe Bsp. 8.a und Bsp. 8.b) wurden auf eine Konzentration von 200 µg/ml eingestellt, zu gleichen Teilen gemischt und durch Gelpermeation (PD10, Pharmacia) auf definierte Pufferbedingungen (6 M GuHCl, 2 mM DTT, 50 mm Tris/HCl, pH 8,0) eingestellt. Die *in vitro*- Faltung und Assoziation erfolgte anschließend durch langsames Eintropfen dieser Lösung in das 30-fache Volumen Faltungspuffer (50 mM KCl, 1 mM EDTA, 100 mM Glucose, 20 mM Laktose, 10 % (v/v) Glyzerin, 1 mM reduziertes Glutathion, 1 mM oxidiertes Glutathion, 50 mM NaH₂PO₄, pH 8,0) unter ständigem Rühren bei 4° C für ca. 12 Stunden. Anschließend wurden nicht-lösliche Bestandteile sedimentiert (30 min 17000 UpM, 4° C, JA20 Rotor) und der Gehalt an löslichem rIML des ca. 10-fach konzentrierten Überstands durch Western-Blot Analyse quantifiziert (Fig. 13). Zur Darstellung von löslichem rML wurde nach der gleichen Methode verfahren, jedoch anstatt rMLB Δ1α1β2γ, das zur Aminosäuresequenz der natürlichen Mistellektin B-Kette identische rMLB eingesetzt (Fig. 12).

### Beispiel 9

### Bestimmung der Cytotoxizität von rIML gegenüber humanen lymphatischen Leukämiezellen

Die Cytotoxizität gegenüber MOLT-4 Zellen des durch *in vitro* Faltung hergestellten und über eine Disulfidbrücke kovalent verbundenen Holo-Protein rIML aus inaktivierter B-Kette (rMLB Δ1α1β2γ) und der rekombinanten Mistellektin A-Kette (rMLA) wurde im Cytotoxizitätstest in einem Konzentrationsbereich von 100 pg/ml - 100 ng/ml nach dem in Referenzbeispiel 3 dargelegten Verfahren bestimmt. Der so ermittelte IC₅₀-Wert von rIML von 25 ng/ml ist gegenüber dem IC₅₀-Wert des zum Vergleich herangezogenen - dem natürlichen Vorbild nML bis auf die Glycosylierung identischen - rML um den Faktor 350 herabgesetzt (*Fig. 14*) und liegt ca. 40-fach über der Toxizität der rekombinanten A-Kette alleine (IC₅₀ > 1µg/ml). Aus diesem Verhalten kann die Schlußfolgerung gezogen werden, daß die Lektinaktivität der B-Kette, die zur unspezifischen Aufnahme des Toxins in beliebige Zelltypen führt, durch die durchgeführten Aminosäureaustausche zumindest stark abgeschwächt werden konnte.

### Beispiel 10

### Konstruktion von Expressionsvektoren mit modular angeordneten Genkassetten für Effektor-, Processing- und Modulator- und Affinitätsmodule

Ausgehend von dem Vektor pT7-ProML, der das Strukturgen für Pro-Mistellektin enthält, wurden durch Modifizierung der DNA-Sequenz durch Oligonukleotid-dirigierte Mutagenese (Deng *et al*., 1992) entsprechende modulare Genkassetten erzeugt, die sich durch relativ einfache Methoden gegen andere Genkassetten mit alternativen Affinitäts-, Effektor-, Modulator- und Processingdomänen austauschen lassen. Durch diese Modifikationen wird außerdem die problemlose Insertion von Targetingmoduien vor oder hinter jedem Modul ermöglicht. Das periplasmatische Zellkompartiment von *E. coli* erfüllt in hohem Maße die Anforderungen eines Disulfidbrücken-haltigen Proteins an die zur Ausbildung einer funktionellen Tertiärstruktur erforderlich Mikroumgebung. Von daher wurden in diesem Beispiel die Genkassetten außerdem in einen periplasmatischen Expressionsvektor eingesetzt.
Ausgehend von dem Strukturgen für ProML wurden durch Oligonukleotid-dirigierte Mutagenese (Deng *et al*., 1992) die am 5'-Ende des Strukturgens des Effektormoduls rMLA vorliegende Nde I-Erkennungssequenz gegen eine Stu I-Erkennungssequenz ausgetauscht, sowie am 5'-Ende des Strukturgens des Modulators (MLB) eine Nhe I-Erkennungssequenz eingeführt *(Fig. 16.1. oben; Fig. 23 a-b*). Das (Carbohydrat-bindende) Modulatormodul rMLB wurde anschließend gegen ein nicht-carbohydrataffines Modulatormodul rIMLB (rMLB Δ1α1β2γ) - aus dem Vektor pT7rMLB Δ1α1β2γ - ausgetauscht (*siehe Fig 16.1 unten*). Dazu wurden die Vektoren pT7ProML (Stu I, Nhe I) sowie pT7rMLBΔ1α1β2γ jeweils mit den Restriktionsendonukleasen Nhe I und Sal I hydrolysiert. Anschließend wurde das 0,8 kbp kg Strukturgen für rIMLB elektrophoretisch in einem Agarosegel (1 % w/v) von dem Expressionsvektor getrennt und aus dem Gelmaterial extrahiert (Qiagen Gel-Extraction Kit). Anschließend wurde das so präparierte Genfragment in einer T4-Ligase-Reaktion kovalent mit dem restringierten und zusätzlich dephosphorylierten Vektor pT7ProML (Stu I, Nhe I) verknüpft. Nach Transformation des Ligationsansatzes in *E. coli* XL1 Blue und Ausplattieren auf ampicillinhaltigem Selektivagar wurde die DNA aus 5 ml über-Nacht-Kulturen ausgewählter expandierender *E. coli*-Klone präpariert (Qia-Präp Kit, Qiagen). Die DNA aus denjenigen Klonen, die den gewünschten Vektor pT7IML (Stu I, Nhe I) enthalten, kann durch Zugabe der Restriktionsendonuklease Tth111 I linearisiert und anhand des Auftretens einer charakteristischen 3,3 kb Bande in der Agarosegel-Elektrophorese identifiziert werden (*Fig. 16.1 unten*). Der so erhaltene Vektor pT7IML (Stu I, Nhe I) wurde erneut durch Oligonukleotid-dirigierte Mutagenese dergestalt modifiziert, daß die Age I Erkennungssequenz im 5'-Bereich des MLA-Gens entfernt, eine Eco NI - Erkennungssequenz nahe des 3' - Endes des IML-Strukturgens in eine Age I - Erkennungssequenz umgewandelt, sowie am 3 ' - Ende des MLA-Gens eine Ava I - Erkennungssequenz eingeführt wurde (*Fig. 16.2, Fig. 23 c. - e*.). Der so erhaltene Vektor pT7IML (Stu I, Ava I, Nhe I, Age I) wurde im molaren Verhältnis von 3:1 mit dem periplasmatischen Expressionsvektor pASK75 (stellt das Gen für die ompA-Signalsequenz im gleichen Leserahmen 5' zur Stu I Erkennungssequenz zur Verfügung) gemischt und mit den Endonukleasen Stu I und Sal I restringiert. Nach Abtrennung der Enzyme (PCR removal kit, Qiagen) wurden die entstandenen DNA-Fragmente durch Inkubation mit T4-Ligase kovalent verknüpft. Nach Abtrennung der T4-Ligase (PCR removal kit, Qiagen) wurden die quantitativ auftretenden unerwünschten Ligationsprodukte durch Behandlung mit den Endonukleasen Eco RI (Erkennungssequenz im Polylinker von pASK75 zwischen den Stu I- und Sal I Erkennungssequenzen) und Cla I (Erkennungssequenz im Vektor pT7) vor der Transformation von *E. coli* XL1Blue linearisiert. Aus 5 ml "über-Nacht"-Kulturen von ausgewählten, nach Ausplattieren des Transformationsansatzes auf Ampicillin Selektivagar heranwachsenden XL1 Blue-Klonen wurde die DNA präpariert (Qia-Prep Kit, Qiagen). In *Fig. 11.e*. ist die beispielhafte Anordnung von Erkennungssequenzen für Restriktionsendonukleasen sowie der Translationsstoppcodons TAG und TAA gezeigt, die eine sekretorische Expression sowie eine Insertion der modularen Genkassette in einen entsprechenden Vektor ermöglicht. Durch Behandlung mit geeigneten Restriktionsendonukieasen und anschließender Agarosegelelektrophorese wurden Klone mit charakteristischen Bandenmustern identifiziert, die das gewünschte Plasmid pIML-02-P intrazellulär etabliert haben *(Fig. 16.2 unten*).
Zur Bereitstellung von Modularität im 3'-Bereich des Modulatormoduls wurden nun entsprechende synthetische Genfragmente kloniert (*Fig. 16.3 oben*). Gleiche Volumina synthetischer zueinander komplementärer Oligonukleotide einer Konzentration von 10 pmol/µl wurden in einem Thermocycler für 1 min auf 95° C erhitzt und durch Abkühlung auf 4° C (3° C / min) hybridisiert. Die Nukleotid-Sequenzen der jeweiligen Oligonukleotid-Paare sind zudem so beschaffen, daß die nach der Hybridisierung entstandenen DNA-Enden komplementär zu den DNA-Enden der zuvor mit entsprechenden Restriktionsendonukleasen behandelten Expressionsvektoren sind (*Fig. 16.3 mitte*). Dazu wurde aus dem Vektor pIML-02-P ein ca. 100 bp langer 3'-Bereich im IMLB-Gen mit den Endonukleasen Age I und Bam HI (Age I und Sal I) herausgespalten. Durch anschließende Behandlung der Lösung mit alkalischer Phosphatase (NEB) und Abtrennung der Enzyme (PCR removal kit) wird die mögliche Religation der Fragmente während der darauffolgenden Ligation verhindert. In jeweils einer T4-Ligasereaktion wurden zum einen ein Genfragment (*Fig. 20)* weiches die Aminosäuresequenz von rIMLB enthält mit dem Age I / Sal I restringierten Vektor (pIML-02-P) fusioniert und zusätzlich die Erkennungssequenzen der Restriktionsendonukleasen Acc 651, Bse RI, Sal I und Bam HI zur Klonierung von Targetingdomänen bereitstellt (*Fig. 16.3*). In einer zweiten Ligasereaktion wurde ein weiteres synthetisches Genfragment mit DNA-Enden, komplementär zu den Age I, Bam HI Restriktionsprodukten des Vektors, welcher außer für die C-terminalen Aminosäuren von rimLB noch für ein Affinitätsmodul (His-Tag) der Sequenz (Gly)₃-Tyr-(His)₆ codiert *(Fig. 21*), ebenfalls fusioniert (*Fig. 16.3 Mitte*).
Die so erhaltenen Expressionsvektoren pIML-03-P und pIML-03-H dienen als Ausgangskonstrukte zur Darstellung von ITF-Toxinen, die sich daraus durch Fusion mit Strukturgenen für die verschiedensten Targetingmodule ergeben (*Fig. 16.3 unten*). Die Targetingmodule können durch die vorhandenen Restriktionsschnittstellen vor oder hinter jedem Modul (Effektor-, Prozessing-, Modulator-, Affinitätsmodul) eingefügt werden (*Fig. 17*).

### Beispiel 11

### Konstruktion einer ITF-Variante mit Toxizität gegenüber einer neurithogenen T-Zellinie

In einem ausgewählten Beispiel wird zur Abtötung einer P2-reaktiven humanen T-Zellinie (Weishaupt *et al*., 1995) ein ITF-Toxin konstruiert, das als Targetingmodul eine für ein Fragment von 26 Aminosäuren (AS 53 - 78) des P2-Proteins (Bestandteil des Myelins im peripheren Nervensystem) codierende synthetische DNA-Sequenz (*Fig. 19*), zwischen Modulator und Affinitätsmodul des Vektors pIML-03-H enthält (*Fig. 17 I. u.*). Dazu wurde der Vektor pIML-03-H - analog zu dem in Beispiel 10 dargelegten Verfahren - mit Acc 651 und Eco RV restringiert, dephosphoryliert, gereinigt und in Gegenwart von T4-Ligase mit den zuvor hybridisierten Oligonukleotiden ligiert. Nach Transformation des Ligationsansatzes in *E. coli* XL1Blue wurde die Plasmid-DNA von ausgewählten, auf Ampicillin Selektivagar proliferierenden Klonen mit der Restriktionsendonuklease Eco RI auf Anwesenheit des Targetingmoduls hin untersucht (linearisierter Vektor im Agarosegelel-Elektropherogramm). Die Sequenz von ausgewählten Plasmiden mit positiver Restriktionskarte wurde schließlich durch Nukieotidsequenz-Analyse verifiziert *(Fig. 18*).

### Beispiel 12

### Bereitstellung von ITF-Toxinen am Beispiel von ITF-P2-C1

### (12.a) Expression von pITF-P2-C1 in E. coli BL21

Zur Expression von pITF-P2-C1 wurde aus einer Glyzerin-Dauerkultur eine 50 ml Vorkultur beimpft und bis zur späten logarithmischen Phase kultiviert (25° C, 150 UpM). Je 10 ml dieser Vorkultur wurden in 1000 ml LB/Amp-Medium (je in 2000 ml Schikanekolben) überimpft. Der Verlauf des Wachstums wurde durch Trübungsmessung bei 578 nm verfolgt. Bei einer OD von 1,0 wurde die Expression des ITF-P2-C1-Gens durch Zugabe von 200 µM Anhydrotetracyclin induziert. Zur Überwachung des Expressionsverlaufs wurden alle 30 min ab Zeitpunkt der Induktion gleiche Zellmengen entnommen und in Probenpuffer aufgekocht (10 % SDS, 200 mM DTT, 50 mM Tris/HCl, pH6,8) und im Westem-Blot analysiert *(Fig. 26*). Nach einer Induktionszeit von zwei Stunden wurden die Zellen sedimentiert (20 min, 6000 UpM, 4° C, JA20 Rotor), in 20 ml / l Kulturvolumen Aufschlußpuffer (600 mM NaCl, 10 mM Imidazol, 10 % (v/v) Glyzerin, 50 mM Na2HPO4, pH 8,0) resuspendiert und anschließend mittels eines N2-Gasdruckhomogenisators (1 x 1500 psi) und nachfolgender Ultraschallbehandlung (2 min, 50 W, 50 % Pulszeit) aufgeschlossen. Anschließend wurde die lösliche Fraktion von den unlöslichen Bestandteilen durch Zentrifugation (45 min, 20000 UpM, 4° C, JA20 Rotor) getrennt.

### (12.b) Funktionalität des Affinitätsmoduls unter nativen Bedingungen am Beispiel der Anreicherung von ITF-P2-C1 aus der löslichen Fraktion von E. coli Extrakten

Während der Expression in *E. coli* löslich akkumuliertes ITF-P2-C1 läßt sich affinitätschromatographisch an Nickel-NTA-Sepharose anreichern. Dazu wird ein Extrakt löslicher *E. coli* Proteine präpariert (*siehe Bsp. 12.a*). 40 ml dieser Proteinlösung werden unter Zusatz von 1 ml Säulenmaterial (Ni-NTA-Sepharose, Qiagen) für 30 min bei 4° C unter Schwenken inkubiert. Anschließend wurde die Säulenmatrix 2 x mit 5 ml Waschpuffer (600 mM NaCl, 20 mM Imidazol, 10 % (v/v) Glyzerin, 50 mM Na₂HPO₄, pH 8,0) gewaschen. Gebundenes Protein wurde anschließend mit Elutionspuffer (600 mM NaCl, 250 mM Imidazol, 10 % (v/v) Glyzerin, 50 mM NaH₂PO₄, pH 6,5) eluiert. Die Eluatfraktionen wurden anschließend im Western-Blot *(Fig. 25)* auf ihren ITF-Gehalt hin untersucht, ausgewählte Fraktionen vereinigt, auf ein Volumen von 2 ml konzentriert und gegen Aufbewahrungspuffer (500 mM NaCl, 10 % (v/v) Glyzerin, 0,1 g/l PVP, 20 mM Na₂HPO₄, pH 7,6) dialysiert. Der ITF-Gehalt der so erhaltenen Lösung wurde durch Western-Blot Analyse anhand einer nML1-Referenzprobe bekannter Konzentration ermittelt.

### (12.c) Funktionalität des Affinitätsmoduls unter denaturierenden Bedingungen am Beispiel der Anreicherung von ITF-P2-C1 aus der unlöslichen Fraktion von E. coli Extrakten

Die ITF-haltigen, im Sediment eines *E. coli* Gesamtzellaufschlußes enthaltenen Einschlußkörper (*siehe. Bsp. 12.a*), wurden durch 12-stündige Inkubation mit 1 ml / Denaturierungspuffer (7 M GuHCl, 50 mM Na₂HPO₄, pH 8,0) unter gleichzeitiger Denaturierung gelöst. Durch Zentrifugation (1 h, 20000 UpM, 4° C, JA20 Rotor) wurden unlösliche Zellbestandteile sedimentiert. Zur Anreicherung von ITF-P2-C1 wurde der lösliche Überstand 2 Stunden mit 1 ml Affinitätsmatrix (Ni-NTA-Sepharose, Qiagen) unter Schwenken inkubiert, das Säulenmaterial mit 2 x 5 ml Waschpuffer gewaschen (7 M GuHCl, 50 mM NaH₂PO₄, pH 6,3) und gebundenes Protein mit 4 ml Elutionspuffer 1 (7 M GuHCl, 50 mM NaH₂PO₄, pH 4,5) und 4 ml Elutionspuffer 2 (7 M GuHCl, 250 mM Imidazol, 50 mM NaH₂PO₄, pH 4,5) eluiert. Der ITF-Gehalt der so erhaltenen Guanidiniumchlorid-Lösung wurde anschließend durch Western-Blot Analyse unter Verwendung des monoklonalen Antikörpers TB33 anhand einer nML1-Probe bekannter Konzentration ermittelt (*Fig. 24*).

### (12.d) Verfahren zur Darstellung von ITF-Toxinen durch in vitro Faltung

Die Darstellung von löslich gefaltetem ITF erfolgt durch langsames Eintropfen einer ITF-haltigen GuHCl-Lösung in das 90-fache Volumen Faltungspuffer (50 mM KCl, 1mM EDTA, 100 mM Glucose, 10 mM Lactose, 10 % (v/v) Glyzerin, 5 mM Glutathion red., 1 mM Glutathion ox., 50 mM Tris/HCl, pH 8,5) unter 12-stündigem Rühren bei 4° C. Anschließend werden nicht-lösliche Bestandteile durch Zentrifugieren (45 min, 20000 UpM, 4° C, JA20 Rotor) sedimentiert und der Überstand um den Faktor 100 einkonzentriert. Nach Dialyse gegen das 1000-fache Volumen Lagerungspuffer (500 mM NaCl, 10 % (v/v) Glyzerin, 0,1 g/l PVP, 20 mM Na₂HPO₄, pH 7,6) wird lösliches, aktives ITF erhalten (*Fig. 27*). Die erhaltene Konzentration an löslichem ITF kann durch Western-Blot Analyse mit monoklonalen Antikörpern gegen nMLB (TB33) anhand einer Referenzprobe mit bekanntem nIML-Gehalt bestimmt werden.

### Beispiel 13

### Bestimmung der Cytotoxizität von ITF-P2-C1 gegen P2-spezifische T-Zellen

Die neuritogene P2-spezifische Zellinie G7TC (Weishaupt et al., 1997) aus der weiblichen Lewis Ratte wurde in RPMI 1640 Medium mit 1% Rattenserum kultiviert. Nach dem Auftauen der Zellen erfolgte die Zählung der lebenden Zellen, die Herstellung der Zellsuspension in einer Dichte von 500 000 Zellen/ml und Aussaat in Platten mit 6 Vertiefungen in einem Volumen von 2,5 ml pro Kavität. Die Behandlung mit dem ITF-Konstrukt P2-C1 (C-terminal an das pro-ML mit inaktivierten Carbohydrat-Bindestellen sind das P2-Peptid und das Affinitätsmodul fusioniert). Die Behandlung erfolgte für 2 h bzw. für 24 h bei 37°C und 5% CO₂ bei Wasserdampfsättigung mit maximal 1/25 Volumen der Testsubstanzverdünnung bzw. dem gleichen Volumen Puffer. Bei einer Konzentration des ITF-P2-C1 von 50 ng/ml ergeben sich bei den gewählten Volumina von 50, 75 und 100 µl in 2,5 ml Kulturvolumen die Endkonzentrationen von 1, 1.5 und 2 ng/ml. Zum Nachweis der Cytotoxizität (Apoptose und Nekrose) wird eine Fluoreszenz-Färbung mit Nachweis per Durchflußcytometrie durchgeführt. Das Prinzip beruht auf der Bindung von FITC-markiertem Annexin V an Phosphatidylserin, das bei Membranen apoptotischer Zellen auf die Aussenseite transloziert wird. Zusätzlich werden mittels DNA-bindendem Propidiumjodid diejenigen Zellen angefärbt, die aufgrund eines toxischen Effekts (direkte Nekrose, sekundäre Nekrose nach Apoptose) eine erhöhte Membranpermeabilität aufweisen, d.h. apoptotische Zellen werden mit FITC gelabelt (Grünfluoreszenz), während nekrotische Zellen doppelt gefärbt sind oder nur PI-Färbung (Rotfluoreszenz) aufweisen. Die Durchführung der Färbung erfolgte nach Anweisung des kommerziell erhältlichen Kits mit jeweils 100 µl Zellsuspension. Die Inkubation P2-spezifischer T-Zellen mit dem ITF resultierte nach 2 h in einer Erhöhung der apoptotischen Zellen bei 1 ng/ml auf das dreifache der Pufferkontrolle (*Fig. 28.a. LR vs. 28.b. LR*) , während bei 2 ng/ml eine Verschiebung zu nekrotischen Zellen aufgetreten ist (*Fig. 28.a. UL vs*. *28.c. UL*). Nach 24 h war ein drastischer Effekt hinsichtlich der Erhöhung des Anteils nekrotischer Zellen von 4 % in der Kontrolle auf 16,6% zu verzeichnen (*Fig 29.a. UL vs. 29.d. UL*). Bei 1 ng/ml ist dagegen eine leichte Erhöhung der Zahl der apoptotischen Zellen (2,7 auf 3,8 %) zu messen (*Fig 29.a. LR vs. 29.b. LR).* Es ist also festzustellen, daß das ITF auf der Basis des Mistellektins wie erfindungsgemäß erwartet beide für dieses Pflanzentoxin beschriebene Wirkungen auf Immunzellen ausübt.

### Abkürzungsverzeichnis

- A: Affinitätsmodul
- bFGF: basischer Fibroblasten Wachstumsfaktor
- DTT: Dithiothreitol
- E: Effektormodul
- EDTA: Ethylendiamintetraacetat
- GFP: "Green-Fluorescent-Protein"
- IgE: Immunglobulin E
- IgG: Immunglobulin G
- IL-2: Interleukin 2
- IPTG: Isopropylthiogalaktosid
- ITF: Immuno-Targeted-Fusionsproteine
- M: Modulatormodul
- MHC: Haupt-Histokompatibilitätskomplex
- P: Prozessierungsmodul
- PAGE: Polyacrylamid-Gelelektrophorese
- ProML: Promistellektin
- RIP: Ribosomen-Inaktivierendes-Protein
- (r)ML: (rekombinantes) Mistellektin
- (r)MLA: (rekombinante) Mistellektin A-Kette
- (r)MLB: (rekombinante) Mistellektin B-Kette
- nMLA: natürliche Mistellektin A-Kette
- nMLB: natürliche Mistellektin B-Kette
- SPDP: N-Succinimidyl-3-(2-pyridyldithio-)propionat
- T: Targetingmodul

Darüber hinaus werden für Aminosäuren gängige Abkürzungen verwendet.

### Literaturverzeichnis

Babbitt, P. C., West, B. L., Buechter, D. D., Kuntz, I. D. und Kenyon, G. L. (1990): Removal of a proteolytic activity associated with aggregates formed from expression of creatine kinase in Escherichia coli leads to improved recovery of active enzyme. Bio/Technology 8, 945-949.
Battelli, M.G., Barbieri, L. und Stirpe, F. (1990). Toxicity of, and histological lesions caused by, ribosome-inactivating proteins, their IgG-conjugates, and their homopolymers. Acta Pathol. Microbiol. Scand. 98, 585-593.
Barbieri, L., Battelli, M.G., Stiirpe, F. (1993): Ribosome-inactivating proteins from plants. Biochim. Biophys. Acta 1154, 237-282.
Benatti, L., Saccardo, M.B., Dani, M., (1989): Nucleotide sequence of cDNA coding for saporin-6, a type-I ribosome-inactivating protein from Saponaria officinalis. Eur. J. Biochem. 183, 465-470.
Better, M., Bernhard, S.L., Williams R.E. et al. (1995) T-cell targeted immunofusion protein from Escherichia coli. J. Biol. Chem. 270, 25, 14951-57.
Bolognesi, A., Tazzari, P.L., Tassi, C., Gromo, G., Gobbi, M. und Stirpe, F. (1992): A comparison of anti-lymphocyte immunotoxins containing different ribosome-inactivating proteins and antibodies. Clin. Exp. Immunol. 89, 341-346.
Brinkmann, U. (1996): Recombinant immunotoxins: protein engineering for cancer therapy. Mol. Med. Today, 10, 439-446.
Bussing, A., Suzart, K., Bergmann, J., Pfüller, U., Schietzel, M., Schweizer, K. (1996): Induction of apoptosis in human lymphocytes treated with *Viscum album* L. is mediated by the mistletoe lectins. CANCER LETTERS, 99, 59-72.
Byers, V.S., Rodvien, R., Grant, K. (1989): Phase I study of monoclonal antibodyricin A chain immunotoxin xXomaZyme 791 in patients with metastatic colon cancer. Cancer Res. 49, 6153-6160.
Carlsson, J., Drevin, H., Axen, R. (1978): Protein thiolation and reversible proteinprotein conjugation. N-Succinimidyl-3-(2-pyridyldithio)propionate, a new heterobifunctional reagent. Biochem. J. 173, 723-737.
Collier, R.J. (1988): Structure activity relationships in diphteria toxin and Pseudomonas aeruginosa exotoxin. A. Cancer Treat Res. 37, 25-35
Cook, J.P., Savage, P.M., Lord, J.M., Roberts, L.M (1993): Biologically active Interleukin 2- ricin A fusion proteins may require inracellular proteolytic cleavage to exhibit a cytotoxic effect.
Deng, W. P. und Nickoloff, J. A. (1992): Site-directed mutagenesis of virtually any plasmid by eliminating a unique site. Anal. Biochem. 200, 81-88.
Frankel, A. E., Burbage, C., Fu, T., Tagge, E., Chandler, J. und Willingham, M. C. (1996): Ricin toxin contains at least three galactose-binding sites located in B chain subdomains 1 alpha, 1 beta, and 2 gamma. Biochemistry 26, 14749-14756.
Gabius, H.J., Walzel, H., Joshi, S.S., Kruip, J., Kojima, S., Gerke, V., Kratzin, H., Gabius, S. (1992): The immunomodutary β-galactoside-specific lectin from mistletoe: partial sequence analysis, cell and tissue binding, and impaction intracellular biosignalling of monocytic leukemia cells. Anticancer Res. 12, 669-676.
Gary, G.L., Smith, D.H., Baldridge, J.S. (1983): Cloning, nucleotide sequence and expression in *Escherischia coli* of the exotoxin A structural gene of Pseudomonas aeruginosa. Proc. Natl. Acad. Sci. USA, 8, 2645-2649.
Glennie, M.J., Brennand, D.M., Bryden, F., McBride, H.M., Stirpe, F., Worth, A.T., Stevenson, G.T. (1988); Bispecific F(ab' gamma)2 antibody for the delivery of saporin in the treatment of lymphoma. J. Immunol. 141, 3662-3670.
Gold, R., Hartung, H.-P., and Lassmann, H. (1997) T-cell apoptosis in autoimmune diseases: termination of inflammation in the nervous system and other sites with specialized immune-defensse mechanisms. TINS 20, 399-403.
Gould, B.J., Borowitz, M.J., Groves, E.S. (1989): Phase I study of an anti-breast cancer immunotoxin by continuous infusion: report of a targeted toxic effect not predicted by animal studies. J. Natl. Cancer Inst. 81, 775-781.
Greenfield, L., Bjom, M.J., Horn, G. (1983): Nucleotide sequence of the structural gene for diphteria toxin carried by corynebacteriophage β. Proc. Natl. Acad. Sci. USA, 80, 6853-6857.
Hara-Nishimura, I., Inoue, K., Nishimura, M. (1991): A unique vacuolar processing enzyme responsible for the conversion of several proprotein precursors into the mature forms. FEBS Lett. 294, 89-93.
Harlowe, E., Spur, D. (1988) In: Antibodies. A laboratory manual. Cold Spring Harbor Laboratory, Cold Spring Harbor, New York.
Hohlfeld, R. (1997) Biotechnological agents for the immunotherapy of multiple sclerosis. Brain, 120, 865-916.
Jansen, F.K., Blythman, H.E., Carriere, D., Casellas, P., Gros, O., Gros, P., Laurent, J.C., Paolucci, F., Pau, B., Poncelet, P., Richer, G., Vidal, H., Voisin G.A. (1982): Immunotoxins: hybrid molecules combining high specificity and potent cytotoxicity. Immunol. Rev. 62, 185-216.
Janssen, O., Scheffler, A., & Kabelitz, D. (1993). In vitro effects of mistletoe extracts and mistletoe lectins. Cytotoxicity towards tumor cells due to the induction of programmed cell death (apoptosis). Drug Res., 43, 1221-7.
Lamb, F.I., Roberts, L.M., Lord, J.M. (1985): Nucleotide sequence of cloned cDNA coding for preproricin. Eur. J. Biochem. 148, 265-270.
Lambert, J.M., Blättler, W.A., Mclntyre, G.D., Goldmacher, V.S. und Scott, C.F.Jr. (1988) In: Immunotoxins (Frankel, A.E. Hrsg.) pp 175-209, Kluwer, Boston.
Lappi, D.A., Baird, A. (1990): Mitotoxins: growth factor-targeted cytotoxic molecules: Prog. Growth Fact. Res. 2, 223-236.
Lappi, D.A., Ying, W., Barthelemy, I., Martineau, D., Prieto, I., Benatti, L., Soria, M., Baird, A. (1994): Expression and activities of a recombinant basic fibroblast growth factor-saporin fusion protein. J. Mol. Biol. 17, 12552-12558.
Lehar, M. S., Pedersen, J. T., Kamath, R. S., Swimmer, C., Goldmacher, V. S., Lambert, J. M., Blättler, W. A. und Guild, B. C. (1994): Mutational and structural analysis of the lectin activity in binding domain 2 of ricin B chain. Prot. Engineering 7, 1261-1266.
Lord, J.M. (1985): Precursors of ricin and *Ricinus communis* agglutinin: glycosylation and processing during synthesis and intracellular transport. Eur. J. Biochem. 146, 411-416.
Magnusson, S., Berg, T. (1993): Endocytosis of ricin by rat liver cells in vivo and *in vitro* is mainly mediated by mannose receptors on sinusoidal endothelial cells. Biochem. J. 291, 749-755.
Masuho, Y., Kishida, K., Saito, M., Umemoto, N., Hara, T. (1982): Importance of the antigen-binding valency and the nature of the cross-linking bond in ricin A-chain conjugates with antibody. J. Biochem. 91, 1583-1591.
Milstein, C., Cuello, A.C. (1983): Hybrid hybridomas and their use in immunohistochemistry. Nature 305, 537-540.
Mosmann, T., (1983). Rapid colorimetric assay for cellular growth and survival: application to proliferation and cytotoxicity assays. J. Immunol. Methods 65, 55-63.
Möckel, B., Schwarz, T., Zinke, H., Eck, J., Langer, M., and Lentzen, H. (1997) Effects of Mistletoe Lectin I on Human Blood Cell Lines and Peripheral Blood Cells. Drug Res. 47, 1145-51.
Nicolson, G.L. (1974): Ultrastructural analysis of toxin binding and entry into mammalian cells. Nature 251, 628-630.
Olive, C. (1995): T cell receptor usage in autoimmune disease. Immunol. Cell. Biol. 73,297-307.
Paprocka, M., Wiedlocha, A., Walzel, H., Radzikowski, C. (1992): The activity of two immunotoxins composed of monoclonal antibody MoAb-16 and A-chain of ricin (Mo-Ab-16-RTA) or A-chain of mistletoe lectin I (MoAb-16-MLIA). ARCHIVUM IMMUNOLOGIAE ET THERAPIAE EXPERIMENTALIS, 40, 223-227.
Pastan, I., FitzGerald, D. (1989): Chimeric toxins. J. Biol. Chem. 264, 15157-15160.
Pastan, I., FitzGerald, D. (1991): Recombinant toxins for cancer treatment. Sience 254, 1173-1177.
Pastan, I., Chaudhary, V., FitzGerald, D.J. (1992): Recombinant toxins as novel therapeutic agents. Annu. Rev. Biochem. 61, 331-354
Price, V.L. (1996) Fusion proteins comprising GM-CSF and antigens and their expression in yeast. International Patent, C12N 15/62, 15/63, WO 96/01903.
Ramakrishnan, S., Fryxell, D., Mohanraj, D., Olson, M., Li, B.Y. (1992): Cytotoxic conjugates containing translational inhibitory proteins. Annu. Rev. Pharmacol. Toxicol. 32, 579-621.
Richardson, P.T., Westby, M., Roberts, M., Gould, J.H., Colman, A., Lord, J.M. (1989): Recombinant proricin binds galactose but does not depurinate 28S rRNA. FEBS Lett. 255, 15-20.
Rutenber, E., Ready., und Robertus., J. D. (1987): Structure and evolution of ricin B chain. Nature 326, 624-626.
Schmied, M., Breitschopf, H., Gold, R., Zischler, H., Rothe, G., Wekerle, H. etal., (1993) Apoptosis of T-lymphocytes in experimental autoimmune encephalomyelitis. Evidence for programmed cell death as a mechanism to control inflammation in the brain. Am. J. Pathol. 143, 446-52.
Scudiero, P.A. et al. (1988). Evaluation of a soluble tetrazolium/formazan assay for cell growth and drug sensitivity in culture using human and other tumor cell lines. Cancer Res. 48, 4827-4833.
Shah, S. A., Halloran, P. M., Ferris, C. A., Levine, B. A., Bourret, L. A., Goldmacher, V. S. und Btättler, W. A. (1993): Anit-B4-blocked ricin immunotoxin shows therapeutic efficacy in foru different SCID mouse tumor models. Cancer Res. 53, 1360-1367.
Shen, G.-L., Li, J.-L., Ghetie, M.A. (1988): Evaluation of four CD22 antibodies as ricin A-chain containing immunotoxins for the in vivo therapy of human B-cell leukemias and lymphomas. Int. J. Cancer 42, 792-797.
Skilleter D.N., Price, R.J., Thorpe, P.E. (1985) Modification of the carbohydrate in ricin with metaperiodate and cyanoborohydride mixtures: effekt on binding, uptake and cytotoxicity to parenchymal cells of rat liver. Biochim. Biophys. Acta 842, 12-21.
Spitler, L., del Rio, M., Khentigan, A. (987): Therapy of patients with malignant melanoma using a monoclonal antimelanoma antibody ricin-A chain immunotoxin. Cancer Res. 47, 1717-1723.
Soer-Rodriguez, A.M., Ghetie, M.A., Oppenheimer-Marks, N., (1993): Ricin A-chain and ricin A-chain immunotoxins rapidly damage human endothelial cells: implications for vascular leak syndrome. Exp. Cell Res. 206, 227-234
Spack, E.G., McCutcheon, M., Corbelletta, N., Nag, B., Passmore, D., and Sharma, S.D. (1995) Induction of tolerance in experimental autoimmune myasthenia gravis with solubilized MHC class II:acetylcholine receptor peptide complexes. J. Autoimmun. 8, 787-807.
Stirpe, F., Barbieri, L., Battelli, M.G., Soria, M., Lappi, D.A. (1992): Ribosome-inactivating proteins from plants: present status and future prospects. Biotechnology 10, 405-412.
Sun, J.-B., Rask, C., Olsson, T., Holmgren, J., and Czerkinsky, C. (1996) Treatment of experimental autoimmune encephalomyelitis feeding myelin basic protein conjugated to cholera toxin B subunit. Proc. Natl. Acad. Sci. USA 93, 7196-7201.
Swimmer, C., Lehar, S. M., McCafferty, J., Chiswell, D. J., Blättler, W. A. und Guild, B. C. (1992): Phage display of ricin B chain and ist single binding domains: System for screening galactose-binding mutants. Proc. Natl. Acad. Sci. USA 89, 3756-3760.
Thorpe, P.E., Brown, A.N.F., Ross, W.C.J., Cumber, A.J., Detre, S.I., Edwards, D.C., Davies, A.J.S. und Stirpe, F. (1981): Blockade of the galactose-binding sites of ricin by its linkage to antibody. Specific cytotoxic effects of the conjugates. Eur. J. Biochem. 116, 447-454.
Tonevitsky, A.G., Toptygin, Ayu., Pfüller, U., Bushueva, T.L., Ershova, G.V., Gelbin, M., Pfuller, K., Agapov, I.I., Franz, H. (1991): Immunotoxin with mistletoe lectin I A-chain and ricin A-chain directed against CD5 antigen of human T-lymphocytes; comparison of efficiency and specificity. INTERNATIONAL JOURNAL OF IMMUNOPHARMACOLOGY, 13, 1037-41.
Tonevitsky, A.G., Rakhmanova, V.A., Agapov, I.I., Shamshiev, A.T., Usacheva, E.A., Prokophév, S.A., Denisenko, O.N., Alekseev, Y. und Pfueller, U. (1995): The interactions of anti-ML1 monoclonal antibodies with isoforms of the lectin from Viscum album. Immunol. Lett. 44, 31-34.
Tonevitsky, A.G., Agapov, I.I., Shamshiev, A.T., Temyakov, D.E., Pohl, P., Kirpichnikov, M.P. (1996): Immunotoxins containing A-chain of mistletoe lectin I are more active than immunotoxins with ricin A-chain. FEBS LETTERS, 392, 166-168.
Vandenbark, A.A. (1996) Treatment of multiple sclerosis with T-cell receptor peptides: results of a double-blind pilot trial. Nature Med. 2, 1109-1115.
Vang, O., PiiLarsen, K., Bog-Hansen, T.C. (1986): A new quantitative and highly specific assay for lectin-binding activity. In: Bog-Hansen, T.C., van Driessche, E. (Hrsg.) Lectins: Biology, Biochemistry, Clinical Biochemistry, Vol. 5, De Gruyter, Berlin, S. 637-644.
Vitetta, E.S., Fulton, R.J., May, R.D., Till, M., Uhr, J.W. (1987): Redesigning nature's poisons to create anti-tumor reagents. Sience 228, 1098-1104.
Vitetta, E.S., Thorpe, P.E. (1991): Immunotoxins containing ricin or its A chain. Semin. Cell Biol. 2, 47-58.
Vitetta, E.S., Thorpe, P.E., Uhr, J.W. (1993): Immunotoxins: magic bullets or misguided missiles? Immunol. Today, 14, 252-259.
Vitetta, E. und Yen, N. (1990): Expression and functional properties of genetically engineered ricin B chain lacking galaktose-binding activity. Biochim. Biophys. Acta 1049, 151-157.
Wales, R., Roberts, L.M., Lord, J.M. (1993): Addition of an endoplasmatic reticulum retrival sequence to ricin A chain significantly increases its cytotoxicity to mammalian cells. J. Biol. Chem. 268, 23986-23990.
Webb, K.S., Ware, J.L., Parks, S.F., Walther, P.J., Paulson, D.F. (1985): Evidence for a novel hybrid immunotoxin recognizing ricin A-chain by one antigen-combining site and a prostate-restricted antigen by the remaining antigen-Cancer Treat. Rep. 69, 663-672.
Weishaupt, A., Gierich, G., Jung, S., Gold, R., Enders, U., Pette, M., Hayasaka, K., Hartung, H.-P. und Toyka, K. V. (1995): T cell antigenic and neuritogenic activity of recombinant human peripheral myelin P2 protein. J. Neuroimmun. 63, 149-156.
Weishaupt, A., Gold, R., Gaupp, S., Giegerich, G., Hartung, H.-P., and Toyka, K.V. (1997) Antigen therapy eliminates T-cell inflammation by apoptosis: Effective treatment of experimental autoimmune neuritis with recombinant myelin protein P2. Proc. Natl. Acad. Sci. USA 94, 1338-43.
Westby, M., Argent, R.H., Pitcher, C., Lord, J.M., Roberts, L.M. (1995): Preparation and characterization of recombinant proricin containing an alternative protease-sensitive linker sequenz. Bioconjugate Chem. 3, 375-381

## Patentansprüche

1. Nukleinsäuremolekül, das ein Fusionsprotein codiert, das die folgenden Komponenten aufweist:
(a) ein Effektormodul, das intrazellulär cytotoxisch wirkt;
(b) ein Prozessierungsmodul, das kovalent mit dem Effektormodul verbunden ist, und das eine Erkennungssequenz für eine Protease aufweist, wobei das Prozessierungsmodul das Mistellektin-Propeptid umfaßt oder ein Fragment oder Derivat davon, wobei das Fragment oder Derivat eine Erkennungssequenz für eine Protease aufweist und die intrazelluläre Freisetzung des Effektormoduls in der Zielzelle durch zelleigene Proteasen im Zuge der rezeptorvermittelten Endocytose in den Endosomen und Prälysosomen stattfinden lässt, umfaßt und wobei das Mistellektin-Propeptid von einem Nukleinsäuremolekül codiert wird, ausgewählt aus der Gruppe bestehend aus:
(i) Nukleinsäuremolekülen, die eine Nukleotidsequenz umfassen, die die in Fig. 11.c angegebene Aminosäuresequenz oder ein Fragment davon codiert;
(ii) Nukleinsäuremolekülen, die die in Fig. 11.c angegebene Nukleotidsequenz oder ein Fragment davon umfassen;
(iii) Nukleinsäuremolekülen, die mit einem Nukleinsäuremolekül aus (i) oder (ii) unter stringenten Bedingungen hybridisieren; und
(iv) Nukleinsäuremolekülen, die zu den in (iii) genannten Nukleinsäuremolekülen entsprechend dem genetischen Code degeneriert sind; und
(c) ein Targetingmodul, das kovalent mit dem Prozessierungsmodul verbunden ist, und das spezifisch an die Oberfläche einer Zelle bindet, wodurch die Internalisierung des Fusionsproteins in die Zelle vermittelt wird.

2. Nukleinsäuremolekül nach Anspruch 1, wobei das Effektormodul ein Effektormodul ist, das intrazellulär cytotoxisch wirkt, wobei das Effektormodul die Mistellektin A-Kette oder ein intrazellulär cytotoxisch wirkendes Fragment oder Derivat davon umfasst, wobei die Mistellektin A-Kette von einem Nukleinsäuremolekül codiert wird, ausgewählt aus der Gruppe bestehend aus:
(i) Nukleinsäuremolekülen, die eine Nukleotidsequenz umfassen, die die in Fig. 11.a angegebene Aminosäuresequenz oder ein Fragment davon codiert;
(ii) Nukleinsäuremofekülen, die die in Fig. 11.a angegebene Nukleotidsequenz oder ein Fragment davon umfassen;
(iii) Nukleinsäuremolekülen, die mit einem Nukleinsäuremolekül aus (i) oder (ii) unter stringenten Bedingungen hybridisieren; und
(iv) Nukleinsäuremolekülen, die zu den in (iii) genannten Nukleinsäuremolekülen entsprechend dem genetischen Code degeneriert sind.

3. Nukleinsäuremolekül nach Anspruch 1, wobei das Effektormodul die Mistellektin A-Kette oder ein intrazellulär cytotoxisch wirkendes Fragment oder Derivat davon umfaßt.

4. Nukleinsäuremolekül nach einem der Ansprüche 1 bis 3, wobei das Fusionsprotein ferner die folgende Komponente aufweist:
(d) ein Modulatormodul, das kovalent mit dem Prozessierungsmodul, dem Effektormodul und/oder mit dem Targetingmodul verbunden ist, und das die intrazelluläre toxische Wirkung des Effektormoduls moduliert.

5. Nukleinsäuremolekül nach Anspruch 4, wobei das Modulatormodul von einem Nukleinsäuremolekül codiert wird, ausgewählt aus der Gruppe bestehend aus:
(i) Nukleinsäuremolekülen, die eine Nukleotidsequenz umfassen, die die in Fig. 11.b angegebene Aminosäuresequenz oder ein Fragment davon codiert, wobei das Fragment die biologische Aktivität der Mistellektin B-Kette besitzt;
(ii) Nukleinsäuremolekülen, die die in Fig. 11.b angegebene Nukleotidsequenz oder ein Fragment davon umfassen, wobei das Fragment eine Aminosäuresequenz codiert, die die biologische Aktivität der Mistellektin B-Kette besitzt;
(iii) Nukleinsäuremolekülen, die mit einem Nukleinsäuremolekül aus (i) oder (ii) unter stringenten Bedingungen hybridisieren; und
(iv) Nukleinsäuremolekülen, die zu den in (iii) genannten Nukleinsäuremolekülen entsprechend dem genetische Code degeneriert sind.

6. Nukleinsäuremolekül nach einem der Ansprüche 1 bis 5, wobei das Fusionsprotein ferner die folgende Komponente aufweist:
(e) ein Affinitätsmodul zur Aufreinigung, das kovalent mit dem Effektormodul, dem Prozessierungsmodul, dem Targetingmodul und/oder dem Modulatormodul verbunden ist.

7. Nukleinsäuremolekül nach einem der Ansprüche 1 bis 6, wobei das Targetingmodul eine Zelle des Immunsystems, eine Tumorzelle oder eine Zelle des Nervensystems spezifisch erkennt.

8. Nukleinsäuremolekül nach Anspruch 7, wobei die Zelle des Immunsystems eine Zelle des spezifischen Immunsystems ist.

9. Nukleinsäuremolekül nach Anspruch 7 oder 8, wobei die Zelle des Immunsystems eine T-Zelle, oder eine Zelle des unspezifischen Immunsystems und wobei die Tumorzelle eine entartete Zelle des Immunsystems ist.

10. Nukleinsäuremolekül nach Anspruch 9, wobei die T-Zelle eine T_{H}2-Zelle ist.

11. Nukleinsäuremolekül nach einem der Ansprüche 6 bis 10, wobei das Affinitätsmodul eine Histidinsequenz, Thioredoxin, Strep-Tag, T7-Tag, Flag-Tag, Maltose-Bindungs-Protein oder GFP umfaßt.

12. Nukleinsäuremolekül nach einem der Ansprüche 4 bis 11, wobei das Modulatormodul die Mistellektin B-Kette oder ein Fragment oder Derivat davon, das die biologische Aktivität der Mistellektin B-Kette besitzt, oder die Peptide KDEL oder HDEL umfaßt.

13. Nukleinsäuremolekül nach Anspruch 12, wobei die Mistellektin B-Kette einen Austausch in Aminosäureposition 23, 38, 68, 70, 75, 79, 235 oder 249 oder eine Kombination derartiger Austausche aufweist.

14. Nukleinsäuremolekül nach Anspruch 13, wobei der Austausch in Position D23 zu A, W38 zu A, D235 zu A, Y249 zu A, Y68 zu S, Y70 zu S, Y75 zu S und F79 zu S vorliegt.

15. Nukleinsäuremolekül nach einem der Ansprüche 1 bis 14, das DNA ist.

16. Nukleinsäuremolekül nach einem der Ansprüche 1 bis 14, das RNA ist.

17. Vektor enthaltend ein Nukleinsäuremolekül nach einem der Ansprüche 1 bis 16.

18. Nicht-humaner Wirt, der mit einem Vektor nach Anspruch 17 transformiert ist oder ein Nukleinsäuremolekül nach einem der Ansprüche 1 bis 16 enthält.

19. Wirt nach Anspruch 18, der ein prokaryontischer Wirt oder eukaryontischer Wirt ist.

20. Wirt nach Anspruch 19, wobei der prokaryontische Wirt E. coli, Bacillus subtilis oder Streptomyces coelicolor oder, wobei der eukaryontische Wirt eine Saccharomyces sp., Aspergillus sp., Spodoptera sp. oder Pichia pastoris ist.

21. Fusionsprotein, das von einem Nukleinsäuremolekül nach einem der Ansprüche 1 bis 16 codiert oder von einem Wirt nach einem der Ansprüche 18, 19 oder 20 produziert wird.

22. Verfahren zur Herstellung eines Fusionsproteins nach Anspruch 21, wobei man einen Wirt nach einem der Ansprüche 18, 19 oder 20 unter geeigneten Bedingungen züchtet und das Fusionsprotein isoliert.

23. Arzneimittel, enthaltend ein Fusionsprotein nach Anspruch 21 und einen pharmazeutisch verträglichen Träger.

24. Arzneimittel, enthaltend ein Fusionsprotein, das von einem Nukleinsäuremolekül nach einem der Ansprüche 1 bis 11, 15 oder 16 codiert wird oder einen Vektor, der das Nukleinsäuremolekül enthält.

25. Arzneimittel enthaltend ein Fusionsprotein, das von einem Nukleinsäuremotekül nach einem der Ansprüche 4 bis 11, 15 oder 16 codiert wird oder einen Vektor, der das Nukleinsäuremolekül enthält, wobei der Modulator die Mistellektin B-Kette oder ein Fragment oder Derivat davon, das die biologische Aktivität der Mistellektin B-Kette besitzt, umfasst.

26. Arzneimittel nach Anspruch 25, wobei die Mistellektin B-Kette einen Austausch in Aminosäureposition 23, 38, 68, 70, 75, 79, 235 oder 249 oder eine Kombination derartiger Austausche aufweist.

27. Arzneimittel nach Anspruch 26, wobei der Austausch in Position 23 ein Austausch D23 zu A, in Position 38 ein Austausch W38 zu A, in Position 235 ein Austausch D235 zu A, in Position 249 ein Austausch Y249 zu A, in Position 68 ein Austausch Y68 zu S, in Position 70 ein Austausch Y70 zu S, in Position 75 ein Austausch Y75 zu S und in Position 79 ein Austausch F79 zu S ist.

28. Kit, enthaltend einen Vektor, der ein Nukleinsäuremolekül nach einem der Ansprüche 1 bis 16 enthält.

29. Kit enthaltend einen Vektor, das von einem Nukleinsäuremolekül nach einem der Ansprüche 4 bis 11, 15 oder 16 codiert wird oder einen Vektor, der das Nukleinsäuremoleküi enthält, wobei der Modulator die Mistellektin B-Kette oder ein Fragment oder Derivat davon, dass die biologische Aktivität der Mistellektin B-Kette besitzt, umfasst.

30. Kit nach Anspruch 29, wobei die Mistellektin B-Kette einen Austausch in Aminosäureposition 23, 38, 68, 70, 75, 79, 235 oder 249 oder eine Kombination derartiger Austausche aufweist.

31. Kit nach Anspruch 30, wobei der Austausch in Position 23 ein Austausch D23 zu A, in Position 38 ein Austausch W38 zu A, in Position 235 ein Austausch D235 zu A, in Position 249 ein Austausch Y249 zu A, in Position 68 ein Austausch Y68 zu S, in Position 70 ein Austausch Y70 zu S, in Position 75 ein Austausch Y75 zu S und in Position 79 ein Austausch F79 zu S ist.

32. Verfahren zur *in vitro* Testung eines prospektiven Modulators, wobei man folgende Schtitte durchführt:
(a) Transfektion einer Zielzelle mit einem Vektor, der ein Nukleinsäuremolekül nach einem der Ansprüche 1 bis 3, 6 bis 11, 15 oder 16 enthält;
(b) Transfektion einer Zielzelle mit einem Vektor, der eine Nukleinsäure enthält, die einen prospektiven Modulator codiert;
(c) Expression der Nukleinsäuren in der Zielzelle; und
(d) Messung der modulierenden Aktivität des prospektiven Modulators auf die Toxizität des Toxins.

33. Verfahren zur *in vitro* Testung eines prospektiven Modulators, wobei man folgende Schritte durchführt:
(a) Transfektion einer Zielzelle, die ein Nukleinsäuremolekül nach einem der Ansprüche 1 bis 3, 6 bis 11, 15 oder 16 enthält, mit einem Vektor, der eine Nukleinsäure enthält, die einen prospektiven Modulator codiert;
(b) Expression der Nukleinsäuren in der Zielzelle; und
(c) Messung der modulierenden Aktivität des prospektiven Modulators auf die Toxizität des Toxins.

34. Verfahren zur Herstellung eines prospektiven Modulators, wobei man folgende Schritte durchführt:
(a) Transfektion einer Zielzelle mit einem Vektor, der ein Nukleinsäuremolekül nach einem der Ansprüche 1 bis 3, 6 bis 11,15 oder 16 enthält;
(b) Transfektion einer Zielzelle mit einem Vektor, der eine Nukleinsäure enthält, die einen prospektiven Modulator codiert;
(c) Expression der Nukleinsäuren in der Zielzelle;
(d) Messung der modulierenden Aktivität des prospektiven Modulators auf die Toxizität des Toxins; und
(e) Isolierung des Modulators.

35. Verfahren zur Herstellung eines prospektiven Modulators, wobei man folgende Schritte durchführt:
(a) Transfektion einer Zielzelle, die ein Nukleinsäuremolekül nach einem der Ansprüche 1 bis 3, 6 bis 11, 15 oder 16 enthält, mit einem Vektor, der eine Nukleinsäure enthält, die einen prospektiven Modulator codiert;
(b) Expression der Nukleinsäuren in der Zielzelle;
(c) Messung der modulierenden Aktivität des prospektiven Modulators auf die Toxizität des Toxins; und
(d) Isolierung des Modulators.

## Claims

1. A nucleic acid molecule encoding a fusion protein with the following components:
(a) an effector module having an intracellular cytotoxic effect;
(b) a processing module which is covalently linked to said effector module and which has a recognition sequence for a protease, wherein the processing module comprises the mistletoe lectin propeptide or a fragment or derivative thereof, wherein the fragment or derivative has a recognition sequence for a protease and which induces the intracellular release of the effector module in the target cell by proteases of the cell itself during the receptor-mediated endocytosis in the endosomes and prelysosomes and wherein the mistletoe lectin propetide is encoded by a nucleic acid molecule selected from the group consisting of:
(i) nucleic acid molecules comprising a nucleotide sequence encoding the amino acid sequence indicated in Fig. 11.c or a fragment thereof;
(ii) nucleic acid molecules comprising the nucleotide sequence indicated in Fig. 11.c or a fragment thereof;
(iii) nucleic acid molecules hybridising with a nucleic acid molecule of (i) or (ii) under stringent conditions; and
(iv) nucleic acid molecules which have degenerated to the nucleic acid molecules indicated in (iii) according to the genetic code; and
(c) a targeting module which is covalently linked to the processing module and which specifically binds to the surface of a cell, whereby the internalisation of the fusion protein into the cell is mediated.

2. The nucleic acid molecule according to claim 1, wherein the effector module is an effector module which has an intracellular cytotoxic effect, wherein the effector module comprises the mistletoe lectin A-chain or a fragment or derivative thereof which has an intracellular cytotoxic effect, wherein the mistletoe A-chain is encoded by a nucleic acid molecule selected from the group consisting of:
(i) nucleic acid molecules comprising a nucleotide sequence encoding the amino acid sequence indicated in Fig. 11.a or a fragment thereof;
(ii) nucleic acid molecules comprising the nucleotide sequence indicated in Fig. 11.a or a fragment thereof;
(iii) nucleic acid molecules hybridising with a nucleic acid molecule of (i) or (ii) under stringent conditions;
and
(iv) nucleic acid molecules which are degenerated with regard to the nucleic acid molecules indicated in (iii) according to the genetic code.

3. The nucleic acid molecule according to claim 1, wherein the effector module comprises the mistletoe lectin A-chain or a fragment or derivative thereof which has an intracellular cytotoxic effect.

4. The nucleic acid molecule according to any one of claims 1 to 3, wherein the fusion protein moreover has the following component:
(d) a modulator module which is covalently linked with the processing module, the effector module and/or the targeting module and which modulates the intracellular toxic effect of the effector module.

5. The nucleic acid molecule according to claim 4, wherein the modulator module is encoded by a nucleic acid molecule selected from the group consisting of:
(i) nucleic acid molecules comprising a nucleotide sequence encoding the amino acid sequence indicated in Fig. 11.b or a fragment thereof; wherein the fragment has the biologic activity of the mistletoe lectin B-chain;
(ii) nucleic acid molecules comprising the nucleotide sequence indicated in Fig. 11.b or a fragment thereof; wherein the fragment encodes an amino acid sequence which has the biologic activity of the mistletoe lectin B-chain;
(iii) nucleic acid molecules hybridising with a nucleic acid molecule of (i) or (ii) under stringent conditions;
and
(iv) nucleic acid molecules which are degenerated with regard to the nucleic acid molecules indicated in (iii) according to the genetic code.

6. The nucleic acid molecule according to any one of claims 1 to 5 wherein the fusion protein moreover has the following component:
(e) an affinity module for purification which is covalently linked with the effector module, the processing module, the targeting module and/or the modulator module.

7. The nucleic acid molecule according to any one of claims 1 to 6, wherein the targeting module specifically recognises a cell of the immune system, a tumour cell or a cell of the nervous system.

8. The nucleic acid molecule according to claim 7 wherein the cell of the immune system is a cell of the specific immune system.

9. The nucleic acid molecule according to claim 7 or 8 wherein the cell of the immune system is a T-cell or a cell of the unspecific immune system and wherein the tumour cell is a degenerated cell of the immune system.

10. The nucleic acid molecule of claim 9, wherein the T-cell is a T_{H}2-cell.

11. The nucleic acid molecule according to any one of claims 6 to 10, wherein the affinity module comprises a histidine sequence, thioredoxin, strep-tag, T7-tag, flag-tag, a maltose-binding protein or GFP.

12. The nucleic acid molecule according to any one of claims 4 to 11, wherein the modulating module comprises the mistletoe lectin B-chain or a fragment or derivative thereof which has the biologic activity of the mistletoe lectin B-chain, of the peptides KDEL or HDEL.

13. The nucleic acid molecule according to claim 12, wherein the mistletoe lectin B-chain has an exchange in amino acid position 23, 38, 68, 70, 75, 79, 235 or 249 or a combination of such exchanges.

14. The nucleic acid molecule according to claim 13, wherein there is an exchange at position D23 to A, W38 to A, D235 to A, Y249 to A, Y68 to S, Y70 to S, Y75 to S and F79 to S.

15. The nucleic acid molecule according to any one of claims 1 to 14 which is DNA.

16. The nucleic acid molecule according to any one of claims 1 to 14, which is RNA.

17. A vector containing a nucleic acid molecule according to any one of claims 1 to 16.

18. A non-human host transformed with a vector according to claim 17 or containing a nucleic acid molecule according to any one of claims 1 to 16.

19. The host according to claim 18 which is a prokaryotic host or an eukaryotic host.

20. The host according to claim 19, wherein the prokaryotic host is E. coli, Bacillus subtilis or Streptomyces coelicolor or wherein the eukaryotic host is a Saccharomyces sp., Aspergillus sp., Spodoptera sp. or Pichia pastoris.

21. A fusion protein encoded by a nucleic acid molecule according to any one of claims 1 to 16 or produced by a host according to any one of claims 18, 19 or 20.

22. A process for the production of a fusion protein according to claim 21, wherein a host according to any one of claims 18, 19 or 20 is cultured under suitable conditions and the fusion protein is isolated.

23. A pharmaceutical composition containing a fusion protein according to claim 21 and a pharmaceutically acceptable carrier.

24. A pharmaceutical composition containing a fusion protein which is encoded by a nucleic acid molecule according to any one of claims 1 to 11, 15 or 16 or which contains a vector containing the nucleic acid molecule.

25. A pharmaceutical composition containing a fusion protein which is encoded by a nucleic acid molecule according to any one of claims 4 to 11, 15 or 16 or a vector containing the nucleic acid molecule wherein the modulator comprises the mistletoe B-chain or a fragment or derivative thereof with the biologic activity of the mistletoe B-chain.

26. A pharmaceutical composition according to claim 25, wherein the mistletoe lectin B-chain has an exchange at amino acid position 23, 38, 68, 70, 75, 79, 235 or 249 or a combination of such exchanges.

27. The pharmaceutical composition according to claim 26, wherein the exchange in position 23 is an exchange D23 to A, in position 38 an exchange W38 to A, in position 235 an exchange D235 to A, in position 249 an exchange Y249 to A, in position 68 an exchange Y68 to S, in position 70 an exchange Y70 to S, in position 75 an exchange Y75 to S and in position 79 an exchange F79 to S.

28. A kit containing a vector which contains a nucleic acid molecule according to any one of claims 1 to 16.

29. A kit containing a vector which is encoded by a nucleic acid molecule according to any one of claims 4 to 11, 15 or 16 or a vector which contains the nucleic acid molecule, wherein the modulator comprises the mistletoe lectin B-chain or a fragment or derivative thereof which has the biologic activity of the mistletoe lectin B-chain.

30. The kit according to claim 29, wherein the mistletoe lectin B-chain has an exchange in amino acid position 23, 38, 68, 70, 75, 79, 235 or 249 or a combination of such exchanges.

31. The kit according to claim 30, wherein the exchange in position 23 is an exchange D23 to A, in position 38 an exchange W38 to A, in position 235 an exchange D235 to A, in position 249 an exchange Y249 to A, in position 68 an exchange Y68 to S, in position 70 an exchange Y70 to S, in position 75 an exchange Y75 to S and in position 79 an exchange F79 to S.

32. A process for the *in vitro* testing of a prospective modulator, wherein the following steps are carried out:
(a) transfection of a target cell with a vector containing a nucleic acid molecule according to any one of claims 1 to 3, 6 to 11, 15 or 16;
(b) transfection of a target cell with a vector containing a nucleic acid which encodes a prospective modulator;
(c) expression of the nucleic acids in the target cell; and
(d) measurement of the modulating activity of the prospective modulator for the toxicity of the toxin.

33. A process for the *in vitro* testing of a prospective modulator, wherein the following steps are carried out:
(a) transfection of a target cell containing a nucleic acid molecule according to any one of claims 1 to 3, 6 to 11, 15 or 16 with a vector containing a nucleic acid which encodes a prospective modulator;
(b) expression of nucleic acids in the target cell; and
(c) measurement of the modulating activity of the prospective modulator for the toxicity of the toxin.

34. A process for producing a prospective modulator, wherein the following steps are carried out:
(a) transfection of a target cell with a vector containing a nucleic acid molecule according to any one of claims 1 to 3, 6 to 11, 15 or 16;
(b) transfection of a target cell with a vector containing a nucleic acid which encodes a prospective modulator;
(c) expression of the nucleic acids in the target cell;
(d) measurement of the modulating activity of the prospective modulators for the toxicity of the toxin; and
(e) isolation of the modulator.

35. A process for producing a prospective modulator, wherein the following steps are carried out:
(a) transfection of a target cell containing a nucleic acid molecule according to any one of claims 1 to 3, 6 to 11, 15 or 16 with a vector containing a nucleic acid which encodes a prospective modulator;
(b) expression of the nucleic acids in the target cell;
(c) measuring the modulating activity of the prospective modulator for the toxicity of the toxin; and
(d) isolation of the modulator.

## Revendications

1. Molécule d'acide nucléique, qui code une protéine de fusion, qui présente les composants suivants :
a) un module effecteur, qui exerce une activité cytotoxique intracellulaire ;
b) un module de traitement, qui est lié de manière covalente avec le module effecteur, et qui présente une séquence de reconnaissance pour une protéase, dans lequel le module de traitement comprend le propetide de la mistellectine ou comprend un fragment ou dérivé de celui-ci, dans lequel le fragment ou dérivé présente une séquence de reconnaissance pour une protéase et la libération intracellulaire du module effecteur a lieu dans la cellule cible par l'intermédiaire de protéases propres à la cellule au cours de l'endocytose médiée par le récepteur dans les endosomes et pré-lysosomes, et dans lequel le propeptide de la mistellectine est codé par une molécule d'acide nucléique, choisie dans le groupe consistant en :
(i) des molécules d'acide nucléique, comprenant une séquence nucléotidique codant pour la séquence d'acides aminés représentée à la figure 11.c ou un fragment de celle-ci ;
(ii) des molécules d'acide nucléique qui comprennent la séquence nucléotidique représentée à la figure 11.c ou un fragment de celle-ci ;
(iii) des molécules d'acide nucléique qui hybrident en conditions stringentes avec une molécule d'acide nucléique de (i) ou (ii) ; et
(iv) des molécules d'acide nucléique, qui ont une dégénérescence selon le code génétique par rapport aux molécules d'acide nucléique désignées sous (iii) ; et
c) un module de ciblage, lié de manière covalente au module de traitement, et qui se lie de manière spécifique à la surface d'une cellule, permettant ainsi l'internalisation de la protéine de fusion dans la cellule.

2. Molécule d'acide nucléique selon la revendication 1, dans laquelle le module effecteur est un module effecteur qui agit par cytotoxicité intracellulaire, le module effecteur comprenant la chaîne A de la mistellectine ou un fragment ou dérivé de celle-ci à activité cytotoxique intracellulaire, la chaîne A de la mistellectine étant codée par une molécule d'acide nucléique choisie dans le groupe consistant en :
(i) des molécules d'acide nucléique, comprenant une séquence nucléotidique codant pour la séquence d'acides aminés représentée à la figure 11.a ou un fragment de celle-ci ;
(ii) des molécules d'acide nucléique qui comprennent la séquence nucléotidique représentée à la figure 11.a ou un fragment de celle-ci ;
(iii) des molécules d'acide nucléique qui hybrident en conditions stringentes avec une molécule d'acide nucléique de (i) ou (ii) ; et
(iv) des molécules d'acide nucléique, qui ont une dégénérescence selon le code génétique par rapport aux molécules d'acide nucléique désignées sous (iii).

3. Molécule d'acide nucléique selon la revendication 1, dans laquelle le module effecteur comprend la chaîne A de la mistellectine ou un fragment ou dérivé de celle-ci exerçant une activité cytotoxique intracellulaire.

4. Molécule d'acide nucléique selon l'une des revendications 1 à 3, dans laquelle la protéine de fusion comprend en outre les composants suivants :
(d) un module modulateur, lié de manière covalente au module de traitement, lié au module effecteur et / ou au module de ciblage, et qui module l'activité toxique intracellulaire du module effecteur.

5. Molécule d'acide nucléique selon la revendication 4, dans laquelle le module modulateur est codé par une molécule d'acide nucléique choisie dans le groupe consistant en :
(i) des molécules d'acide nucléique, comprenant une séquence nucléotidique codant pour la séquence d'acides aminés représentée à la figure 11.b ou un fragment de celle-ci, ledit fragment possédant l'activité biologique de la chaîne B de la mistellectine;
(ii) des molécules d'acide nucléique qui comprennent la séquence nucléotidique représentée à la figure 11b ou un fragment de celle-ci, ledit fragment codant une séquence d'acides aminés qui possède l'activité biologique de la chaîne B de la mistellectine ;
(iii) des molécules d'acide nucléique qui hybrident en conditions stringentes avec une molécule d'acide nucléique de (i) ou (ii) ; et
(iv) des molécules d'acide nucléique, qui ont une dégénérescence selon le code génétique par rapport aux molécules d'acide nucléique désignées sous (iii).

6. Molécule d'acide nucléique selon l'une des revendications 1 à 5, dans laquelle la protéine de fusion présente en outre les composants suivants :
(e) un module d'affinité destiné à la purification, lié de manière covalente au module effecteur, au module de traitement, au module de ciblage et / ou au module modulateur.

7. Molécule d'acide nucléique selon l'une des revendications 1 à 6, dans laquelle le module de ciblage reconnaît spécifiquement une cellule du système immunitaire, une cellule tumorale ou une cellule du système nerveux.

8. Molécule d'acide nucléique selon la revendication 7, dans laquelle la cellule du système immunitaire est une cellule du système immunitaire spécifique.

9. Molécule d'acide nucléique selon la revendication 7 ou 8, dans laquelle la cellule du système immunitaire est une cellule T, ou une cellule du système immunitaire non spécifique et dans laquelle la cellule tumorale est une cellule dégénérée du système immunitaire.

10. Molécule d'acide nucléique selon la revendication 9, dans laquelle la cellule T est une cellule T_{H}2.

11. Molécule d'acide nucléique selon l'une des revendications 6 à 10, dans laquelle le module d'affinité comprend une séquence d'histidine, la thioredoxine, Strep-Tag, T7-Tag, Flag-Tag, la protéine de liaison au maltose ou GFP.

12. Molécule d'acide nucléique selon l'une des revendications 4 à 11, dans laquelle le module modulateur comprend la chaîne B de la mistellectine ou un fragment ou dérivé de celle-ci, qui possède l'activité biologique de la chaîne B de la mistellectine, ou les peptides KDEL ou HDEL.

13. Molécule d'acide nucléique selon la revendication 12, dans laquelle la chaîne B de la mistellectine présente une substitution de l'acide aminé en position 23, 38, 68, 70, 75, 79, 235 ou 249 ou une combinaison de telles substitutions.

14. Molécule d'acide nucléique selon la revendication 13, dans laquelle la substitution a lieu en position D23 en A, W38 en A, D235 en A, Y249 en A, Y68 en S, Y70 en S, Y75 en S et F79 en S.

15. Molécule d'acide nucléique selon l'une des revendications 1 à 14, qui est de l'ADN.

16. Molécule d'acide nucléique selon l'une des revendications 1 à 14, qui est de l'ARN.

17. Vecteur contenant une molécule d'acide nucléique selon l'une des revendications 1 à 16.

18. Hôte non humain, transformé avec un vecteur selon la revendication 17 ou contenant une molécule d'acide nucléique selon l'une des revendications 1 à 16.

19. Hôte selon la revendication 18, qui est un hôte procaryote ou eucaryote.

20. Hôte selon la revendication 19, dans lequel l'hôte procaryote est E. coli, Bacillus subtilis ou Streptomyces coelicolor, ou dans lequel l'hôte eucaryote est Saccharomyces sp., Aspergillus sp., Spodoptera s.p. ou Pichia pastoris.

21. Protéine de fusion, codée par une molécule d'acide nucléique selon l'une des revendications 1 à 16 ou produite par un hôte selon l'une des revendications 18, 19 ou 20.

22. Procédé de préparation d'une protéine de fusion selon la revendication 21, dans lequel on cultive un hôte selon l'une des revendications 18, 19 ou 20 dans des conditions appropriées et on isole la protéine de fusion.

23. Médicament, contenant une protéine de fusion selon la revendication 21 et un support pharmaceutiquement acceptable.

24. Médicament contenant une protéine de fusion codée par une molécule d'acide nucléique selon l'une des revendications 1 à 11, 15 ou 16 ou un vecteur contenant la molécule d'acide nucléique.

25. Médicament contenant la protéine de fusion codée par une molécule d'acide nucléique selon l'une des revendications 4 à 11, 15 ou 16 ou un vecteur contenant la molécule d'acide nucléique, dans lequel le modulateur comprend la chaîne B de la mistellectine ou un fragment ou dérivé de celle-ci, qui possède l'activité biologique de la chaîne B de la mistellectine.

26. Médicament selon la revendication 25, dans lequel la chaîne B de la mistellectine présente une substitution de l'acidé aminé en position aminé 23, 38, 68, 70, 75, 79, 235 ou 249 ou une combinaison de telles substitutions.

27. Médicament selon la revendication 26, dans lequel la substitution en position 23 est une substitution D23 en A, en position 38 une substitution W38 en A, en position 235 une substitution D235 en A, en position 249 une substitution Y249 en A, en position 68 une substitution Y68 en S, en position 70 une substitution Y70 en S, en position 75 une substitution Y75 en S et en position 79 une substitution F79 en S.

28. Kit, contenant un vecteur qui contient une molécule d'acide nucléique selon l'une des revendications 1 à 16.

29. Kit contenant un vecteur codé par une molécule d'acide nucléique selon l'une des revendications 4 à 11, 15 ou 16 ou un vecteur qui contient la molécule d'acide nucléique dans lequel le modulateur comprend la chaîne B de la mistellectine ou un fragment ou dérivé de celle-ci, qui possède l'activité biologique de la chaîne B de la mistellectine.

30. Kit selon la revendication 29, dans lequel la chaîne B de la mistellectine présente une substitution dans les positions des acides aminés 23, 38, 68, 70, 75, 79, 235 ou 249 ou une combinaison de telles substitutions.

31. Kit selon la revendication 30, dans lequel la substitution en position 23 est une substitution D23 en A, en position 38 une substitution W38 en A, en position 235 une substitution D235 en A, en position 249 une substitution Y249 en A, en position 68 une substitution Y68 en S, en position 70 une substitution Y70 en S, en position 75 une substitution Y75 en S et en position 79 une substitution F79 en S.

32. Procédé pour tester in vitro un modulateur prospectif, dans lequel on effectue les étapes suivantes :
a. transfection d'une cellule cible avec un vecteur contenant une molécule d'acide nucléique selon l'une des revendications 1 à 3, 6 à 11, 15 ou 16 ;
b. transfection d'une cellule cible avec un vecteur, qui contient une acide nucléique qui code pour un modulateur prospectif ;
c. expression des acides nucléiques dans la cellule cible ; et
d. mesure de l'activité modulatoire du modulateur prospectif sur la toxicité de la toxine.

33. Procédé pour tester in vitro un modulateur prospectif, dans lequel on effectue les étapes suivantes :
(a) transfection d'une cellule cible qui contient une molécule d'acide nucléique selon l'une des revendications 1 à 3, 6 à 11, 15 ou 16, avec un vecteur qui contient une acide nucléique codant un modulateur prospectif ;
(b) expression des acides nucléiques dans la cellule cible ; et
(c) mesure de l'activité modulatoire du modulateur prospectif sur la toxicité de la toxine.

34. Procédé de préparation d'un modulateur prospectif, dans lequel on effectue les étapes suivantes :
(a) transfection d'une cellule cible avec un vecteur qui contient une molécule d'acide nucléique selon l'une des revendications 1 à 3, 6 à 11, 15 ou 16 ;
(b) transfection d'une cellule cible avec un vecteur qui contient un acide nucléique codant un modulateur prospectif ;
(c) expression des acides nucléiques dans la cellule cible;
(d) mesure de l'activité modulatoire du modulateur prospectif sur la toxicité de la toxine ; et
(e) isolement du modulateur.

35. Procédé de préparation d'un modulateur prospectif, dans lequel on effectue les étapes suivantes :
(a) transfection d'une cellule cible qui contient une molécule d'acide nucléique selon l'une des revendications 1 à 3, 6 à 11, 15 ou 16 avec un vecteur qui contient une acide nucléique codant un modulateur prospectif ;
(b) expression des acides nucléiques dans la cellule cible ;
(c) mesure de l'activité modulatoire du modulateur prospectif sur la toxicité de la toxine ; et
(d) isolement du modulateur.
